# EUROPEAN PATENT SPECIFICATION

(11) **EP 2 969 009 B1**
(45) Date of publication and mention of the grant of the patent: **28.11.2018**
(21) Application number: 14764045.2
(22) Date of filing: 14.03.2014
(51) Int. Cl.: A61P 37/00, A61K 38/00, C07K 14/715, C07K 14/705, A61K 38/17, C07K 16/00, C12P 21/00, C12N 15/62

(54) **MODIFIED FC FUSION PROTEINS**
MODIFIZIERTE FC-FUSIONSPROTEINE
PROTÉINES DE FUSION DE FC MODIFIÉES

(30) Priority: 15.03.2013 US 201361798285 P; 18.07.2013 US 201361847975 P
(43) Date of publication of application: 20.01.2016
(73) Proprietor: Pyranose Biotherapeutics, Inc., Los Gatos, CA 95032 (US)
(72) Inventor: WARNER, Thomas, G., San Carlos, CA 94070 (US); COLON, Grace, Los Gatos, CA 95032 (US)
(74) Representative: Carpmaels & Ransford LLP
(86) International application number: PCT/US2014/029107
(87) International publication number: WO 2014/144621

(56) References cited:
- WO-A1-2011/068993
- WO-A1-2012/170938
- WO-A2-2007/005786
- WO-A2-2013/003649
- US-A1- 2005 031 584
- ROUILLER Y ET AL: "Effect of hydrocortisone on the production and glycosylation of an Fc-fusion protein in CHO cell cultures", BIOTECHNOLOGY PROGRESS, AMERICAN INSTITUTE OF CHEMICAL ENGINEERS, US, vol. 28, no. 3, 1 May 2012 (2012-05-01), pages 803-813, XP002733928, ISSN: 8756-7938, DOI: 10.1002/BTPR.1530 [retrieved on 2012-04-26]
- RAJU ET AL: "Terminal sugars of Fc glycans influence antibody effector functions of IgGs", CURRENT OPINION IN IMMUNOLOGY, ELSEVIER, OXFORD, GB, vol. 20, no. 4, 1 August 2008 (2008-08-01) , pages 471-478, XP025771206, ISSN: 0952-7915, DOI: 10.1016/J.COI.2008.06.007 [retrieved on 2008-07-17]
- DAMIANI ET AL.: 'Stable expression of a human-like sialylated recombinant thyrotropin in a Chinese hamster ovary cell line expressing alpha2,6-sialyltransferase' PROTEIN EXPRESSION AND PURIFICATION vol. 67, no. 1, 12 April 2009, pages 7 - 14, XP026133567
- CODDEVILLE ET AL.: 'Structural analysis of trisialylated biantennary glycans isolated from mouse serum transferrin Characterization of the sequence Neu5Gc(alpha2-3)Gal(betal-3)[Neu5Gc (alpha2-6)]GlcNAc(betal-2)Man' BIOCHIMICA ET BIOPHYSICA ACTA vol. 1475, no. 3, 26 July 2000, pages 321 - 328, XP004276653
- YLONEN ET AL.: 'Glycosylation analysis of two cysteine proteinase inhibitors from Atlantic salmon skin: di-O-acetylated sialic acids are the major sialic acid species on N-glycans' GLYCOBIOLOGY vol. 11, no. 7, 01 July 2001, pages 523 - 553, XP055281706
- LATTOVA ET AL.: 'Mass Spectrometric Profiling of N-Linked Oligosaccharides and Uncommon Glycoform in Mouse Serum with Head and Neck Tumor' JOURNAL OF THE AMERICAN SOCIETY FOR MASS SPECTROMETRY vol. 19, 14 February 2008, pages 671 - 685, XP022671110

## Description

### BACKGROUND OF THE INVENTION

In general, Fc-based fusion proteins comprise an immunoglobulin Fc region that is directly linked to a protein of interest such as an extracellular domain of a receptor, ligand, enzyme, or peptide. Fc fusion proteins are emerging as an important class of therapeutic agent and as of the end of 2011, six Fc fusion drugs were on the market, with four in phase 3 clinical trials and others at different stages of pre-clinical development. However, Fc fusion protein therapeutics may suffer from inadequate serum-life time compared to other clinically used human, humanized, chimeric, or mouse antibodies (Suzuki, T. et al. J. Immunol. 184, 1968-1976, 2010).

An example of a fusion protein characterized by a relatively short serum residence is Etanercept (Enbrel®), a drug currently marketed world-wide as a treatment for a variety of inflammatory diseases including moderate to severe rheumatoid arthritis, moderate to severe plaque psoriasis, psoratic arthritis, moderate to severe juvenile idiopathic arthritis and ankylosing spondylitis. Although the therapeutic protein has been found to be safe and effective for these treatments for over 10 years, the utilization of the molecule is limited by an unexpected short serum half life (80-120 hours about 4 days) when administered by subcutaneous injection. To achieve maximal efficacy of the molecule frequent injections are required. High frequency of dosing represents considerable discomfort to the subject and thus, over long periods of use the therapeutic may become poorly accepted, resulting in decreased patient compliance. In addition, frequent dosing poses a risk of injection-related infections particularly when the dosing is self-administrated.

Studies have shown that the neonatal Fc receptor, FcRn, may play an key role in the serum half-life of naturally occurring immunoglobulins, recombinant monoclonal antibodies and fusion molecules that contain an IgG Fc peptide linked to the C-terminus of the therapeutic peptide or protein. The FcRn binds the Fc domain of IgG at acidic pH in the endosome and protects the IgG from proteolytic degradation. The FcRn-bound IgG is then recycled and reintroduced into the circulation giving rise to a long serum half-life of the IgG. Molecules with high affinity binding for the receptor tend to have longer plasma residence times,
because they are better protected from endosome protolytic degradation. Those IgG molecules that bind poorly to the FcRn receptor are not shielded from endosomal proteolysis where they are degraded and do not return to the circulation. Thus, their serum residence time is reduced significantly.

In vitro measurements using surface plasmon resonance analysis have shown that Etanercept has a greatly reduced affinity for the FcRn compared to other Fc fusion molecules, naturally occurring immunoglobulins, and recombinant monoclonal antibodies. Without wishing to be bound by theory, it is hypothesized that the TNF receptor peptide portion of the Etanercept molecule interferes with the binding of the Fc portion of the molecule with the neonatal receptor. Steric interactions by the TNFR peptide or its conformationally induced changes in the Fc peptide, or the receptor N-linked glycans may adversely affect the binding affinity. This reduces the ability of the FcRn to protect the Etanercept molecule from proteolytic degradation, and ultimately, reduces the amount of therapeutic that can be recycled back into the circulation. Overall, this is manifested in a shorter than expected serum half-life.

Confounding the conclusion of these studies are results from analysis of serum clearance of another receptor-Fc fusion molecule, Lenercept, a potential therapeutic in development. Lenercept is constructed from the soluble TNF receptor p55 or type I receptor, fused to the Fc portion of an IgG1 peptide. The receptor segment of the molecule contains three N-linked glycosylation sites at positions Asn 33, 124 and 130. The pharmacokinetics of Lenercept appears to be mediated primarily by the partially completed N-linked glycans in the receptor region, specifically those structures which contain terminal N-acetyl glucosamine residues and somewhat by those containing exposed galactose residues. These residues drive serum clearance by carbohydrate binding cell surface receptors found on macrophages and other cell types that specifically recognize the terminal glucosamine residues. The hepatic receptor binds terminal galactose and in some systems may be the predominant clearance mechanism. N-acetyl glucosamine residues are not normally exposed on the glycan chains. Metabolically completed glycan chains do not contain terminal glucosamine residues because they substituted with galactose which is subsequently linked to sialic acid residues.

Serum clearance mechanisms for glycoprotein-Fc fusion proteins may differ from those of naturally occurring immunoglobulins and recombinant monoclonal antibodies. Two clearance pathways may moderate serum residence times for Fc fusion proteins. Clearance may be driven by a lack of protection from proteolysis resulting from low affinity binding to the neonatal FcRn and a second, competing pathway, clearance via carbohydrate receptors such as, the macrophage mannose/GlcNAc receptor and/or the hepatic galactose receptor.

Fc fusion proteins modified at the DNA level to include additional glycosylation sites into the amino acid sequence of the receptor portion of the molecule have been shown to have an enhanced serum half life over the corresponding unmodified Fc fusion proteins. However, although these modifications may provide enhancement in pharmacodynamic properties, re-engineering efforts such as these are difficult and cumbersome. Also, the degree of glycan substitution (site occupancy) at the newly introduced sites on the peptide can be highly problematic and the optimal locations for glycan insertion sites on the peptide must be determined by empirical experimentation. Moreover, the alteration to the peptide amino acid composition, may give rise to an unfavorable immune response by the patient.

Other approaches for improving the half-life of Fc fusion proteins involve increasing the levels of sialic acid on the molecule by altering the components of the cell culture media during manufacture to bolster the metabolic capacity of the expression cells and achieve a greater degree of glycan chain completion. This process involves the inclusion of a number of carbohydrate molecules in the cell culture media that are known to be precursors of the carbohydrate components of the glycans found on Fc fusion proteins. In addition, after cell culture is complete, the modified Fc fusion proteins are purified from the cell culture fluid. Fc fusion protein molecules with more complete glycan chains are selected out of the crude protein preparation by using anion exchange chromatography. This process tends to enrich the final product with more highly sialylated species (completed glycan chains). Although some improvement in the sialylated glycan content of the molecule results from these manufacturing processes, the method is inefficient and results in lower yields of the protein because fractionation of the crude cell culture fluid is required and the amount of recombinant protein recovered is greatly reduced. Rouiller et al., (2012) (Biotechnology Progress, American Institute of Chemical Engineers, vol. 28(3) pp 803-813) discloses the effect of hydrocortisone on the production and glycosylation of an Fc-fusion protein in CHO cell cultures. WO 2007/005786 discloses methods of increasing the sialylation of the oligosaccharides in the Fc region of the fusion proteins. WO 2011/068993 discloses the introduction of additional glycosylation sites in the Fc part of the fusion protein.

Recently a recombinant form of Etanercept has been expressed in a strain of yeast specifically engineered to give human-type glycosylation patterns on proteins expressed in this host (Liu, L. et al., Pharma Res. 2012). Under optimal culture conditions this expression system also gave incomplete glycan chain extension on the recombinant product. Analysis of the IEF profile reveals that the resulting yeast-derived TNFR-IgG also lacked complete sialylation and it was notably less than the CHO produced protein (commercial Etanercept). These studies support the notion that the molecular features of the TNFR-IgG molecule render it an intransigent substrate for the metabolic glycosylation machinery even in this extensively glyco-engineered microbial host. There is a need for an Etanercept therapeutic with significantly longer serum half-life.

Accordingly there remains a need for reliable methods to improve the serum half-life of Fc fusion proteins, including but not limited to Etanercept, without decreasing their therapeutic efficacy.

### SUMMARY OF THE INVENTION

The invention provides an Fc fusion protein, comprising an immunoglobulin Fc region linked to a biologically active polypeptide comprising one or more oligosaccharide, wherein:
i. more than 10% of all oligosaccharides on the biologically active polypeptide terminate in sialic acid; and
ii. at least one of the oligosaccharides linked to the biologically active polypeptide comprises a biantennary glycan structure terminating in at least 3 sialic acid molecules, or a triantennary glycan structure terminating in at least 4 sialic acid molecules, or a tetraantennary glycan structure terminating in at least 5 sialic molecules; and
wherein the serum half-life of the Fc fusion protein is at least 30% longer relative to a corresponding Fc fusion protein in which none of the oligosaccharides comprise a biantennary glycan structure terminating in 3 or 4 sialic acid molecules.

The invention also provides a method of preparing an Fc fusion protein of the invention, said method comprising:
a) providing a Fc fusion protein, wherein the Fc fusion protein comprises an immunoglobulin Fc region linked to a biologically active polypeptide comprising at least one oligosaccharide; and
b) exposing the Fc fusion protein to the action of at least one glycosyltransferase to result in the Fc fusion protein of any preceding claim.

The invention also provides an Fc fusion protein of the invention for use in treating a disorder in a subject in need thereof.

In one aspect, the disclosure provides a Fc fusion protein, wherein the Fc fusion protein comprises an immunoglobulin Fc region linked to a biologically active polypeptide comprising one or more oligosaccharide, such that more than 10% of all oligosaccharides on the biologically active polypeptide in the population terminate in sialic acid and at least one of the oligosaccharides linked to the biologically active polypeptide comprises a biantennary glycan structure terminating in at least 3 sialic acid molecules, or a triantennary glycan structure terminating in at least 4 sialic acid molecules, or a tetraantennary glycan structure terminating in at least 5 sialic molecules. In some examples, at least 5%, 10%, 15%, 20% of the oligosaccharides linked to the biologically active polypeptide comprise a biantennary glycan structure terminating in at least 3 sialic acid molecules. In some examples, at least 5%, 10%, 15%, 20% of the oligosaccharides linked to the biologically active polypeptide comprise a triantennary glycan structure terminating in at least 4 sialic acid molecules. In some examples at least 5%, 1 0%, 15%, 20% of the oligosaccharides linked to the biologically active polypeptide comprise a tetraantennary glycan structure terminating in at least 5 sialic molecules.

In some examples, at least one of the oligosaccharides linked to the biologically active polypeptide comprises a biantennary glycan structure terminating in at least 3 or 4 sialic acid molecules. In some examples, more than 20%, 30%, 40%, 50%, 60%, 70%, 80%, or 90% of all oligosaccharides in the population terminate in sialic acid. In some examples more than 10%, 20%, 30%, 40%, 50%, 60%, 70%, 80%, or 90% of N-linked oligosaccharides terminate in sialic acid. In some examples, more than 10%, 20%, 30%, 40%, 50%, 60%, 70%, 80%, or 90% of O-linked oligosaccharides terminate in sialic acid.

In some examples, at least one of the N-linked oligosaccharide in the Fc fusion protein terminates in sialic acid molecules linked to a 3 and a 6 position of a first galactose residue on a first antenna of each oligosaccharide, and a 6 position of a second galactose residue on a second antenna of the same oligosaccharide.

In some examples, at least one of the N-linked oligosaccharide in the Fc fusion protein terminates in sialic acid molecules linked to a 3 and a 6 position of a first galactose residue on a first antenna of each oligosaccharide, and a 3 position of a second galactose residue on a second antenna of the same oligosaccharide.

In some examples, at least one N-linked oligosaccharide in the Fc fusion protein terminates in sialic acid molecules linked to a 3 position of a first galactose residue on a first antenna of each oligosaccharide, and a 3 and a 6 position of a second galactose residue on a second antenna of the same oligosaccharide.

In some examples, at least one N-linked oligosaccharide in the Fc fusion protein terminates in sialic acid molecules linked to a 6 position of a first galactose residue on a first antenna of each oligosaccharide and a 3 and a 6 position of a second galactose residue on a second antenna of the same oligosaccharide.

In some examples, at least one N-linked oligosaccharide in the Fc fusion protein terminates in a sialic acid molecule linked to a first sialic acid residue linked to a 3 or a 6 position of a first galactose residue on a first antenna of each oligosaccharide, and a sialic acid molecule linked to a second sialic acid residue linked to a 3 or a 6 position of a second galactose residue on a second antenna of each oligosaccharide.

In some examples, at least one N-linked oligosaccharide in the Fc fusion protein terminates in a sialic acid molecule linked to a first sialic acid residue linked to a 3 or a 6 position of a first galactose residue on a first antenna of each oligosaccharide, and a sialic acid molecule linked to a 3 or a 6 position of a second galactose residue on a second antenna of each oligosaccharide.

In some examples at least one of N-linked oligosaccharide terminates in a sialic acid molecule linked to a 3 or a 6 position of a first galactose residue on a first antenna of each oligosaccharide, and a sialic acid molecule linked to a sialic acid residue linked to a 3 or a 6 position of a second galactose residue on a second antenna of each oligosaccharide.

In some examples, at least one of N-linked oligosaccharide terminates in a 2,8 sialic acid molecule linked to a 2,3 sialic acid molecule linked to a first galactose residue on a first antenna of the oligosaccharide, and a 2,8 sialic acid molecule linked to a 2,3 sialic acid molecule linked to a second galactose residue on a second antenna of the oligosaccharide.

In some examples, at least one N-linked oligosaccharide terminates in a 2,8 sialic acid molecule linked to a 2,3 sialic acid molecule linked to a first galactose residue on a first antenna of the oligosaccharide, and a 2,3 sialic acid molecule linked to a second galactose residue on a second antenna of the oligosaccharide.

In some examples, at least one of N-linked oligosaccharides in the Fc fusion protein is selected from the group consisting of

Neu5Ac Neu5Ac Neu5Ac
Gal Gal
GlcNAc GlcNAc
Man Man
Man
GlcNAc
GlcNAc
3,6 disialic-3 arm; 3 or 6 mono sialic-6 arm ,

Neu5Ac Neu5Ac Neu5Ac
Gal Gal
GlcNAc GlcNAc
Man Man
Man
GlcNAc
GlcNAc Fuc
3,6 disialic-3 arm; 3 or 6 mono sialic-6 arm ,

Neu5Ac
Neu5Ac Neu5Ac
Gal
Gal
GlcNAc
GlcNAc
Man
Man
Man
GlcNAc
GlcNAc
3,6 disialic-6 arm; 3 or 6 mono sialic-3 arm ,

Neu5Ac Neu5Ac Neu5Ac Neu5Ac Neu5Ac Neu5Ac Neu5Ac Neu5Ac Neu5Ac Neu5Ac Neu5Ac
Gal Gal Gal Gal Gal Gal
GlcNAc GlcNAc GlcNAc GlcNAc GlcNAc GlcNAc
Man Man Man Man Man Man
Man Man Man
GlcNAc GlcNAc GlcNAc
GlcNAc —Fuc GlcNAc GlcNAc —Fuc
3,6 disialic-6 arm; 3 or 6 mono sialic-3 arm , 3,6 disialic acid; both 3 and 6 arm , 3,6 disialic acid; both 3 and 6 arm ,
or tetra sialic -bianatennary or tetra sialic -bianatennary

Neu5Ac Neu5Ac Neu5Ac Neu5Ac
Neu5Ac Neu5Ac Neu5Ac Neu5Ac
Gal Gal Gal Gal
GlcNAc GlcNAc GlcNAc GlcNAc
Man Man Man Man
Man Man
GlcNAc GlcNAc
GlcNAc GlcNAc Fuc
2,8 sialic-sialic; 2,3 sialic-galactose; 3 and 6 arm , 2,8 sialic-sialic; 2,3 sialic-galactose; 3 and 6 arm ,

Neu5Ac
Neu5Ac
Neu5Ac
Neu5Ac
Neu5Ac
Neu5Ac
Gal
Gal
Gal
Gal
GlcNAc
GlcNAc
GlcNAc
GlcNAc
Man
Man
Man
Man
Man
Man
GlcNAc
GlcNAc
GlcNAc
GlcNAc—Fuc
2,8 sialic-sialic; 2,3 sialic-galactose; 3 or 6 arm , and 2,8 sialic-sialic; 2,3 sialic-galactose; 3 or 6 arm .

The Fc fusion protein may comprise at least one of N-linked oligosaccharide terminates in a (Neu5Ac)ₙ, wherein n is an integer between 3-30, for examples n may be an integer of value 3-10 or 3-20. In some examples n is an integer of value 3-5.

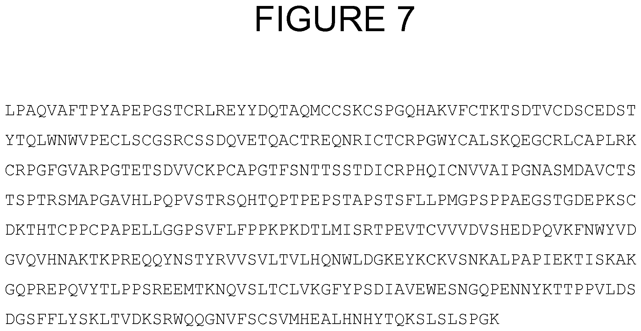

In some examples, the biologically active polypeptide may comprise a TNFR2 fragment, for example at least a part of an extracellular domain of TNFR2. The Fc fusion protein may comprise a sequence which has at least 70%, 75%, 80%, 85%, 90%, 95%, or 99% homology to the amino acid sequence of Figure 7. The Fc fusion proteins may comprise the amino acid sequence of Figure 7. The population of the Fc Fusion protein may comprises at least about 4, 4.5, 5, 5.5, 6, 6.5, 7, 7.5 or 8 moles of *N*-linked sialic acid for each mole of fusion proteins. In further examples, the biologically active polypeptide may comprises a TNFR1 fragment, for example at least a part of an extracellular domain of TNFR1.

The serum half-life of the population of Fc fusion proteins may be longer relative a corresponding population of Fc fusion proteins in which none of the oligosaccharides comprise a biantennary glycan structure terminating in 3 or 4 sialic acid molecules. For example, the serum half-life may be at least 30%, 40%, 50%, 60%, 70%, 80%, 100% or 200% longer relative to a corresponding population of Fc fusion proteins in which none of the oligosaccharides comprise a biantennary glycan structure terminating in 3 or 4 sialic acid molecules.

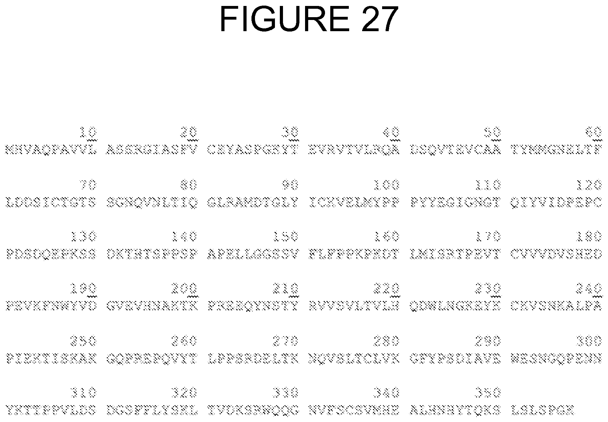

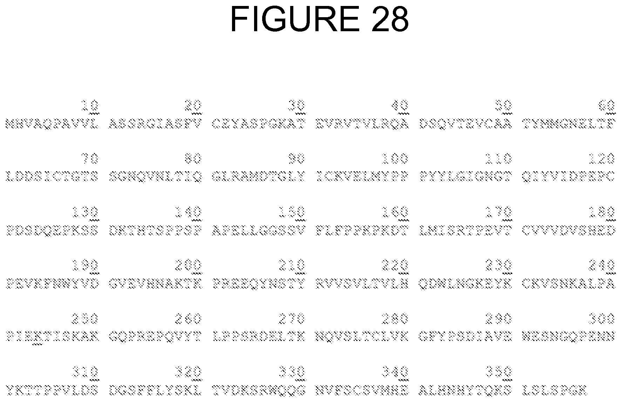

In some examples, the biologically active peptide comprises a CTLA-4 fragment, for example extracellular domain of CTLA-4. In some examples, the Fc fusion protein comprises a sequence which has at least 70%, 75%, 80%, 85%, 90%, 95%, or 99% homology to the amino acid sequence of Figure 27 or Figure 28. In some examples, the Fc fusion protein comprise the amino acid sequence of Figure 27 or Figure 28.

The biologically active peptide may also comprise protective antigen (PA) for example PA83.

The disclosure also provides a composition comprising a Fc fusion protein of the disclosure and at least one glycosyltransferase. The at least one glycosyltransferase may be a galactosyltransferase or a sialyltransferase. The galactosyltransferase may be_bovine or human β4Gal T. The sialyltransferase may be Pd2,6ST, rST6Gal 1, or 2,3 sialyltransferase CSTII.

In another aspect, the disclosure provides a method of preparing an enhanced Fc fusion protein, the method comprising providing a Fc fusion protein, wherein the Fc fusion protein comprises an immunoglobulin Fc region linked to a biologically active polypeptide comprising at least one oligosaccharide, and exposing the Fc fusion protein to the action of at least one glycosyltransferase to result in the enhanced Fc fusion protein characterized in that more than 10% of all oligosaccharides on the biologically active polypeptide in the Fc fusion protein terminate in sialic acid with at least one of the oligosaccharides in the enhanced population comprises a biantennary glycan structure terminating in 3 sialic acid molecules or a triantennary glycan structure terminating in 4 sialic acid molecules, or a tetraantennary glycan structure terminating in 5 sialic acid molecules.. In some cases, at least one of the oligosaccharides in the enhanced Fc fusion protein comprises a biantennary glycan structure terminating in 4 sialic acid molecules. The method may not require chromatographic enrichment for sialylated glycan content of the crude Fc fusion proteins. The method may be carried out *in vivo* or *in vitro.* The at least one glycosyltransferase may be a galactosyltransferase or a sialyltransferase. In some examples, the method at least two glycosyltransferases are used. In some cases two glycosyltransferases are used such that a first glycosyltransferase is a galactosyltransferase and a second glycosyltransferase is a sialyltransferase. The method of the disclosure may be used to prepare at least 5 mg, at least 10 mg, at least 15 mg, or at least 20 mg of said enhanced population of Fc fusion proteins.

In some examples the biologically active peptide may comprise a TNFR2 fragment, for example a part of an extracellular domain of TNFR2. In some examples the biologically active peptide may comprise a TNFR1 fragment, for example a part of an extracellular domain of TNFR1. The Fc fusion protein may comprise a sequence which has at least 70%, 75%, 80%, 85%, 90%, 95%, or 99% homology to the amino acid sequence of Figure 7. The Fc fusion protein may comprise the amino acid sequence of Figure 7. The enhanced Fc fusion protein may comprise at least about 4, 4.5, 5, 5.5, 6, 6.5, 7, 7.5 or 8 moles of sialic acid for each mole of fusion proteins. In other examples, the biologically active peptide comprises a CTLA-4 fragment, for example, an extracellular domain of CTLA-4. The Fc fusion protein may comprise a sequence which has at least 70%, 75%, 80%, 85%, 90%, 95%, or 99% homology to the amino acid sequence of Figure 27 or Figure 28. The Fc fusion protein may comprise the amino acid sequence of Figure 27 or Figure 28. In other examples, the biologically active peptide comprises protective antigen (PA), for example PA83.

The enhanced population of Fc fusion proteins obtained by the methods of the disclosure may have a serum half-life of at least 30%, 40%, 50%, 60%, 70%, 80%, 100% or 200% longer relative to a corresponding population of Fc fusion proteins in which none of the oligosaccharides comprises a biantennary glycan structure terminating in 3 or 4 sialic acid molecules.

In another aspect, the disclosure provides a method of treating a disorder in a subject in need thereof, comprising administering to the subject a pharmaceutical composition comprising the population of Fc fusion proteins of the instant disclsoure. The disorder may be an inflammatory disorder. The inflammatory disorder may be arthritis. The inflammatory disorder may also be rheumatoid arthritis. The inflammatory disorder may also be juvenile rheumatoid arthritis. The inflammatory disorder may also be psoriatic arthritis. In some examples, the inflammatory disorder is ankylosing spondylitis. In some examples, the disorder may be Anthrax poisoning. The pharmaceutical composition may be administered at a frequency of no more than once a week or no more than once every two weeks.

In one aspect, the disclosure provides a population of Fc fusion proteins. In one embodiment, the Fc fusion proteins each comprise an immunoglobulin Fc region linked to a biologically active polypeptide comprising at least one oligosaccharide, and further wherein more than 30%, 40%, 50%, 60%, 70%, 80%, or 90% of all oligosaccharides in the population terminate in sialic acid. In some embodiments, the biologically active polypeptide is a TNFR2 fragment. In some embodiments, the serum half-life of the population of Fc fusion proteins is at least 30%, 40%, 50%, 60%, 70%, 80%, 100% or 200% longer relative to a corresponding population of Fc fusion proteins in which less than 20% of oligosaccharides of the corresponding population terminate in sialic acid. In some embodiments, more than 50%, 60%, 70%, 80%, or 90% of N-linked or O-linked oligosaccharides terminate in sialic acid. In some embodiments, more than 50%, 60%, 70%, 80%, or 90% of N-linked oligosaccharides terminate in sialic acid linked to a 3 position of a galactose residue within each oligosaccharide. In some embodiments, more than 50%, 60%, 70%, 80%, or 90% of N-linked oligosaccharides terminate in sialic acid linked to a 3 position of a first galactose residue on a first antenna of each oligosaccharide and to the 6 position of a second galactose residue on a second antenna of the same oligosaccharide. In some embodiments, more than 50%, 60%, 70%, 80%, or 90% of N-linked oligosaccharides terminate in sialic acid linked to 2,3 and 2,6 position of galactose residues as a mixture on both antennae or branches of the oligosaccharide structure. In some embodiments, more than 30 %, 40%, 50 %, 60%,70% , 80% or 90% of N-linked Fab oligosaccharides terminate in sialic acid linked to both the 2,3 and 2,6 position of a single galactose on one or both antennae or branches. In some embodiments, more than 90% of O-linked oligosaccharides terminate in sialic acid linked to the 3 position of galactose. In some embodiments, more than 50% of the N and O linked glycans terminate in sialic acid.

In some embodiments, the TNFR2 fragment comprises at least a part of an extracellular domain of TNFR2. In some embodiments, the Fc fusion proteins comprise a sequence which has at least 70%, 75%, 80%, 85%, 90%, 95%, or 99% homology to the amino acid sequence of Figure 7. In some embodiments, the Fc fusion proteins comprise the amino acid sequence of Figure 7. In some embodiments, the population comprises at least about 2, 2.5, 3, 3.5, 4, 8, 16, or more mols of N-linked sialic acid for each mol of fusion protein (e.g. each fusion protein monomer). A composition may comprise a population of Fc fusion proteins in combination with at least one glycosyltransferase, such as at least one galactosyl transferase and/or at least one sialyltransferase.

In one aspect, the disclosure provides a method of preparing an enhanced population of Fc fusion proteins. In one embodiments, the method comprises (a) providing a population of Fc fusion proteins, wherein the Fc fusion proteins each comprise an immunoglobulin Fc region linked to a biologically active polypeptide comprising at least one oligosaccharide; and (b) exposing the population of Fc fusion proteins to the action of at least one glycosyltransferase to result in the enhanced population of Fc fusion proteins characterized in that more than 30% of oligosaccharides in the population terminate in sialic acid. In some embodiments, the biologically active peptide is a TNFR2 fragment. In some embodiments, the serum half-life of the population of Fc fusion proteins is at least 30%, 40%, 50%, 60%, 70%, 80%, 100% or 200% longer relative to a corresponding population of Fc fusion proteins in which less than 20% of oligosaccharides of the corresponding population terminate in sialic acid. In some embodiments, the method does not require chromatographic enrichment for sialylated glycan content of the crude Fc fusion proteins. The method may be carried out in vivo or in vitro. The method may use at least one (e.g. 1, 2, 4, 5, or more) glycosyltransferases, such as at least one galactosyl transferase and/or at least one sialyltransferase. In some embodiments, the method is used to prepare at least 5mg, at least 10mg, or more of said enhanced population of Fc fusion proteins.

In some embodiments, the TNFR2 fragment comprises at least a part of an extracellular domain of TNFR2. In some embodiments, the Fc fusion proteins comprise a sequence which has at least 70%, 75%, 80%, 85%, 90%, 95%, or 99% homology to the amino acid sequence of Figure 7. In some embodiments, the Fc fusion proteins comprise the amino acid sequence of Figure 7. In some embodiments, the population comprises at least about 2, 2.5, 3, 3.5, 4, 8, 16, or more mols of N-linked sialic acid for each mol of fusion protein (e.g. each fusion protein monomer). A composition may comprise a population of Fc fusion proteins in combination with at least one glycosyltransferase, such as at least one galactosyl transferase and/or at least one sialyltransferase.

In one aspect, the disclosure provides a method of treating a disorder in a subject in need thereof. In one embodiments, the method comprises administering to the subject a pharmaceutical composition comprising a population of Fc fusion proteins as described herein, such as with regard to any other aspect of the present disclosure. Non-limiting examples of disorders that may be treated in accordance with this embodiment include inflammatory disorders, such as arthritis (e.g. rheumatoid arthritis, juvenile rheumatoid arthritis, psoriatic arthritis, and ankylosing spondylitis.). In some embodiments, the pharmaceutical composition is administered at a frequency of no more than once a week, or no more than once every two weeks.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

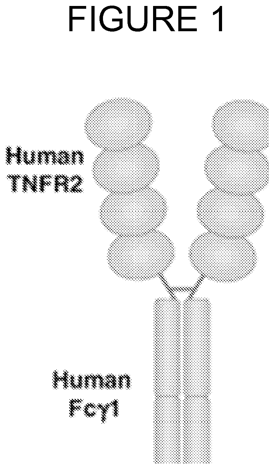

Fig. 1 shows the domain organization of Etanercept (Enbrel®).
Fig. 2 shows examples of different glycosylation patterns.

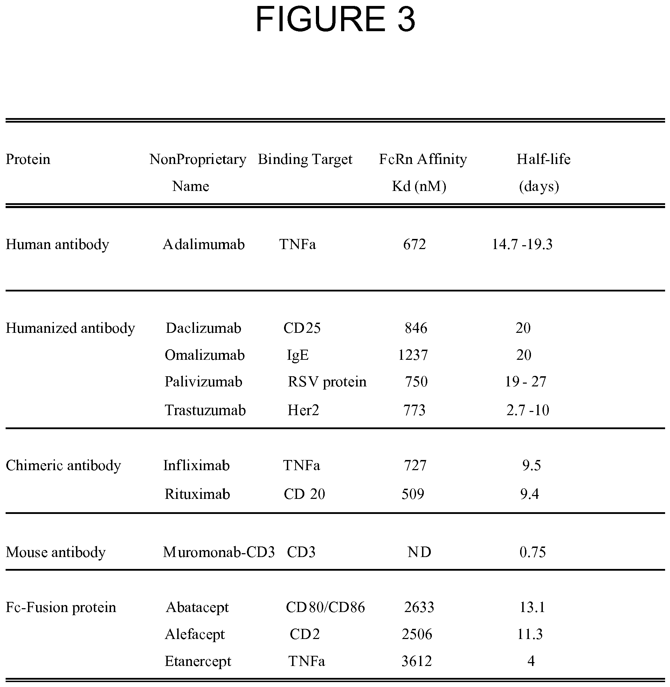

Fig. 3 shows comparison of neonatal Fc receptor binding and serum half-life of immunoglobulins, recombinant antibodies and Fc fusion proteins.
Fig. 4 shows an isoelectric focusing (IEF) acrylamide gel of commercial Etanercept.
Fig. 5 shows the glycan profile of the N-linked glycans on Etanercept.

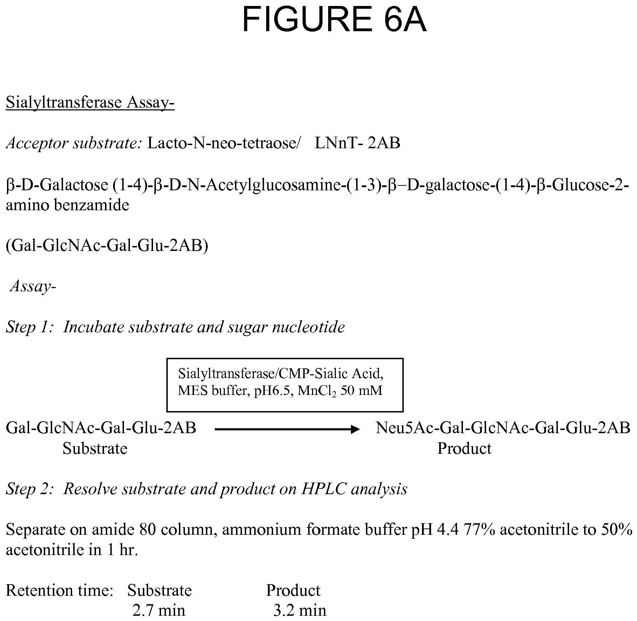

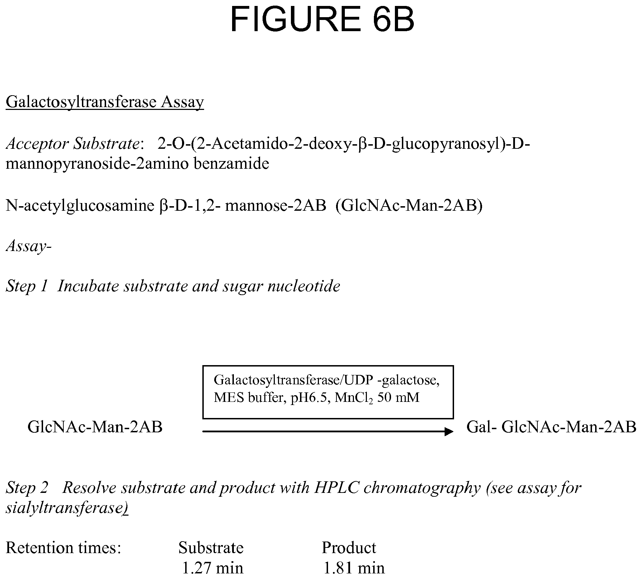

Figs. 6a and 6b show the acceptor substrates and assays for quantifying sialyltransferase and galactosyltransferase activities.
Fig. 7 shows the amino acid sequence of a monomeric unit of Etanercept.
Fig. 8A shows determination of optimal reaction time and enzyme concentration for Etanercept treated with Pd2,6ST and bovine β4GalT.
Fig. 8B shows isoelectric focusing (IEF) acrylamide gel analysis of preparative level of crude reaction product of etanercept with Pd2,6ST and bovine beta-galactosyltransferase.
Figs. 9A. and 9B shows isoelectric focusing (IEF) acrylamide gel analysis of preparative level, protein A-purified etanercept glycoengineered with Pd2,6ST and bovine beta-galactosidase. B. shows SDS polyacrylamide gel analysis of purified glyco etanercept under denaturing and non-denaturing conditions.
Fig. 10 shows sensor gram analysis to determine binding affinity of glyco etanercept and etanercept toward immobilized Tumor necrosis factor, TNF, using Forte Bio instrumentation.
Fig. 11A shows glycoprofile of N-linked glycans on glyco engineered etanercept modified with Pd2,6ST and bovine beta-galactosyltransferase. 11 B. shows MALDI TOF mass spectra of permethylated N- linked glycan pool on glyco-etanercept. 11 C. shows the potential major glycan structural forms on glyco-etanercept consistent with the observed molecular masses of the components in the N-linked glycan pool.
Fig. 12 shows a comparison in the glyco profiles of N- linked glycans on etanercept and glyco-etanercept verifying that > 90% of the glycans terminate in sialic acid after transferase reaction with Pd2,6ST and bovine beta-galactosyltransferase.
Fig. 13 shows the change in the Fc glycans on etanercept after treatment with Pd2,6ST and bovine beta-galactosyltransferase.
Fig. 14A shows selected peaks taken from HPLC glyco profile subjected to permethylation/MALDI mass spec analysis. 14 B. shows MALDI TOF mass spectral analysis of the 2 AB labeled-glycan components of selected peaks from glyco profile.
Fig. 15 shows linkage analysis in the glycan pool of etanercept and glyco-etanercept using permethylated alditol acetate analysis. Shown is gas chromatographic analysis of the resulting alditol acetates of the glycan pool.
Fig. 16 shows mass spectral fragmentation pattern, Ms/Ms, of 2AB labeled glycan HPLC fractions # 9 and #12 as their permethylated derivatives.
Fig. 17 shows IEF acrylamide gel analysis of crude glycosylation reaction, optimization of reaction time and enzyme levels at an analytical scale for etanercept reaction products obtained with human ST6Gal1 and human β4GalT after 4 and 24 hours at 37 °C.
Fig.18 shows IEF analysis of crude preparative level reaction of etanercept with human ST6Gal1 and human β4GalT at 4 hr and 8 hr reaction time. Also shown is comparison to final reaction product obtained with Pd2,6ST.
Fig 19 Panel A shows the glyco profile of the N-linked glycans of the glycosylation product of glycoetanercept obtained using human ST6Gal1 and human β4GalT. Panel B shows corresponding MALDI TOF mass spectrum of the permethylated glycan pool.
Fig. 20 shows IEF acrylamide gel electrophoresis of reaction conditions optimization of 2,3 sialyltransferase CST II and human β4GalT on etanercept at an analytical scale.
Fig 21. Panel A shows the glyco profile of the N-linked glycans of glycoetanercept obtained using 2,3 sialyltransferase CST II and human β4GalT. Panel B shows the corresponding MALDI TOF mass spectrum of the permethylated glycan pool.
Fig. 22 shows IEF acrylamide gel electrophoresis comparison of protein A-purified glyco etanercept versions prepared with (1) Pd2,6ST/bovine β4GalT, (2) humanST6Gal1/human β4 GalT and (3) Campylobacter CST II/human β4GalT.
Fig 23 shows summary PK parameters for etanercept and glyco-etanercept determined by model-independent analysis of mean concentration versus time data.
Fig 24 shows summary PK parameters etanercept and glyco-etanercept determined by model-dependent analysis of mean concentration versus time data.
Fig 25 shows isoelectric acrylamide analysis of glycosylation of abatacept. Glyco-remodeling reactions were carried out a pH 6.4 and 7.5 and the products purified using protein A chromatography prior to gel electrophoresis.

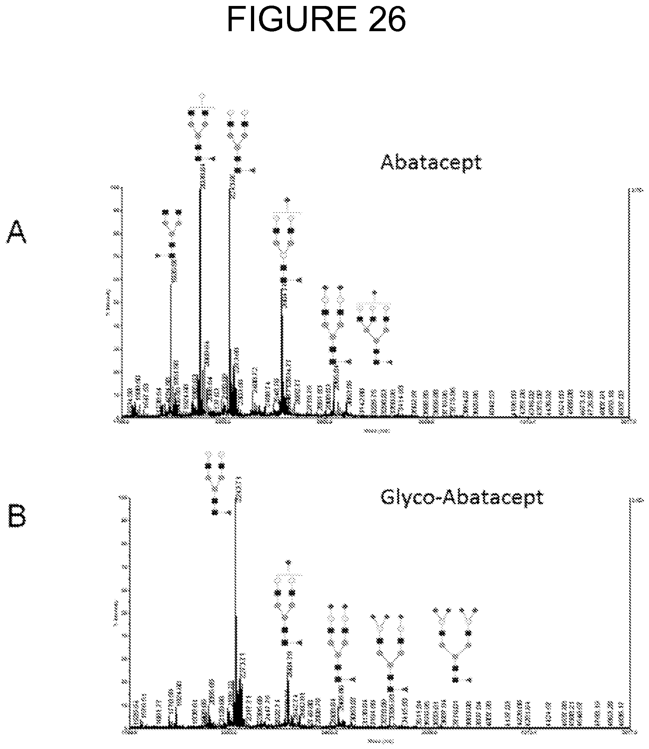

Fig 26 shows the MALDI TOF mass spectra glycoprofile of N-linked glycans of (A) Abatacept and (B) Glyco-abatacept, modified at pH 7.5 with Pd2,6 ST and human β4GalT.
Fig 27 shows the amino acid sequence of a monomeric unit of Abatacept.
Figure 28 shows shows the wild-type amino acid sequence of a monomeric unit of Abtacept.

### DETAILED DESCRIPTION OF THE INVENTION

The present invention provides Fc fusion proteins, and methods to prepare Fc fusion proteins. Fc fusion proteins of the invention have improved biological properties, such as for example increased serum half-life. The present invention also provides methods to modify the oligosaccharide structures present in the fusion-proteins, *in vitro,* with suitable glycosyltransferses to result in metabolically complete glycan chains. More efficient manufacturing processes can be achieved using these modifications, for example because chromatographic fractionation of the crude protein material during purification may not be required to achieve an enrichment of the fusion proteins with higher sialic acid content.

### Definitions

Unless otherwise stated, the following terms used in this application, including the specification and claims, have the definitions given below. It must be noted that, as used in the specification and the appended claims, the singular forms "a," "an" and "the" include plural referents unless the context clearly dictates otherwise.

The terms "effective amount" or "therapeutically effective amount," as used herein, refer to a sufficient amount of an agent (e.g. a fusion protein) being administered which will relieve to some extent one or more of the symptoms of the disease or condition being treated. The result can be reduction and/or alleviation of the signs, symptoms, or causes of a disease, or any other desired alteration of a biological system. For example, an "effective amount" for therapeutic uses is the amount of the composition comprising an agent as disclosed herein required to provide a clinically significant decrease in disease symptoms. An appropriate "effective" amount in any individual case is determined using techniques, such as a dose escalation study.

The terms "enhance" or "enhancing," as used herein, means to increase or prolong either in potency or duration a desired effect. Thus, in regard to enhancing the effect of therapeutic agents, the term "enhancing" refers to the ability to increase or prolong, either in potency or duration, the effect of other therapeutic agents on a system. An "enhancing-effective amount," as used herein, refers to an amount adequate to enhance the effect of another therapeutic agent in a desired system.

The terms "kit" and "article of manufacture" are used as synonyms.

The term "pharmaceutical composition" refers to a mixture of a protein as described, with other chemical components, such as carriers, stabilizers, diluents, dispersing agents, suspending agents, thickening agents, and/or excipients. The pharmaceutical composition facilitates administration of the agent (e.g. fusion protein) to an organism. Multiple techniques of administering the composition exist include, but are not limited to: intravenous, oral, aerosol, parenteral, ophthalmic, pulmonary and topical administration.

The term "subject" or "patient" encompasses mammals and non-mammals. Examples of mammals include, but are not limited to, any member of the Mammalian class: humans, non-human primates such as chimpanzees, and other apes and monkey species; farm animals such as cattle, horses, sheep, goats, swine; domestic animals such as rabbits, dogs, and cats; laboratory animals including rodents, such as rats, mice and guinea pigs, and the like. Examples of non-mammals include, but are not limited to, birds, fish and the like. In one embodiment of the methods provided herein, the mammal is a human.

The terms "treat," "treating" or "treatment," as used herein, include alleviating, abating or ameliorating a disease or condition symptoms, preventing additional symptoms, ameliorating or preventing the underlying metabolic causes of symptoms, inhibiting the disease or condition, e.g., arresting the development of the disease or condition, relieving the disease or condition, causing regression of the disease or condition, relieving a condition caused by the disease or condition, or stopping the symptoms of the disease or condition either prophylactically and/or therapeutically.

The terms "oligosaccharide," "polysaccharide" and "glycan" are used interchangeably herein to refer to carbohydrate molecules comprising more than one monosaccharide.

As used herein, the term "Fc fusion protein" refers to a polypeptide chain of an Fc region linked to a biologically active polypeptide, for example a TNF receptor polypeptide or a fragment thereof, as described herein. Fc fusion proteins may be in monomeric, dimeric or tetrameric forms as appropriate.

The terms "biologically active" and "bioactive," as used herein, indicate that a composition or compound itself has a biological effect, or that it modifies, causes, promotes, enhances, blocks, or reduces a biological effect, or which limits the production or activity of, reacts with and/or binds to a second molecule that has a biological effect. The second molecule can be, but need not be, endogenous. A "biological effect" can be but is not limited to one that stimulates or causes an immunoreactive response; one that impacts a biological process in a
cell, tissue or organism (e.g., in an animal); one that impacts a biological process in a pathogen or parasite; one that generates or causes to be generated a detectable signal; and the like. Biologically active compositions, complexes or compounds may be used in investigative, therapeutic, prophylactic, and/or diagnostic methods and compositions. Biologically active compositions, complexes or compounds act to cause or stimulate a desired effect upon a cell, tissue, organ or organism (e.g., an animal). Non-limiting examples of desired effects include modulating, inhibiting or enhancing gene expression in a cell, tissue, organ, or organism; preventing, treating or curing a disease or condition in an animal suffering therefrom; limiting the growth of or killing a pathogen in an animal infected thereby; augmenting the phenotype or genotype of an animal; stimulating a prophylactic immunoreactive response in an animal; and diagnosing a disease or disorder in an animal.

Unless stated otherwise, the percentage increase in the serum half-life of the population of a modified Fc fusion protein relative to the corresponding unmodified Fc fusion protein is calculated by the formula: Percentage increase in the serum half life = [Serum half life of the modified Fc fusion protein - Serum half life of the unmodified Fc fusion protein]/ Serum half life of the unmodified Fc fusion protein.

Unless stated otherwise the percentage increase in the sialic acid content of the modified Fc fusion protein relative to the corresponding unmodified Fc fusion protein is calculated by the formula: Percentage increase in sialic acid content = [sialic acid content of the modified Fc fusion protein - sialic acid content of the unmodified Fc fusion protein]/ sialic acid content of the unmodified Fc fusion protein. Wherein the sialic acid content of any Fc fusion protein is the moles of sialic acid present in one mole of that Fc fusion protein

### Fc fusion proteins

In some aspects, the present disclosure provides modified fusion proteins comprising an Fc domain (region) linked to a biologically active polypeptide. For example, modified fusion proteins of the disclosure are obtained by modification of pre-existing glycan structures in the receptor region of the Fc fusion protein with suitable glycosyltransferases to result in metabolically complete glycan chains.

The modified fusion proteins of the present disclosure comprise a polypeptide fused directly or indirectly to the Fc domain (Fc region) of an immunoglobulin heavy chain. In some, the Fc domain is the Fc region of human immunoglobulin G (human IgG). In some embodiments the Fc domain may correspond to human IgG1 Fc region, human IgG2 Fc region, human IgG3 Fc region or human IgG4 Fc regions.

The Fc region may be a "native sequence Fc region" comprising an amino acid sequence identical to the amino acid sequence of an Fc region found in nature. The Fc region may also be a "variant Fc region" comprising an amino acid sequence that differs from the native Fc region sequence by virtue of at least one amino acid modification. The variant Fc region may be obtained by any suitable means including addition, substitution or deletion of one or more amino acids. In some embodiments, the variant Fc region is about 70%, about 75%, about 80%, about 85%, about 90%, about 95% or about 99% homologus to the corresponding native sequence Fc region.

The polypeptide may be any polypeptide including a protein, antibody, ligand and an extracellular domain of a receptor. In some embodiments the polypeptide has a molecular weight of about 5 KDa, about 10 KDa, about 15 KDa, about 20 KDa, about 25 KDa, about 30 KDa, about 35 KDa, about 40 KDa, about 45 KDa, about 50 KDa, about 55 KDa, about 60 KDa, about 65 KDa, about 70 KDa, about 80 KDa, about 85 KDa, 90 KDa, about 95 KDa or about 100 KDa. The polypeptide may comprise about 10 amino acids, about 20 amino acids, about 30 amino acids, about 40 amino acids, about 50 amino acids, about 60 amino acids, about 70 amino acids, about 80 amino acids, about 90 amino acids, about 100 amino acids, about 110 amino acids, about 120 amino acids, about 130 amino acids, about 140 amino acids, about 150 amino acids, about 160 amino acids, about 170 amino acids, about 180 amino acids, about 190 amino acids or about 200 amino acids.

In some embodiments, the polypeptide is an extracellular domain of a receptor, such as a TNFR2 receptor protein. The polypeptide described herein includes any naturally or nonnaturally occurring TNFR2 polypeptide or fragments thereof. Also included are variant TNFR2 receptors that retain useful activity. Suitable TNFR2 variants include mutants, fragments, fusions and peptidomimetic forms. In some embodiments, the TNFR2 polypeptides include polypeptides having at least 70%, at least 75%, at least 80%, at least 90% atleast 95% or at least 99% homology to a TNFR2 polypeptide. In some embodiments the polypeptide is human TNFR2 polypeptide. In some examples, the polypeptide is the soluble portion of the tumor necrosis factor receptor type II (TNFR2), also known as p 75 TNF receptor. In some cases the modified fusion proteins of the current disclosure comprise no additional N or O glycosylation sites as compared to the naturally occurring sequence.

In some embodiments, the modified fusion protein of the invention is Etanercept. Etanercept is a recombinant homodimeric polypeptide, comprising of two, identical single chain monomeric units linked through disulfide bonding. Each monomer is composed of the soluble portion of the tumor necrosis factor receptor type II ("TNFR2" or "TNFRII"), also known as p75 TNF receptor, fused to a human IgG Fc peptide. The TNF receptor portion of Etanercept contains two N-linked glycans attached at position Asn 149 and Asn 171. In addition, the peptide is also substituted with up to 26 O-linked glycan structures attached to serine and threonine residues near the C-terminus of the receptor peptide (13 O-linked glycan structures for each monomeric unit). The Fc portion of the molecule has typical truncated N-linked glycans (G1, G2, G0) near the hinge region. The amino acid sequence of a monomeric unit of the Etanercept fusion protein is shown in Figure 7. Etanercept is currently sold under the trade name Enbrel® (Amgen Inc., Thousand Oaks, CA).

### Glycosylation of Fc fusion proteins

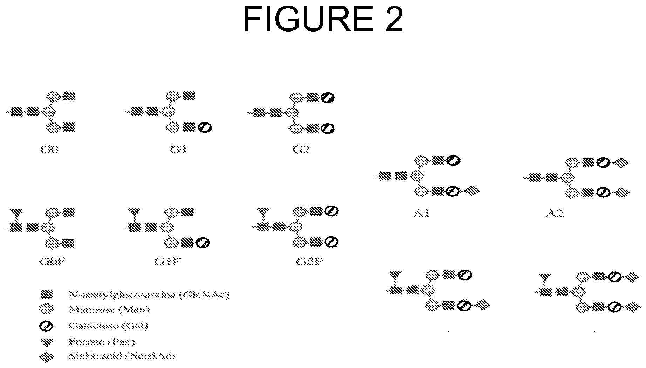

Modified Fc fusion proteins of the present disclosure may comprise a variety of glycan structures at the glycosylation sites. Examples of N-linked glycan structures include, but are not limited to G0, G0F, G1, G1F, G2, G2F, A1, A2, A1F, and A2F. In some embodiments, the N-linked glycan structure is independently any structure (G0, G0F, G1, G1F, G2, G2F, A1, A2, A1F, or A2F) shown in Figure 2. In some embodiments, a majority of N-linked glycan chains are A2 glycan chains.

Modified Fc fusion proteins obtained by the methods of the present disclosure may have an improved glycosylation profile relative to the corresponding unmodified Fc fusion protein. In some cases more than 10%, more than 15%, more than 20%, more than 25%, more than 30%, more than 35%, more than 40%, more than 45%, more than 50%, more than 55%, more than 60%, more than 65%, more than 70%, more than 75% more than 80%, more than 85% more than 90% or more than more than 95% of the available N-glycans on the receptor portion of the Fc fusion protein are metabolically extended and terminate in sialic acid.

The proteins characterized by metabolically complete glycan chains that terminate with sialic acid may exhibit significantly improved serum half-life compared to the protein with only partially completed glycan structures because the involvement of the carbohydrate receptor pathway might be minimized. In addition, more complete glycan chains may serve to increase the protein stability, further enhancing the serum half-life, even in a proteolytic environment. Extended and sialylated glycan chains may increase protein survival in the endosome and permit some material to be recycled into the circulation.

In some embodiments, the methods of the present disclosure may also affect the glycosylation profile of the available O-glycosylation sites in the Fc fusion protein. In some cases more than 10%, more than 15%, more than 20%, more than 25%, more than 30%, more than 35%, more than 40%, more than 45%, more than 50%, more than 55%, more than 60%, more than 65%, more than 70%, more than 75% more than 80%, more than 85% more than 90% or more than more than 95% of the available O-glycans on the biologically active polypeptide (e.g. receptor fragment) of the Fc fusion protein are metabolically extended and terminate in sialic acid.

In some cases more than 50%, more than 55%, more than 60%, more than 65%, more than 70%, more than 75% more than 80%, more than 85% more than 90% or more than more than 95% of the available glycosylation site (N and O) in the Fc fusion protein are metabolically extended and terminate in sialic acid.

In addition to altering the glycosylation profile in the receptor region of the Fc fusion proteins, the methods described herein can also improve the glycosylation profile in the Fc region of the Fc fusion protein. In some cases more than 50%, more than 55%, more than 60%, more than 65%, more than 70%, more than 75% more than 80%, more than 85% more than 90% or more than more than 95% of the available N-glycans in the Fc fusion protein are metabolically extended and terminate in sialic acid. In some embodiments, more than 50%, more than 55%, more than 60%, more than 65%, more than 70%, more than 75% more than 80%, more than 85% more than 90% or more than more than 95% of the available O-glycans in the Fc fusion protein are metabolically extended and terminate in sialic acid.

Modified Fc fusion proteins obtained by the methods of the present disclosure comprise pre-existing glycan structures that have been modified by suitable glycosyltransferases to give metabolically complete glycan chains. In some embodiments complete glycan chains may terminate in sialic acid, and therefore the modified Fc fusion proteins of the current disclosure may have an increased content of sialic acid (moles of sialic acid per mole of Fc fusion protein) relative to the corresponding unmodified Fc fusion proteins. In some cases the modified Fc fusion proteins have a sialic acid content at least 10%, at least 20%, at least 30%, at least 40%, at least 50%, at least 60%, at least 70%, at least 80%, at least 90%, at least 100%, at least110%, at least120%, at least130%, at least 140%, at least 150%, at least160%, at least 170%, at least 180%, at least 190%, at least 200%, at least 210%, at least 220%, at least 230%, at least 240%, at least 250%, at least 260%, at least 270%, at least 290% or at least 300% higher than the sialic acid content of the corresponding unmodified Fc fusion protein.

In some embodiments, the modified Fc fusion proteins obtained by methods of the present disclosure have an increased serum half-life relative to the corresponding unmodified Fc fusion protein. In some cases the modified Fc fusion proteins have a serum half-life at least 10%, at least 20%, at least 30%, at least 40%, at least 50%, at least 60%, at least 70%, at least 80%, at least 90%, at least 100%, at least110%, at least120%, at least130%, at least 140%, at least 150%, at least160%, at least 170%, at least 180%, at least 190%, at least 200%, at least 210%, at least 220%, at least 230%, at least 240%, at least 250%, at least 260%, at least 270%, at least 290% or at least 300% longer than the serum half-life of the corresponding unmodified Fc fusion protein. For example, in some cases the modified Etanercept obtained by the methods of the present disclosure has a serum half-life at-least 30% longer than the serum half-life of the unmodified Etanercept. The modified Etanercept obtained by the methods of the current disclosure may exhibit an improved serum half-life as compared to the unmodified Etanercept. In some cases the modified Etanercept has serum half-life greater than 4 days, greater than 5 days, greater than 6 days, greater than 7 days, greater than 8 days, greater than 9 days, greater than 10 days, greater than 11 days, greater than 12 days, greater than 13 days, greater than 14 days or greater than 15 days.

In some embodiments, modified Fc fusion proteins having increased serum half-lives are administered at lower doses and/or at lower frequency than indicated for the corresponding unmodified Fc fusion proteins. In some embodiments, dose of administration for a modified Fc fusion protein is about or less than about 95%, 90%, 80%, 70%, 60%, 50%, 40%, 30%, 25%, 20%, 15%, or 10% of the dose indicated for the corresponding unmodified Fc fusion protein. In some embodiments, frequency of administration for a modified Fc fusion protein is about or less than about 95%, 90%, 80%, 70%, 60%, 50%, 40%, 30%, 25%, 20%, 15%, or 10% of the frequency indicated for the corresponding unmodified Fc fusion protein. In some embodiments, the modified Fc fusion protein is administered to a subject about or less than about once every 1, 2, 3, 4, 5, 6, 7, 8, 9, 10, 11, 12, 13, or 14 days; about or less than about once every 1, 2, 3, 4, 5, 6, 7, or 8 weeks; or less than about 1, 2, 3, 4, 5, or 6 months.

In other embodiments, provided are Fc fusion proteins comprising other biologically active polypeptides. For example, the methods could be used to improve the glycosylation profile of Alefacept (Amevive®) which is a fusion protein combining the first extracellular domain of LFA3 (CD58) with constant regions (CH₂ and CH₃) and the hinge domain of human IgG1. Alefacept is used to control inflammation in moderate to severe psoriasis with plaque formation, where it interferes with lymphocyte activation. It is also being studied in the treatment T-cell lymphoma and T-cell non-Hodgkim lymphoma.

The methods of the current disclosure may be used to prepare Abatacept (Orencia®) with improved glycosylation profile. Abatacept is a fusion protein composed of the Fc region of the immunoglobulin IgG1 fused to the extracellular domain of CTLA-4. It is a molecule capable of binding with more avidity to CD80 (B7-1) than to CD86 (B7-2). Abatacept is a selective co-stimulation modulator as it inhibits the costimulation of T-cells. It is licensed in the United States for the treatment of rheumatoid arthrits in the case of inadequate response to anti-TNFa therapy. Although the overall structural characteristics are similar to etanercept, with both containing an IgG1 Fc peptide, abatacept differs from etanercept in that it may not act as a TNF scavenger directly, but rather it may inhibit T-lymphocyte activation by binding to CD 80 and CD 86, thereby blocking the interaction with CD28. By decreasing T cell proliferation, abatacept may inhibit the production of cytokines such as TNF alpha, interferon, gamma, and interleukin 2. The 92 kDa protein (monomer molecular weight), is similar to etanercept and has both N-linked and O-linked glycans. The peptide portion of abatacept that serves as an antigen and is recognized by CD 80 and CD 86 contains two N-linked glycosylation sites (Asn 76, and Asn 108), and a single O-linked site. In addition, the typical Fc N-linked glycosylation sites are present in the IgG1 derived Fc peptide.

The glycan structures on abatacept different significantly from the simple biantennary type structures found on etanercept. More complex triantennary and tetraantennary multibranched asialylated glycans, some lacking terminal sialic acid residues have been identified on abatacept.

The methods of the instant disclosure may also be used with Fc fusion proteins that are fusions of receptors for Anthrax toxin. The anthrax toxin receptor may comprise protective antigen, for example PA83. Such Fc Fusion proteins may be used for treating or preventing anthrax poisoning. The treatment/prevention may also be done in the absence of a vaccine.

Similarly the methods of the current invention could also be used with Rilonacept (Arcalyst®) which is a dimeric fusion protein consisting of the ligand-binding domains of the extracellular portions of the human interleukin-1 receptor component (IL-1R1) and IL-1 receptor accessory protein (IL-1RAcP) linked in-line to the Fc portion of human IgG1 that binds and neutralizes IL-1. It is currently used for the treatment of cryopyrin-associated periodic syndromes (CAPS), including familial cold autoinflammatory syndrome, Muckle-Wells syndrome and neonatal onset multisystem inflammatory disease(not approved in the U.S. on this indication).

In some further examples methods of the current invention could be used to prepare Aflibercept (Eyelea®) with improved glycosylation profile. Aflibercept is a fusion protein approved in the United States for the treatment of wet macular degeneration and metastatic colorectal cancer. It is an inhibitor of vascular endothelial growth factor (VEGF) and is designed to bind to VEGF-A, VEGF-B, and placental growth factor (PIGF).

In some further examples methods of the current invention could be used to prepare Belatacept (Nulojix®) with improved glycosylation profile. Belatacept is a fusion protein composed of the Fc fragment of a human IgG1 immunoglobulin linked to the extracellular domain of CTLA-4, which is a molecule crucial for T-cell costimulation, selectively blocking the process of T-cell activation. It is intended to provide extended graft survival while limiting the toxicity generated by standard immune suppressing regimens, such as calcineurin inhibitors.

Further non-limiting examples of polypeptides comprising an Fc region, or polypeptides to which an Fc region may be fused, for modification by methods disclosed herein include Atacicept, Romiplostim (Nplate), Abatacept (Orencia), ACE-536, Recombinant factor VIII--Fc, ACE-011 (Sotatercept), ALT-801, AMG 386, Dulaglutide, Aflibercept (Eylea), asfotase alfa, Alefacept (Amevive), CVX-096, ALXN1102/ALXN1103, CSL-654, CSL-689, F-627, FP-1039, MM-111, NGR-TNF, denileukin diftitox (Ontak), SL-401, PB-1046, Albiglutide (Syncria), PB-1023, BMN-701, DAS-181 (Fludase), ID-93/GLA-SE, VAX102 (flagellin.HuM2e), VAX125 (flagellin.HuHa), BA-210 (Cethrin), LY2189365, APG101 (Apocept), AMG 623, CNTO 528, SB 087, TRU 015, ART 621, Belatacept (BMS 224818), Briobacept (BR3-Fc), YSPSL (PSGL-Ig), ACE 031 (RAP 011), FP 1039, ALT-802, Rilonacept, Lenercept, p55, GLP-1, factor IX, factor VIIa, human serum albumin, interleukins (e.g. IL-1, IL-2, IL-3, IL-15, IL-17, IL-22), ELP, human hyaluronidase (e.g. rHuPH20), insulin, IGF-2, flagellin, Rho, GA733, CD95, nuclease protein (e.g. DNase 1, TREX 1, DNase 1L3), recombinant versions thereof, and fusion proteins comprising one or more of these polypeptides (e.g. 1, 2, 3, 4, 5, or more).

### Methods of preparing glycosylated Fc fusion proteins

In another aspect, provided herein are methods to manufacture modified Fc fusion proteins by modification of the pre-existing glycan structures in the receptor region of the Fc fusion protein with one or more suitable glycosyltransferases that give metabolically complete glycan chains. In some embodiments, the method comprises treating Fc fusion proteins (produced, e.g. by expression in a suitable eukaryotic or prokaryotic cell line) with a suitable enzyme to chemically modify the pre-existing glycan chains to result in metabolically complete glycan chains. In some embodiments, the enzyme may be a glycosyltransferase including galactosyltransferase. Non-limiting examples of galactosyltransferases include β4-galactosyltransferase-I (β4-GalT-I), β4-galactosyltransferase-II (β4-GalT-II), β4-galactosyltransferase-III (β4-GalT-III), β4-galactosyltransferase-IV(β4-GalT-IV), β4-galactosyltransferase-V(β4-GalT-V), β4-galactosyltransferase-VI(β4-GalT-VI), β4-galactosyltransferase-VII (β4-GalT-VII), β3-galactosyltransferase-I (β3-GalT-I), β3-galactosyltransferase-II (β3-GalT-II), β3-galactosyltransferase-III (β3-GalT-III), β3-galactosyltransferase-IV (β3-GalT-IV), β3-galactosyltransferase-V (β3-GalT-V), α3-galactosyltransferase (α3-GalT), α1-3 galactosyltransferase (α1-3 GalT), α1-4 galactosyltransferase (α1-4 GalT), α1-6 galactosyltransferase (α1-6 GalT),β1-3 galactosyltransferase (β1-3 GalT), β1-4 galactosyltranferase (β1-4 GalT), or β1-4/α1-3 galactosyltranferase (β1-4/α1-3 GalT).

In some embodiments, the enzyme is a human galactosyltransferase, for example human β4-GalT-I, human β4-GalT-II, human β4-GalT-III, human β4-GalT-IV, human β4-GalT-V, human β4-GalT-VI, human β4-GalT-VII, human β3-GalT-I, human β3-GalT-II, β3-GalT-III, human β3-GalT-IV, human β3-GalT-V, α3-GalT, or recombinant versions thereof. In some embodiments, the enzyme is β4-GalT-I. In some embodiments, the enzyme is human β4-GalT. In some embodiments, the enzyme is bovine β4-GalT.

In some embodiments, the enzyme is a bacterial galactosyltransferase, for example S. dysenteriae α1-3 GalT (e.g. RfpB), K. pneumonia α1-3 GalT (e.g. RfbF), C. coli α1-3 GalT (e.g. RfbF), S. marcescens α1-3 GalT (e.g. RfbF), S. typhimurium α1-3 GalT (e.g. RgaI), N. gonorrhoeae α1-4 GalT (e.g. LgtC), N. meningitidis α1-4 GalT (e.g. LgtC), E. amylovora α1-6 GalT (e.g. AmsD), E. coli α1-6 GalT (e.g. RfaB), S. typhimurium α1-6 GalT (e.g. RfaB), R. leguminosarum α1-6 GalT (e.g. LpcA), C. jejuni β1-3 GalT (e.g. CgtB, WlaN), N. gonorrhoeae β1-4 GalT (e.g. LgtB, LgtE), N. meningitidis β1-4 GalT (e.g. LgtB, LgtE), S. pneumoniae β1-4 GalT (e.g. Cps14G, Cps14J), E. coli β1-4 GalT (e.g. WaaX), H. pylori β1-4 GalT (e.g. HpgalT), N. meningitidis β1-4/α1-3 GalT (e.g. PglA), or recombinant versions thereof.

In some embodiments, the sialic acid is linked to the inner sugar residue galactose (Gal) via α2,6- or α2,3-linkage. In some embodiments, the sialic acid is linked to galactosamine or N-acetylgalactosamine (GalNAc) via α2,6- or α2,3-linkage. Alternatively, sialic acid may be linked to the C8 position of another sialic acid residue.

Transfer of sialic acid may be achieved via use of one or more sialyltransferase. Non-limiting examples of sialyltransferases that may be used in the methods of current disclosure include ST3-galactosideα-2,3-sialyltransferase 1 (ST3GAL1), ST3-galactosideα-2,3-sialyltransferase 2 (ST3GAL2), ST3-galactosideα-2,3-sialyltransferase 3 (ST3GAL3), ST3-galactosideα-2,3-sialyltransferase 4 (ST3GAL4), ST3-galactosideα-2,3-sialyltransferase 5 (ST3GAL5), ST3-galactosideα-2,3-sialyltransferase 6 (ST3GAL6), ST6-βgalactosamideα-2,6-sialyltransferase 1 (ST6GAL1), ST6-βgalactosamideα-2,6-sialyltransferase 2 (ST6GAL2), ST6 (α-N-acetyl-neuraminyl-2,3-β-galactosyl-1,3)-N-acetylgalactosaminide α-2,6-sialyltransferase 1 (ST6GALNAC1), (α-N-acetyl-neuraminyl-2,3-β-galactosyl-1,3)-N-acetylgalactosaminide α-2,6-sialyltransferase 2 (ST6GALNAC2), ST6 (α-N-acetyl-neuraminyl-2,3-β-galactosyl-1,3)-N-acetylgalactosaminide α-2,6-sialyltransferase 3 (ST6GALNAC3), ST6 (α-N-acetyl-neuraminyl-2,3-β-galactosyl-1,3)-N-acetylgalactosaminide α-2,6-sialyltransferase 4 (ST6GALNAC4), ST6 (α-N-acetyl-neuraminyl-2,3-β-galactosyl-1,3)-N-acetylgalactosaminide α-2,6-sialyltransferase 5 (ST6GALNAC5), ST6 (α-N-acetyl-neuraminyl-2,3-β-galactosyl-1,3)-N-acetylgalactosaminide α-2,6-sialyltransferase 6 (ST6GALNAC6), ST8 α-N-acetyl-neuraminide α-2,8-sialyltransferase 1 (ST8SIA1), ST8 α-N-acetyl-neuraminide α-2,8-sialyltransferase 2 (ST8SIA2), ST8 α-N-acetyl-neuraminide α-2,8-sialyltransferase 3 (ST8SIA3), ST8 α-N-acetyl-neuraminide α-2,8-sialyltransferase 1 (ST8SIA4), ST8 α-N-acetyl-neuraminide α-2,8-sialyltransferase 5 (ST8SIA5), ST8 α-N-acetyl-neuraminide α-2,8-sialyltransferase 6 (ST8SIA6), α 2-8-polysialyltransferase, α 2-9-polysialyltransferase, α 2-8/9-polysialyltransferase, α 2-3-sialyltransferase, α 2-6-sialyltransferase, α 2-8-sialyltransferase, α 2-3/6-sialyltransferase, α 2-3/8-sialyltransferase, and recombinant versions thereof.

In some embodiments, the one or more sialyltransferases includes one or more mammalian (e.g. rat, mouse, pig, porcine) sialyltransferase, for example mammalian SIAT4C, mammalian SIAT9, mammalian ST3GAL1, mammalian ST3GAL2, mammalian ST3GAL3, mammalian ST3GAL4, mammalian ST3GAL5, mammalian ST3GAL6, mammalian ST3GalIII, mammalian ST6GAL1, mammalian ST6GAL2, mammalian ST6Gal, mammalian ST8SIA1, mammalian ST8SIA2, mammalian ST8SIA3, mammalian ST8SIA4, mammalian ST8SIA5, mammalian ST8SIA6, mammalian ST8Sia. In some embodiments the sialyltransferase comprises rat N-2,3 sialyltransferase, porcine O-2,3 sialyltransferase, rat O-2,3 sialyltransferase, or recombinant versions thereof.

In some embodiments, the one or more sialyltransferase includes one or more human sialyltransferase for example human SIAT4C, human SIAT9, human ST3GAL1, human ST3GAL2, human ST3GAL3, human ST3GAL4, human ST3GAL5, human ST3GAL6, human ST3GalIII, human ST6GAL1, human ST6GAL2, human ST6Gal, human ST8SIA1, human ST8SIA2, human ST8SIA3, human ST8SIA4, human ST8SIA5, human ST8SIA6, human ST8Sia, or recombinant versions thereof. In some embodiments, the enzyme is human ST6GAL1.

In some embodiments, the one or more sialyltransferase includes one or more bacterial sialyltransferase for example *E. coli* α2-8-polysialyltransferase (e.g. NeuS), *E. coli* α2-8/9-polysialyltransferase, *N. meningitidis* α2-8-polysialyltransferase (e.g. SiaD), *N. meningitidis* α2-9-polysialyltransferase (e.g. *synE*), *C. jejuni* α2-3-sialyltransferase, *C. jejuni* α2-3/8-sialyltransferase (e.g. Cst-II), *P. multocida* α2-3-sialyltransferase (e.g. PmST3), *H. influenzae* α2-3-sialyltransferase (e.g. *lic3A*), *H. influenzae* α2-3/8-sialyltransferase (e.g. Lic3B), *N. meningitidis* α2-3/6-sialyltransferase (e.g. Lsts), *N. gonorrheae* α2-3/6-sialyltransferase (e.g. Lsts), *H. influenzae* α2-3-sialyltransferase (e.g. LsgB), *H. ducreyi* α2-3-sialyltransferase (e.g. Lst), *P. multocida* α2-3-sialyltransferase (e.g. PmST2), *S. agalactiae* α2-3-sialyltransferase (e.g. CpsK), *P. multocida* α2-3/6-sialyltransferase (e.g. PmST1), *H. ducreyi* α2-3-sialyltransferase (e.g. Hd2,3ST), *P. damselae* α2-6-sialyltransferase (e.g. Pd2,6ST), *P. leiognathi* α2-6-sialyltransferase, *P. phosphoreum* α2-3-sialyltransferase, *Vibrio* α2-3-sialyltransferase, *Photobacterium JT-ISH-224* α2-3-sialyltransferase, *Photobacterium JT-ISH-224* α2-6-sialyltransferase, or recombinant versions thereof. In some embodiments, the enzyme is Photobacterium damselae α2-6-sialyltransferase. In some embodiments human ST6GAL1 is used. In some embodiments a combination of C. jejuni α2-3/8-sialyltransferase (e.g. Cst-II) is used.

In some embodiments a combination of a sialyltransferase and galactosyltransferase may be employed. Combinations may in include 1, 2, 3, 4, 5, or more galactosyltransferases combined with 1, 2, 3, 4, 5, or more sialyltransferases. Galactosyltransferases and sialyltransferases used in combination may include any Galactosyltransferases and sialyltransferases described herein. For example one or more of sialyltransferase selected from a group comprising ST3GAL1, ST3GAL2, ST3GAL3, ST3GAL4, ST3GAL5, ST3GAL6, ST6GAL1, ST6GAL2, ST6GALNAC1, ST6GALNAC2, ST6GALNAC3, ST6GALNAC4, ST6GALNAC5, ST6GALNAC6, ST8SIA1, ST8SIA2, ST8SIA3, ST8SIA4, ST8SIA5, ST8SIA6, α 2-8-polysialyltransferase,α2-9-polysialyltransferase,α2-8/9-polysialyltransferase,α2-3-sialyltransferase,α2-6-sialyltransferase,α2-8-sialyltransferase,α2-3/6-sialyltransferase, orα2-3/8-sialyltransferase may be used in combination with one or more galactosyltransferase selected from a group comprising β4-GalT-I, β4-GalT-II, β4-GalT-III, β4-GalT-IV, β4-GalT-V, β4-GalT-VI, β4-GalT-VII, β3-GalT-I, β3-GalT-II, β3-GalT-III, β3-β3-GalT-IV, β3-GalT-V, α3-GalT, α1-3 GalT, α1-4 GalT, α1-6 GalT, β1-3 GalT, β1-4 GalT, or β1-4/α1-3 GalT. In some embodiments, the enzyme is Photobacterium damselae α2-6-sialyltransferase (e.g. Pd2,6ST) and bovine β4-GalT is used. In some embodiments a combination of human ST6GAL1 and human β4-GalT is used. In some embodiments a combination of C. jejuni α2-3/8-sialyltransferase (e.g. Cst-II) and human β4-GalT is used.

In some embodiments, the methods of the present disclosure may be performed *in vivo.* The manufacture of the glycosylated Fc fusion peptides according to the methods of the present disclosure could be carried out in any available cell line. A wide variety of cell lines for tissue culture are known in the art. Examples of cell lines include, but are not limited to, C8161, CCRF-CEM, MOLT, mIMCD-3, NHDF, HeLa-S3, Huh1, Huh4, Huh7, HUVEC, HASMC, HEKn, HEKa, MiaPaCell, Panc1, PC-3, TF1, CTLL-2, C1R, Rat6, CV1, RPTE, A10, T24, J82, A375, ARH-77, Calu1, SW480, SW620, SKOV3, SK-UT, CaCo2, P388D1, SEM-K2, WEHI-231, HB56, TIB55, Jurkat, J45.01, LRMB, Bcl-1, BC-3, IC21, DLD2, Raw264.7, NRK, NRK-52E, MRC5, MEF, Hep G2, HeLa B, HeLa T4, COS, COS-1, COS-6, COS-M6A, BS-C-1 monkey kidney epithelial, BALB/ 3T3 mouse embryo fibroblast, 3T3 Swiss, 3T3-L1, 132-d5 human fetal fibroblasts; 10.1 mouse fibroblasts, 293-T, 3T3, 721, 9L, A2780, A2780ADR, A2780cis, A172, A20, A253, A431, A-549, ALC, B16, B35, BCP-1 cells, BEAS-2B, bEnd.3, BHK-21, BR 293, BxPC3, C3H-10T1/2, C6/36, Cal-27, CHO, CHO-7, CHO-IR, CHO-K1, CHO-K2, CHO-T, CHO Dhfr -/-, COR-L23, COR-L23/CPR, COR-L23/5010, COR-L23/R23, COS-7, COV-434, CML T1, CMT, CT26, D17, DH82, DU145, DuCaP, EL4, EM2, EM3, EMT6/AR1, EMT6/AR10.0, FM3, H1299, H69, HB54, HB55, HCA2, HEK-293, HeLa, Hepalclc7, HL-60, HMEC, HT-29, Jurkat, JY cells, K562 cells, Ku812, KCL22, KG1, KYO1, LNCap, Ma-Mel 1-48, MC-38, MCF-7, MCF-10A, MDA-MB-231, MDA-MB-468, MDA-MB-435, MDCK II, MDCK II, MOR/0.2R, MONO-MAC 6, MTD-1A, MyEnd, NCI-H69/CPR, NCI-H69/LX10, NCI-H69/LX20, NCI-H69/LX4, NIH-3T3, NALM-1, NW-145, OPCN / OPCT cell lines, Peer, PNT-1A / PNT 2, RenCa, RIN-5F, RMA/RMAS, Saos-2 cells, Sf-9, SkBr3, T2, T-47D, T84, THP1 cell line, U373, U87, U937, VCaP, Vero cells, WM39, WT-49, X63, YAC-1, YAR, and transgenic varieties thereof. Cell lines are available from a variety of sources known to those with skill in the art (see, e.g., the American Type Culture Collection (ATCC) (Manassus, Va.)). In some embodiments, glycosylation is performed in mammalian cell lines including but not limited to CHO cell line (e.g. CHO-DG44, CHO-DuxB11, CHO-S, CHO-K1, and CHO-SV), NS0 cell line, CSO cell line, mouse myeloma SP2/0 cell line, human HT-1080 cell line, human lymphoblastoid cell line, and HEK236 cell line. In other embodiments, modified Fc fusion peptides are expressed in non-mammalian cell lines including insect cells, microbial cells, and plant cells.

The methods of production of Fc fusion proteins disclosed herein may allow large scale production of Fc fusion proteins with consistent glycosylation profile. In some embodiments, the methods provide access to up to 0.5 mg, 0.75 mg, 1.0 mg, 1.25 mg, 1.50 mg, 1.75 mg, 2.0 mg, 2.25 mg, 2.5 mg, 2.75 mg, 3.0 mg, 3.25 mg, 3.5 mg, 3.75 mg, 4.0 mg, 4.25 mg, 4.50 mg, 4.75 mg, 5.0 mg, 5.25 mg, 5.5 mg, 5.75 mg, 6.0 mg, 6.25 mg, 6.5 mg, 6.75 mg, 7.0 mg, 7.25 mg, 7.5 mg, 7.75 mg, 8.0 mg, 8.25 mg, 9.0 mg, 9.25 mg, 9.5 mg, 9.75 mg, 10 mg, 20 mg, 30 mg, 50 mg, 100 mg, 200 mg, 500 mg, or 1g of modified Fc fusion proteins. For example in some cases the methods of the present disclosure may be used to manufacture up to 5 mg of the modified Etanercept. In some further embodiments the methods of the present disclosure may also be used to manufacture greater than 10 mg of modified fusion proteins. In some embodiments, methods of the present disclosure are used to produce at least 5mg, 10mg, 15mg, 20mg, 25mg, 30mg, 35mg, 40mg, 45mg, 50mg, or more of modified Fc fusion proteins.

### Methods of use

In another aspect, the present disclosure provides a method for treatment of disease in a subject in need thereof. The method comprises administering to the subject a therapeutically effective amount of modified Fc fusion protein, wherein the modified Fc fusion protein is prepared by treatment of an initial population of Fc fusion with suitable glycosyltransferases, such that a high fraction of the resulting Fc fusion proteins comprise metabolically complete glycan chains.

In various embodiments, the patients treated with the modified Fc fusion proteins, or candidate patients to be treated by the modified Fc fusion proteins of the disclosure, may be mammals, including rodents, primates, and humans.

In one embodiment, the disorders to be treated using Fc fusion proteins of the invention are characterized by abnormal or excessive TNF-α levels. Increased levels of TNF-alpha have been implicated in the progression of numerous medical disorders. For example, patients with chronic heart failure show elevated levels of serum TNF-α, which are correlated with the severity of disease (see, for example, Levine et al., N Eng J Med 323:236-241, 1990). A variety of other diseases are associated with elevated levels of TNF-alpha (see, for example, Feldman et al., Transplantation Proceedings 30:4126-4127, 1998). It has been suggested that the suppression of TNF-alpha might be beneficial in patients suffering from disorders characterized by abnormal or excessive TNF-alpha expression.

In some embodiments, the disclosure provides methods for treatment of a variety of TNF mediated disorders (non-limiting examples of which include chronic heart failure, abdominal aortic aneurism, familial combined hyperlipidemia (FCH), cervicogenic headache, idiopathic bronchiectasis, adult and juvenile rheumatoid arthritis, spondylo-arthropathy, ankylosing spondylitis, psoriatic arthritis, chronic Lyme arthritis, Crohn's disease, plaque psoriasis, atherosclerosis, myocardial infarction, heart failure, myocarditis, cardiac, allograft rejection, chronic bronchitis, chronic obstructive pulmonary disease, acute respiratory distress syndrome, asthma, ischaemic renal injury, renal transplant rejection, glomerulonephritis, therapy-resistant sarcoidosis, inflammatory myopathies, Behcet disease, and inflammatory eye disease) by administering to the subject in need thereof a therapeutically effective amount of a TNF receptor fusion protein modified according to the methods of the current disclosure.

In some embodiments, the disclosure provides methods for treatment of a variety of autoimmune diseases (non-limiting examples of which include adult and juvenile rheumatoid arthritis, psoriatic arthritis, plaque psoriasis, ankylosing spondylitis, systemic and multiple sclerosis, lupus erthematosus (systemic, discoid, drug-induced and neonatal), sarcoidosis, severe and refractory myasthenia gravis, and immune thrombocytopenic purpura) by administering to the subject in need thereof a therapeutically effective amount of modified Fc fusion protein according to the methods of the current disclosure.

In some embodiments, the disclosure provides methods for treatment of a variety of blood disorders (non-limiting examples of which include anemia, paroxysmal nocturnal hemoglobinuria, hemophilia A and B, von Willebrand disease and other blood coagulation disorders, disseminated intravascular coagulation, vasculitis, beta-thalassemia, sickle cell anemia, idiopathic CD4-positive lymphocytopenia, congential factor XIII deficiency, hemorrhage and surgical blood loss) by administering to the subject in need thereof a therapeutically effective amount of modified Fc fusion protein according to the methods of the current disclosure.

In some embodiments, the disclosure provides methods for treatment of a variety of cancers (non-limiting examples of which include breast cancer such as mammary ductal carcinoma, medullary carcinomas, colloid carcinomas, tubular carcinomas, and inflammatory breast cancer; ovarian cancer; uterine cancer; cervical cancer such as squamous cell carcinoma and other adenocarcinomas; prostate cancer; pancreatic cancer such as pancreatic epithelioid carcinoma and adenocarcinoma; bladder cancer such as a transitional cell carcinoma; leukemia such as chronic and acute myeloid leukemia (AML), chronic and acute lymphocytic leukemia, hairy cell leukemia, myelodysplasia, myeloproliferative disorders, mastocytosis, multiple myeloma (MM), and myelodysplastic syndrome (MDS); bone cancer; lung cancer such as non-small cell lung cancer (NSCLC) and small cell lung cancer (SCLC); skin cancer such as basal cell carcinoma, melanoma, squamous cell carcinoma and actinic keratosis; eye retinoblastoma; cutaneous and intraocular melanoma; kidney cancer; thyroid cancer such as papillary, follicular, medullary and anaplastic; AIDS-related lymphoma such as diffuse large B-cell lymphoma, B-cell immunoblastic lymphoma and small non-cleaved cell lymphoma; Kaposi's Sarcoma; viral-induced cancers including hepatitis B virus (HBV), hepatitis C virus (HCV), and hepatocellular carcinoma; human lymphotropic virus-type 1 (HTLV-1) and T-cell leukemia; human papilloma virus (HPV) and cervical cancer; brain cancers such as gliomas (astrocytoma, oligodendroglioma, and ependymoma), glioblastoma, acoustic neuroma, malignant peripheral nerve sheath tumor (MPNST), neurofibromas and schwannomas, malignant fibrous cytoma, malignant fibrous histiocytoma, malignant meningioma, malignant mesothelioma, and malignant mixed Müllerian tumor; oral cavity cancer such as hypopharyngeal cancer, laryngeal cancer, nasopharyngeal cancer, and oropharyngeal cancer; stomach cancer such as lymphomas, gastric stromal tumors, and carcinoid tumors; testicular cancer such as germ cell tumors (GCTs), gonadal stromal tumors, Leydig cell tumors and Sertoli cell tumors; thymus cancer such as thymomas, thymic carcinomas, Hodgkin disease, non-Hodgkin lymphomas; rectal cancer; colon cancer; liver cancer; renal cancer; gastric cancer; esophageal cancer; vulvovaginal cancer; endometrial cancer; cervical cancer; head and neck cancer; mesothelioma; hematological malignancies; and other lipomas and solid tumors) by administering to the subject in need thereof a therapeutically effective amount of modified Fc fusion protein according to the methods of the current disclosure.

In some embodiments, the disclosure provides methods for treatment of a variety of cardiovascular diseases (non-limiting examples of which include atherosclerosis, coronary artery disease, ischemic heart disease (also known as myocardial ischemia), myocardial infarction, ischemic heart failure, stroke, ischemic heart and organ disorders, hypercholesterolemia, peripheral arterial and vascular disease, congestive heart failure, atrial fibrillation, dilated cardiomyopathy, vascular restinosis, advanced heart failure, essential hypertension, and other heart disorders) by administering to the subject in need thereof a therapeutically effective amount of modified Fc fusion protein according to the methods of the current disclosure.

In some embodiments, the disclosure provides methods for treatment of a variety of digestive disorders (non-limiting examples of which include pouchitis, distal and proximal ulcerative colitis, celiac disease, inflammatory bowel disease (IBD), Crohn's disease, and gastrointestinal reflux disease (GERD)) by administering to the subject in need thereof a therapeutically effective amount of modified Fc fusion protein according to the methods of the current disclosure.

In some embodiments, the disclosure provides methods for treatment of a variety of eye conditions (non-limiting examples of which include optic neuritis, age-related macular degeneration, diabetic macular edema, uveitis (anterior, intermediate or posterior), Stargardt's disease, retinitis pigmentosa, optic neuropathy, and neuromyelitis optica) by administering to the subject in need thereof a therapeutically effective amount of modified Fc fusion protein according to the methods of the current disclosure.

In some embodiments, the disclosure provides methods for treatment of a variety of genetic disorders (non-limiting examples of which include adrenoleucodystrophy, alpha-1-antitrypsin deficiency, lysosomal storage disease, transthyretin (TTR)-mediated amyloidosis, hypophosphatasia, mucopolysaccharidosis, glycogen storage disease, familial adenomatous polyposis, muscular dystrophy, adenosine deaminase severe combined immune deficiency (ADA-SCID), Niemann-Pick disease, Pelizaeus-Merzbacher disease, familial amyloid polyneuropathy, cryopyrin--associated periodic syndrome, X-linked hypophosphatemic rickets, familial hypercholesterolemia, leber congenital amaurosis, Neuronal ceroid lipofuscinoses (NCLS), phenylketonuria, Fabry's disease, hereditary angioedema, Wolman disease (lysosomal acid lipase deficiency), usher syndrome, and Wiskott-Aldrich syndrome) by administering to the subject in need thereof a therapeutically effective amount of modified Fc fusion protein according to the methods of the current disclosure.

In some embodiments, the disclosure provides methods for treatment of a variety of infectious agents and conditions (non-limiting examples of which include Clostridium difficile, enterotoxigenic Escherichia coli (ETEC), diphtheria, pertussis, tetanus, human immunodeficiency virus (HIV), tuberculosis, influenza A virus, human papillomavirus (HPV), respiratory syncytial virus (RSV), anthrax, hepatitis B virus (HBV), hepatitis B virus (HCV) ; cytomegalovirus (CMV) virus, Marburg virus, Ebola virus, sepsis, meningococcal disease, botulism, chikungunya virus, cholera, dengue fever, poliomyelitis, haemophilus influenza, malaria, rabies, varicella zoster virus, pneumonia, West Nile virus, herpes simplex virus, smallpox, pseudomonas, Lyme disease, measles, mumps, rubella, meningitis, onychomycosis, candidiasis, Staphylococcus aureus, norovirus, yersinia, ricin poisoning, rotavirus hemolytic uremic syndrome (HUS), streptococcus, tularemia, visceral leishmaniasis, and herpes zoster) by administering to the subject in need thereof a therapeutically effective amount of modified Fc fusion protein according to the methods of the current disclosure.

In some embodiments, the disclosure provides methods for treatment of a variety of muscoskeletal disorders (non-limiting examples of which include osteoarthritis, metabolic bone diseases, muscular atrophy, psoriatic arthritis, osteoporosis, inclusion body myositis, acute cartilage injury, spondylarthritis, gouty arthritis, intervertebral disc displacement, synovitis, spinal fusion, and spondylosis) by administering to the subject in need thereof a therapeutically effective amount of modified Fc fusion protein according to the methods of the current disclosure.

In some embodiments, the disclosure provides methods for treatment of a variety of neurological disorders (non-limiting examples of which include Alzheimer's Disease (AD), Becker muscular dystrophy, chronic pain, postherpetic neuralgia, migraines, spinal cord injury, postoperative pain, motor neuron disease. amyotrophic lateral sclerosis (ALS), Parkinson's disease, diabetic neuropathy, and post-stroke spasticity) by administering to the subject in need thereof a therapeutically effective amount of modified Fc fusion protein according to the methods of the current disclosure.

In some embodiments, the disclosure provides methods for treatment of a variety of respiratory disorders (non-limiting examples of which include acute lung injury, adult respiratory distress syndrome, asthma, chronic obstructive pulmonary disease (COPD), pulmonary fibrosis, allergic asthma, allergic rhinitis, allergic rhinoconjunctivitis, and eosinophilic esophagitis) by administering to the subject in need thereof a therapeutically effective amount of modified Fc fusion protein according to the methods of the current disclosure.

In some embodiments, the disclosure provides methods for treatment of a variety of dermatological diseases (non-limiting examples of which include psoriasis, acne vulgaris, leg ulcer, hidradenitis suppurativa, dermal scarring, atopic dermatitis, lateral canthal lines, chronic wounds, pachyonychia congenital, and chronic idiopathic urticaria) by administering to the subject in need thereof a therapeutically effective amount of modified Fc fusion protein according to the methods of the current disclosure.

In some embodiments, the disclosure provides methods for facilitating a variety of transplantation conditions (non-limiting examples of which include prevention of organ transplant rejection, graft-versus-host disease, and delayed graft function; immunosuppression; hematopoietic stem cell transplantation; facilitate engraftment in core blood transplants; bone marrow regeneration; and presensitization for organ transplant) by administering to the subject in need thereof a therapeutically effective amount of modified Fc fusion protein according to the methods of the current disclosure.

In some embodiments, the disclosure provides methods for treatment of a variety of other disorder and conditions (non-limiting examples of which include acute kidney injury, overactive bladder with urinary incontinence, cognition disorders following treatment of malignant glioma, chronic fatigue syndrome, lupus nephritis, idiopathic overactive bladder, benign prostatic hyperplasia, autistic disorder, acute radiation syndrome, liver fibrosis, myelofibrosis, interstitial cystitis, Cushing syndrome chronic kidney disease, diabetic nephropathy, methamphetamine abuse, lipodystrophy, hypoparathyroidism, cystectomy, acute renal failure, hyperhidrosis, somatropic deficiency, biliary cirrhosis, antibiotic-associate diarrhea, and Peyronie's disease) by administering to the subject in need thereof a therapeutically effective amount of modified Fc fusion protein according to the methods of the current disclosure.

The agent administered is dependent on the subject being treated, the severity of the disorder or condition, the rate of administration, the disposition of the agent and the discretion of the prescribing physician. In various embodiments the amount of modified fusion proteins administered is less than, greater than, or equal to the amount of the corresponding un-modified fusion protein that may be used to treat a similar disorder or condition.

### Pharmaceutical Compositions and Formulations

In some embodiments, the agents (e.g. fusion proteins) described herein are formulated into pharmaceutical compositions. In specific embodiments, pharmaceutical compositions are formulated in a conventional manner using one or more physiologically acceptable carriers comprising excipients and auxiliaries which facilitate processing of the active agents into preparations which can be used pharmaceutically. Proper formulation is dependent upon the route of administration chosen. Any pharmaceutically acceptable techniques, carriers, and excipients are used as suitable to formulate the pharmaceutical compositions described herein: Remington: The Science and Practice of Pharmacy, Nineteenth Ed (Easton, Pa.: Mack Publishing Company, 1995); Hoover, John E., Remington's Pharmaceutical Sciences, Mack Publishing Co., Easton, Pennsylvania 1975; Liberman, H.A. and Lachman, L., Eds., Pharmaceutical Dosage Forms, Marcel Decker, New York, N.Y., 1980; and Pharmaceutical Dosage Forms and Drug Delivery Systems, Seventh Ed. (Lippincott Williams & Wilkins1999).

Disclosed herein are pharmaceutical compositions comprising an agent as described herein, and a pharmaceutically acceptable diluent(s), excipient(s), or carrier(s). In certain embodiments, the agents described are administered as pharmaceutical compositions in which agents of the invention are mixed with other active ingredients as a combination therapy. In specific embodiments, the pharmaceutical compositions include one or more agents of the invention.

A pharmaceutical composition, as used herein, refers to a mixture of an agent of the invention, with other chemical components, such as carriers, stabilizers, diluents, dispersing agents, suspending agents, thickening agents, and/or excipients. In certain embodiments, the pharmaceutical composition facilitates administration of the agent to an organism. In some embodiments, practicing the methods of treatment or use provided herein, therapeutically effective amounts of agents of the invention, provided herein are administered in a pharmaceutical composition to a mammal having a disease or condition to be treated. In specific embodiments, the mammal is a human. In certain embodiments, therapeutically effective amounts vary depending on the severity of the disease, the age and relative health of the subject, the potency of the agent used and other factors. The agents described herein are used singly or in combination with one or more therapeutic agents as components of mixtures.

In one embodiment, one or more agents of the invention is formulated in an aqueous solutions. In specific embodiments, the aqueous solution is selected from, by way of example only, a physiologically compatible buffer, such as Hank's solution, Ringer's solution, or physiological saline buffer. In other embodiments, one or more agent of the invention, is formulated for transmucosal administration. In specific embodiments, transmucosal formulations include penetrants that are appropriate to the barrier to be permeated. In still other embodiments wherein the agents described herein are formulated for other parenteral injections, appropriate formulations include aqueous or nonaqueous solutions. In specific embodiments, such solutions include physiologically compatible buffers and/or excipients.

In another embodiment, agents described herein are formulated for oral administration. Agents described herein are formulated by combining the active agents with, e.g., pharmaceutically acceptable carriers or excipients. In various embodiments, the agents described herein are formulated in oral dosage forms that include, by way of example only, tablets, powders, pills, dragees, capsules, liquids, gels, syrups, elixirs, slurries, suspensions and the like.

In certain embodiments, pharmaceutical preparations for oral use are obtained by mixing one or more solid excipient with one or more of the agents described herein, optionally grinding the resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as: for example, maize starch, wheat starch, rice starch, potato starch, gelatin, gum tragacanth, methylcellulose, microcrystalline cellulose, hydroxypropylmethylcellulose, sodium carboxymethylcellulose; or others such as: polyvinylpyrrolidone (PVP or povidone) or calcium phosphate. In specific embodiments, disintegrating agents are optionally added. Disintegrating agents include, by way of example only, cross-linked croscarmellose sodium, polyvinylpyrrolidone, agar, or alginic acid or a salt thereof such as sodium alginate.

In one embodiment, dosage forms, such as dragee cores and tablets, are provided with one or more suitable coating. In specific embodiments, concentrated sugar solutions are used for coating the dosage form. The sugar solutions, optionally contain additional components, such as by way of example only, gum arabic, talc, polyvinylpyrrolidone, carbopol gel, polyethylene glycol, and/or titanium dioxide, lacquer solutions, and suitable organic solvents or solvent mixtures. Dyestuffs and/or pigments are also optionally added to the coatings for identification purposes. Additionally, the dyestuffs and/or pigments are optionally utilized to characterize different combinations of active agent doses.

In certain embodiments, therapeutically effective amounts of at least one of the agents described herein are formulated into other oral dosage forms. Oral dosage forms include push-fit capsules made of gelatin, as well as soft, sealed capsules made of gelatin and a plasticizer, such as glycerol or sorbitol. In specific embodiments, push-fit capsules contain the active ingredients in admixture with one or more filler. Fillers include, by way of example only, lactose, binders such as starches, and/or lubricants such as talc or magnesium stearate and, optionally, stabilizers. In other embodiments, soft capsules, contain one or more active agent that is dissolved or suspended in a suitable liquid. Suitable liquids include, by way of example only, one or more fatty oil, liquid paraffin, or liquid polyethylene glycol. In addition, stabilizers are optionally added.

In other embodiments, therapeutically effective amounts of at least one of the agents described herein are formulated for buccal or sublingual administration. Formulations suitable for buccal or sublingual administration include, by way of example only, tablets, lozenges, or gels. In still other embodiments, the agents described herein are formulated for parental injection, including formulations suitable for bolus injection or continuous infusion.

In some embodiments, formulations for injection are provided. Such formulations are, for example, presented in unit dosage form (e.g., in ampoules) or in multi-dose containers. Preservatives are, optionally, added to the injection formulations. In other embodiments, the pharmaceutical composition of the invention are formulated in a form suitable for parenteral injection as a sterile suspensions, solutions or emulsions in oily or aqueous vehicles. Parenteral injection formulations optionally contain formulatory agents such as suspending, stabilizing and/or dispersing agents. In specific embodiments, pharmaceutical formulations for parenteral administration include aqueous solutions of the active agents in water-soluble form. In additional embodiments, suspensions of the active agents are prepared as appropriate oily injection suspensions. Suitable lipophilic solvents or vehicles for use in the pharmaceutical compositions described herein include, by way of example only, fatty oils such as sesame oil, or synthetic fatty acid esters, such as ethyl oleate or triglycerides, or liposomes. In certain specific embodiments, aqueous injection suspensions contain substances which increase the viscosity of the suspension, such as sodium carboxymethyl cellulose, sorbitol, or dextran. Optionally, the suspension contains suitable stabilizers or agents which increase the solubility of the agents to allow for the preparation of highly concentrated solutions. Alternatively, in other embodiments, the active ingredient is in powder form for constitution with a suitable vehicle, e.g., sterile pyrogen-free water, before use.

In still other embodiments, the agents of the invention are administered topically. The agents described herein are formulated into a variety of topically administrable compositions, such as solutions, suspensions, lotions, gels, pastes, medicated sticks, balms, creams or ointments. Such pharmaceutical compositions optionally contain solubilizers, stabilizers, tonicity enhancing agents, buffers and preservatives.

In yet other embodiments, the agents of the invention, are formulated for transdermal administration. In specific embodiments, transdermal formulations employ transdermal delivery devices and transdermal delivery patches and can be lipophilic emulsions or buffered, aqueous solutions, dissolved and/or dispersed in a polymer or an adhesive. In various embodiments, such patches are constructed for continuous, pulsatile, or on demand delivery of pharmaceutical agents. In additional embodiments, the transdermal delivery of the agents of the invention, is accomplished by means of iontophoretic patches and the like. In certain embodiments, transdermal patches provide controlled delivery of the agents of the invention. In specific embodiments, the rate of absorption is slowed by using rate-controlling membranes or by trapping the agent within a polymer matrix or gel. In alternative embodiments, absorption enhancers are used to increase absorption. Absorption enhancers or carriers include absorbable pharmaceutically acceptable solvents that assist passage through the skin. For example, in one embodiment, transdermal devices are in the form of a bandage comprising a backing member, a reservoir containing the agent optionally with carriers, optionally a rate controlling barrier to deliver the agent to the skin of the host at a controlled and predetermined rate over a prolonged period of time, and means to secure the device to the skin.

In other embodiments, the agents of the invention, are formulated for administration by inhalation. Various forms suitable for administration by inhalation include, but are not limited to, aerosols, mists or powders. Pharmaceutical compositions of the invention, are conveniently delivered in the form of an aerosol spray presentation from pressurized packs or a nebuliser, with the use of a suitable propellant (e.g., dichlorodifluoromethane, trichlorofluoromethane, dichlorotetrafluoroethane, carbon dioxide or other suitable gas). In specific embodiments, the dosage unit of a pressurized aerosol is determined by providing a valve to deliver a metered amount. In certain embodiments, capsules and cartridges of, such as, by way of example only, gelatin for use in an inhaler or insufflator are formulated containing a powder mix of the agent and a suitable powder base such as lactose or starch.

In still other embodiments, the agents of the invention, are formulated in rectal compositions such as enemas, rectal gels, rectal foams, rectal aerosols, suppositories, jelly suppositories, or retention enemas, containing conventional suppository bases such as cocoa butter or other glycerides, as well as synthetic polymers such as polyvinylpyrrolidone, PEG, and the like. In suppository forms of the compositions, a low-melting wax such as, but not limited to, a mixture of fatty acid glycerides, optionally in combination with cocoa butter is first melted.

In certain embodiments, pharmaceutical compositions are formulated in any conventional manner using one or more physiologically acceptable carriers comprising excipients and auxiliaries which facilitate processing of the active agents into preparations which can be used pharmaceutically. Proper formulation is dependent upon the route of administration chosen. Any pharmaceutically acceptable techniques, carriers, and excipients are optionally used as suitable. Pharmaceutical compositions comprising an agent of the invention, are manufactured in a conventional manner, such as, by way of example only, by means of conventional mixing, dissolving, granulating, dragee-making, levigating, emulsifying, encapsulating, entrapping or compression processes.

Pharmaceutical compositions include at least one pharmaceutically acceptable carrier, diluent or excipient and at least one agent of the invention, described herein as an active ingredient. The active ingredient is in free-acid or free-base form, or in a pharmaceutically acceptable salt form. In addition, the methods and pharmaceutical compositions described herein include the use of N-oxides, crystalline forms (also known as polymorphs), as well as active metabolites of these agents having the same type of activity. All tautomers of the agents described herein are included within the scope of the agents presented herein. Additionally, the agents described herein encompass unsolvated as well as solvated forms with pharmaceutically acceptable solvents such as water, ethanol, and the like. The solvated forms of the agents presented herein are also considered to be disclosed herein. In addition, the pharmaceutical compositions optionally include other medicinal or pharmaceutical agents, carriers, adjuvants, such as preserving, stabilizing, wetting or emulsifying agents, solution promoters, salts for regulating the osmotic pressure, buffers, and/or other therapeutically valuable substances.

Methods for the preparation of compositions comprising the agents described herein include formulating the agents with one or more inert, pharmaceutically acceptable excipients or carriers to form a solid, semi-solid or liquid. Solid compositions include, but are not limited to, powders, tablets, dispersible granules, capsules, cachets, and suppositories. Liquid compositions include solutions in which an agent is dissolved, emulsions comprising an agent, or a solution containing liposomes, micelles, or nanoparticles comprising an agent as disclosed herein. Semi-solid compositions include, but are not limited to, gels, suspensions and creams. The form of the pharmaceutical compositions described herein include liquid solutions or suspensions, solid forms suitable for solution or suspension in a liquid prior to use, or as emulsions. These compositions also optionally contain minor amounts of nontoxic, auxiliary substances, such as wetting or emulsifying agents, pH buffering agents, and so forth.

In some embodiments, pharmaceutical composition comprising at least one agent of the invention, illustratively takes the form of a liquid where the agents are present in solution, in suspension or both. Typically when the composition is administered as a solution or suspension a first portion of the agent is present in solution and a second portion of the agent is present in particulate form, in suspension in a liquid matrix. In some embodiments, a liquid composition includes a gel formulation. In other embodiments, the liquid composition is aqueous.

In certain embodiments, useful aqueous suspension contain one or more polymers as suspending agents. Useful polymers include water-soluble polymers such as cellulosic polymers, e.g., hydroxypropyl methylcellulose, and water-insoluble polymers such as cross-linked carboxyl-containing polymers. Certain pharmaceutical compositions described herein comprise a mucoadhesive polymer, selected for example from carboxymethylcellulose, carbomer (acrylic acid polymer), poly(methylmethacrylate), polyacrylamide, polycarbophil, acrylic acid/butyl acrylate copolymer, sodium alginate and dextran.

Useful pharmaceutical compositions also, optionally, include solubilizing agents to aid in the solubility of an agent of the invention. The term "solubilizing agent" generally includes agents that result in formation of a micellar solution or a true solution of the agent. Certain acceptable nonionic surfactants, for example polysorbate 80, are useful as solubilizing agents, as can ophthalmically acceptable glycols, polyglycols, e.g., polyethylene glycol 400, and glycol ethers.

Furthermore, useful pharmaceutical compositions optionally include one or more pH adjusting agents or buffering agents, including acids such as acetic, boric, citric, lactic, phosphoric and hydrochloric acids; bases such as sodium hydroxide, sodium phosphate, sodium borate, sodium citrate, sodium acetate, sodium lactate and tris-hydroxymethylaminomethane; and buffers such as citrate/dextrose, sodium bicarbonate and ammonium chloride. Such acids, bases and buffers are included in an amount required to maintain pH of the composition in an acceptable range.

Additionally, useful compositions also, optionally, include one or more salts in an amount required to bring osmolality of the composition into an acceptable range. Such salts include those having sodium, potassium or ammonium cations and chloride, citrate, ascorbate, borate, phosphate, bicarbonate, sulfate, thiosulfate or bisulfite anions; suitable salts include sodium chloride, potassium chloride, sodium thiosulfate, sodium bisulfite and ammonium sulfate.

Other useful pharmaceutical compositions optionally include one or more preservatives to inhibit microbial activity. Suitable preservatives include mercury-containing substances such as merfen and thiomersal; stabilized chlorine dioxide; and quaternary ammonium agents such as benzalkonium chloride, cetyltrimethylammonium bromide and cetylpyridinium chloride.

Still other useful compositions include one or more surfactants to enhance physical stability or for other purposes. Suitable nonionic surfactants include polyoxyethylene fatty acid glycerides and vegetable oils, e.g., polyoxyethylene (60) hydrogenated castor oil; and polyoxyethylene alkylethers and alkylphenyl ethers, e.g., octoxynol 10, octoxynol 40.

Still other useful compositions include one or more antioxidants to enhance chemical stability where required. Suitable antioxidants include, by way of example only, ascorbic acid and sodium metabisulfite.

In certain embodiments, aqueous suspension compositions are packaged in single-dose non-reclosable containers. Alternatively, multiple-dose reclosable containers are used, in which case it is typical to include a preservative in the composition.

In alternative embodiments, other delivery systems are employed. Liposomes and emulsions are examples of delivery vehicles or carriers useful herein. In certain embodiments, organic solvents such as N-methylpyrrolidone are also employed. In additional embodiments, the agents described herein are delivered using a sustained-release system, such as semipermeable matrices of solid hydrophobic polymers containing the therapeutic agent. Various sustained-release materials are useful herein. In some embodiments, sustained-release capsules release the agents for a few weeks up to over 100 days. Depending on the chemical nature and the biological stability of the therapeutic reagent, additional strategies for protein stabilization are employed.

In certain embodiments, the formulations described herein comprise one or more antioxidants, metal chelating compounds, thiol containing compounds and/or other general stabilizing agents. Examples of such stabilizing agents, include, but are not limited to: (a) about 0.5% to about 2% w/v glycerol, (b) about 0.1% to about 1% w/v methionine, (c) about 0.1% to about 2% w/v monothioglycerol, (d) about 1 mM to about 10 mM EDTA, (e) about 0.01% to about 2% w/v ascorbic acid, (f) 0.003% to about 0.02% w/v polysorbate 80, (g) 0.001% to about 0.05% w/v. polysorbate 20, (h) arginine, (i) heparin, (j) dextran sulfate, (k) cyclodextrins, (1) pentosan polysulfate and other heparinoids, (m) divalent cations such as magnesium and zinc; or (n) combinations thereof.

The modified fusion proteins described herein can be used alone or in combination with other suitable therapeutic agents, depending on the condition being treated. In some embodiments the modified fusion proteins of the invention will be co-administered with other agents. When used in combination, the fusion proteins described herein may be administered with the second agent simultaneously or separately. This administration in combination can include simultaneous administration of the two agents in the same dosage form, simultaneous administration in separate dosage forms, and separate administration. That is, the modified fusion proteins described herein and other therapeutic agents may be formulated together in the same dosage form and administered simultaneously. Alternatively, the modified fusion proteins of the present invention and other therapeutic agents may be simultaneously administered, wherein both the agents are present in separate formulations. In another alternative, the modified fusion proteins of the present invention may be administered followed by other therapeutic agents, or vice versa. In a separate administration protocol, the modified fusion proteins of the present invention and therapeutic agents may be administered a few minutes apart, or a few hours apart, or a few days apart.

### Kits/Articles of Manufacture

For use in the therapeutic applications described herein, kits and articles of manufacture are also disclosed. In some embodiments, such kits comprise a carrier, package, or container that is compartmentalized to receive one or more containers such as vials, tubes, and the like, each of the container(s) comprising one of the separate elements to be used in a method described herein. Suitable containers include, for example, bottles, vials, syringes, and test tubes. The containers are formed from a variety of materials such as glass or plastic.

The articles of manufacture disclosed herein contain packaging materials. Packaging materials for use in packaging pharmaceutical products Include those found in, e.g., U.S. Pat. Nos. 5,323,907, 5,052,558 and 5,033,252. Examples of pharmaceutical packaging materials include, but are not limited to, blister packs, bottles, tubes, inhalers, pumps, bags, vials, containers, syringes, bottles, and any packaging material suitable for a selected formulation and intended mode of administration and treatment. For example, the container(s) includes one or more fusion proteins described herein, optionally in a composition or in combination with another agent as disclosed herein. The container(s) optionally have a sterile access port (for example the container is an intravenous solution bag or a vial having a stopper pierceable by a hypodermic injection needle). Such kits optionally comprising a fusion protein with an identifying description or label or instructions relating to its use in the methods described herein.

For example, a kit typically includes one or more additional containers, each with one or more of various materials (such as reagents, optionally in concentrated form, and/or devices) desirable from a commercial and user standpoint for use of a fusion protein described herein. Non-limiting examples of such materials include, but not limited to, buffers, diluents, filters, needles, syringes; carrier, package, container, vial and/or tube labels listing contents and/or instructions for use, and package inserts with instructions for use. A set of instructions will also typically be included. A label is optionally on or associated with the container. For example, a label is on a container when letters, numbers or other characters forming the label are attached, molded or etched into the container itself, a label is associated with a container when it is present within a receptacle or carrier that also holds the container, e.g., as a package insert. In addition, a label is used to indicate that the contents are to be used for a specific therapeutic application. In addition, the label indicates directions for use of the contents, such as in the methods described herein. In certain embodiments, the pharmaceutical compositions is presented in a pack or dispenser device which contains one or more unit dosage forms containing a fusion protein provided herein. The pack for example contains metal or plastic foil, such as a blister pack. Or, the pack or dispenser device is accompanied by instructions for administration. Or, the pack or dispenser is accompanied with a notice associated with the container in form prescribed by a governmental agency regulating the manufacture, use, or sale of pharmaceuticals, which notice is reflective of approval by the agency of the form of the drug for human or veterinary administration. Such notice, for example, is the labeling approved by the U.S. Food and Drug Administration for prescription drugs, or the approved
product insert. In some embodiments, Compositions containing a fusion protein provided herein formulated in a compatible pharmaceutical carrier are prepared, placed in an appropriate container, and labeled for treatment of an indicated condition.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims be covered thereby.

### EXAMPLES

### Example 1: Assay for multiple charged variants of Etanercept and its desiaylated forms with isoelectric focusing polyacrylamide gels.

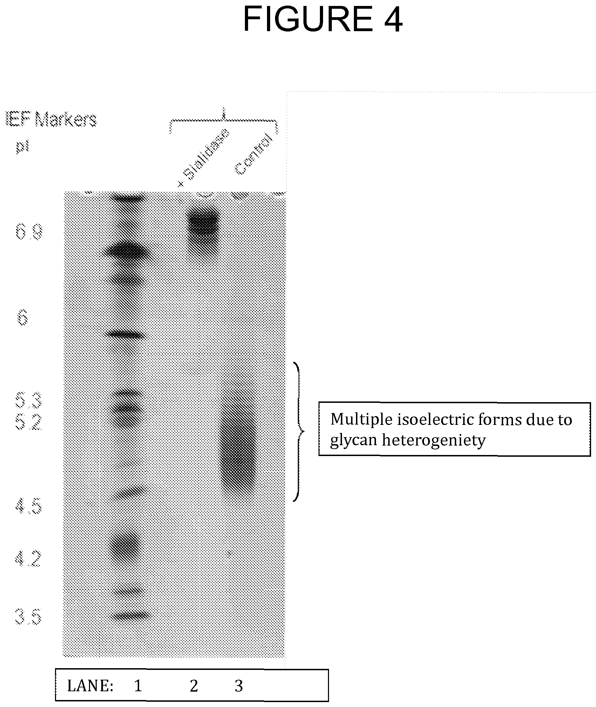

Glycan heterogeniety of Etanercept that results from incomplete glycan biosynthesis can be visualized by analyzing the protein, in its native state, using isoelectric focusing with polyacrylamide gels impregnated with polymeric ampholines. Acrylamide gels of this type provide a continuous pH gradient that allows the protein isoforms to be separated from one another based on their isoelectric point when subjected to an electric field. The number of sialic acid residues on the glycans of the protein greatly influences the net ionic charge on the molecule. Those species of the protein that have higher levels of sialic acid tend to have a more acidic IEP. Protein forms that contain lesser amounts of sialic acid will have more basic (higher pH) IEP. The resolution range that is employed for analysis of Etanercept is pH 7.0 to pH 3.5. The result of a representative experiment is shown in Figure 4.

Lane 3 shows the complex series of protein isoforms (> 17-18 protein bands) of the commercial Enbrel material with isoelectric points ranging between pH 6.5 to pH 4.5.

Lane 2 shows the result of treatment of the Etanercept sample with sialidase ex arthrobacter, which removes all sialic acid residues, from both O and N linked glycans, from the protein. The resulting protein pattern represents isoforms of the protein that arise from charge variations on the peptide core structure such as deamidation of asparagine residues or oxidation of methionine, etc. The IEP of the desialylated protein is now in the basic pH range about 7.0.

This analysis confirms that the multiple isoforms on the native, commercial Etanercept, prior to sialidase digestion, are due to charge heterogeneity of diverse glycan structures that contain varying amounts of sialic acid.

IEF gel analysis serves as a convenient analytical method for monitoring the results of glycotransferase activity on the protein substrate. The incomplete glycan structures on the Fc-IgG fusion molecule acquire sialic acid residues with glycosyltransferase treatment and this decreases the observed charge heterogeneity and imparts a strong negative net charge to the peptide. The resulting IEF pattern collapses to fewer bands on the gel that migrates with a more acidic IEP.

### Example 2: Glycoprofiling of N-linked Glycans on commercial Enbrel and determining the potential susceptibility of the glycans for glycotransferase remodeling

In order to determine the extent of incomplete glycan chains that are present on commercial Etanercept quantitative analysis of the glycans on the protein is determined. Commercial Enbrel (obtained from the pharmacy) is treated with PNGase F digestion, followed by reductive amination in the presence of 2 aminobenzamide (2AB), followed by normal phase-HPLChromatography (Bigge, J. et al. Anal. Biochem. 230:229-38, 1995). Identification of the released and labeled glycans is made using glycan standards of known structure and retention times. Quantification of the relative amounts of each structure is made by measuring the fluorescent signal using the chemically linked 2AB as a reporter group on the reducing end of the glycan.

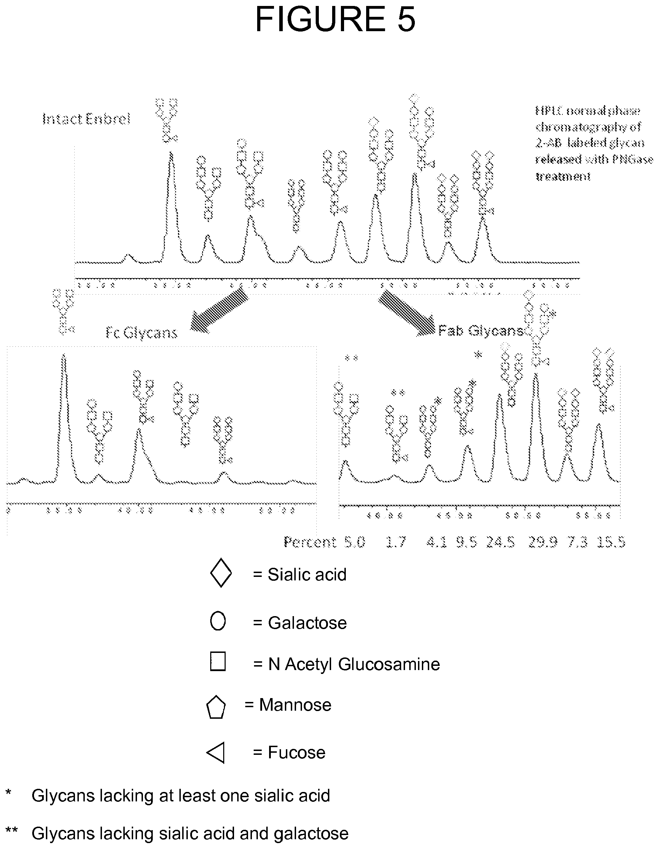

The resulting analysis (Figure 5, top panel), shows that the glycans on commercial Enbrel are an extremely heterogeneous mixture of at least 10 distinct glycan species of varying sizes (metabolic completeness) and charge. All structures are biantennary types, containing only two glycan branches emanating from the core mannose residue.

The glycoprofile obtained in this manner is a composite of the N-linked glycans found on the TNF-receptor component of the molecule and the N-linked glycans at the hinge region of the Fc peptide. Because only the TNF-receptor glycans are involved in carbohydrate-receptor clearance, additional glycoprofiling is done by first treating the Etanercept molecule with a commercially available protease preparation (Von Pawel-rammingen, et al. EMBO J. 21 :1607, 2002) that specifically cleaves the molecule into the Fab and Fc fractions. The two peptides are separated using Protein A chromatography, the Fab peptide flows through the column while the Fc peptide binds to the column resin and is eluted with a mildly acid buffer (pH 3.5). Glycoprofiling on each peptide fragment is carried out, separately, as described above (bottom panel, Figure 2).

This analysis allows the glycans on the receptor portion of the molecule to be studied independently of the Fc glycans.

Inspection of the receptor glycoprofile (right bottom panel) reveals that the majority (about 70%) of the glycans structures on the receptor are metabolically incomplete and lack terminal sialic acid residues. The most abundant biantennary structures (with and without core fucose) contain one terminal galactose residue exposed and comprise about 54% of the total glycans. Structures with two terminal galactose residues comprise about 14%, while about 7% of the glycans are lacking both terminal sialic acid and galactose and have at least one glucosamine residue at the reducing terminus.

This study indicates that Etanercept is suitable for glycan remodeling with both sialyltransferases and galactosyltransferases. The extent and completeness of O-glycosylation cannot be determined from this analysis since these glycans are not released from the protein with PNGase treatment. Alternate analytical strategies are required that employ chemical means to liberate the O-linked glycans from the peptide core (Patel, T. J. et al, Biochemistry 32:679-93, 1993).

### Example 3. Glycoengineering Etanercept with Sialyltransferase (ST6) ex Photobacterium damselae, Pd2,6ST, and bovine β4GalT; determination of optimal reaction time and enzyme concentration on an analytical scale.

The sialyltransferase Pd2,6ST from Photobacterium damselae has high specific activity toward a variety of low molecular weight carbohydrates, oligosaccharide structures and gangliosides. The Pd2,6ST enzyme was chosen for glycoengineering of Etanercept because it has a high specific activity and may be produced in large quantities as a recombinant product in bacterial expression hosts. In order to determine the optimal reaction conditions that give complete or near complete coverage of available galactose residues, a series of analytical-scale reactions were carried out with varying concentrations of the enzyme and reaction times. The progress of the sialylation reaction was monitored using IEF acrylamide gel analysis as described in above examples. Glycoprofiling analysis of etanercept indicated that a significant percentage of the glycans on the TNF receptor region lacked not only terminal sialic acid but some of the glycans also lacked terminal galactose residues and terminated with glucosamine. For this reason, the bovine beta galactosyltransferase, β 4GalT, was also included in the glycosylation reaction mixture. This enzyme added galactose to these exposed glucosamine sites and provided additional substrates for the sialyltransferase action. The amount of β 4GalT was maintained constant in these experiments.

In a typical experiment, commercial etanercept was obtained from the local pharmacy. The incipient buffer was exchanged with 50 mM 2-(N-morpholino)ethanesulfonic acid, MES buffer, pH 6.4 using a centrifuge concentrator (100 KDa cut off membrane). The final concentration was adjusted to 8 mg protein/mL. Damsela sialyltransferase (Sigma Chemical, Co.) was obtained as a lyophilized powder and was suspended in MES buffer to be 6 U/mL. Serial dilutions were made to give solutions of 3 U/mL, and 1.5 U/mL. Bovineβ4Gal T (G5507, Sigma, inc) was suspended in MES butter to be 6 U/mL.

Reactions were carried out with 160 µg of etanercept, 125 µg UDP galactose (Carbosynth, inc) (∼ 5.6 mM) and 125 µg CMP-sialic acid (Carbosynth, Inc)(∼5.4 mM), 0.033, 0.015 and 0.008 U of Pd2,6ST. Each reaction contained 0.033 U β 4Gal T 1, and 1.7 mM MnCl₂ in a total volume of ∼ 38 µL in 0.5 mL Eppendorf conical centrifuge tube. A reaction in which sugar nucleotides were substituted with buffer served as a control. The reaction was initiated by transfer of the tubes to a 37 °C heating bath and a 5 µL aliquot was taken from the reaction containing 0.033 U of ST6 immediately and stored on ice and served as a zero time control. The remaining reaction samples were heated for 4 h, 8 h, and 24 h. At the designated time, an aliquot (5 µL) of each reaction mixture was removed and stored on ice until the product was examined using IEF acrylamide gel analysis.

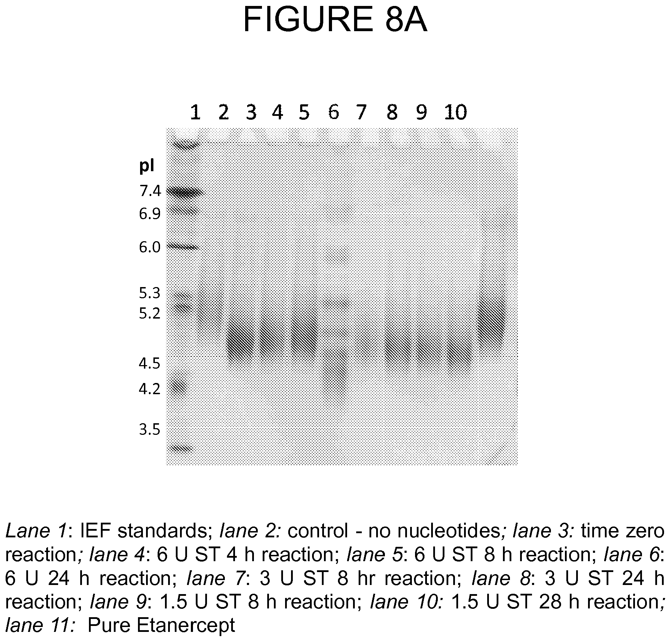

The results of these experiments are shown in Figure 8A. The expected multi banded pattern for etanercept can be seen in lane 11. The addition of sialic acid residues to the molecule resulted in a reduction in glycan heterogeneity (collapse of the multiband pattern) with the formation of a diffuse protein band with a significantly more acidic pI than the starting material. Apparently, the transferase reaction occurred very rapidly under these conditions, as the reaction appeared to be complete or near-complete in the zero time control sample (lane 3). No change in the etanercept protein pattern was observed in the control sample that lacked sugar nucleotides (lane 2). Extensive protein degradation was detected in the sample with 0.033 U of Pd2,6ST heated for 24 h (lane 6). All reaction times and Pd2,6ST concentrations (with the exception of those in lane 6), including the zero time control, gave a similar banding pattern.

### Example 4: Large scale preparation of modified etanercept using Pd2,6ST and b β 4Gal T 1

### Reaction Conditions

A preparative scale glycosylation reaction was carried out to provide sufficient material for glycoprofiling analysis and functional assays. Based on the results of the analytical scale reaction, 22 mg of buffer-exchanged etanercept in 50 mM MES buffer was added to a 15 mL conical centrifuge tube followed by 16.2 mg UDP-Gal and CMP Sialic acid, 4.1 U b β 4 GalT and 0.42 U of Pd2,6ST and 1.8 mM MnCl₂ in a total volume of ∼4.5 mL. The reaction was allowed to proceed at 37 °C for 4 hours. A sample was removed from the reaction mixture and the progress of the reaction was monitored with IEF acrylamide gel. The remaining reaction mixture was stored on ice overnight until the results of the gel analysis could be determined.

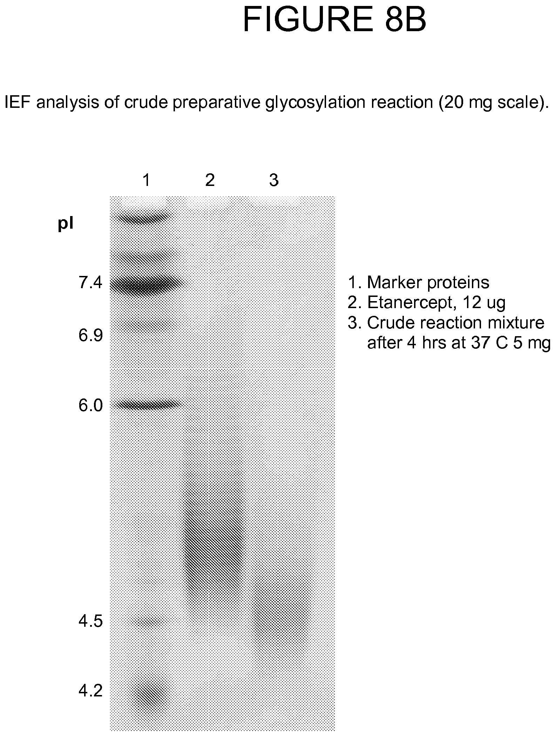

The IEF analysis indicated that the reaction was complete after 4 h. In order to avoid potential protein breakdown, as observed in the analytical-scale study (at 24 h incubation), in this case the reaction was terminated at 4 h and no further incubation was carried out. The results of this experiment are shown in Figure 8B.

### Endotoxin removal-

Since the above material was to be tested *in vivo* in a pharmacokinetic study, it was essential that the glyco-modified etanercept had low endotoxin levels (< 1.2-1.5 EU /injection in rats). For this reason, the crude reaction mixture was subjected to phase partitioning extraction using Triton X -114 to reduce endotoxin levels which were determined to be about 57 EU/ml.

In a typical extraction, the crude glycosylation reaction mixture was adjusted to 150 mM NaCl with 0.47 mL of 1.5 M NaCl solution in water followed by EDTA 0.47 mL of 100 mM EDTA in MES buffer, pH 6.4 to a final concentration of 10 mM. After mixing, the crude reaction mixture was adjusted with 0.112 mL with Triton X-114 to be a final concentration 1% (from a stock solution of ∼ 50% w/v in water). The mixture was placed on ice and thoroughly mixed for 60 seconds. The reaction samples were then placed in a 37 °C heating block incubator for 5 minutes to allow two phases to form. The samples were then centrifuge for 7 seconds in a micro centrifuge maintained at 37 °C in an incubator oven. The oily droplet at the bottom of the tube was separated by removing the upper aqueous phase. The extracted material was stored at 4 °C until further purification.

Endotoxin levels were then determined on the extracted material using the Limulus amebocyte lysate assay ,LAL, kit (Lonza, Inc.). The extracted solution contained about 1.3 EU/mL of endotoxin after a single extraction cycle.

### Purification on Protein A chromatography-

After detergent treatment the crude glycosylation reaction mixture was adjusted to 10 mM Tris-HCl, pH 7.4. The solution (at room temperature) was applied to a 3 mL Protein A Sepharose (Pharmacia) equilibrated in 10 mM Tris buffer, pH 7.4. In order to insure efficient retention of the protein, the solution was applied to the column three times. The contaminating transferases and residual sugar nucleotides were then eluted by washing the column with ∼6 column volumes of Tris buffer at room temperature.

The modified glyco-etanercept was eluted using (∼ 5 column volumes) of a 'Gentle Ab/Ag elution buffer' (Pierce Chem. Co., Inc) at pH 6.6, in order to minimize losses of sialic acid from the protein.

The eluted protein fraction was collected and buffer exchanged into 10 mM Tris buffer, pH 7.4 using a centrifuge concentrator (100 kDa cut off membrane) and sterile filtered (0.22 micro filter). The protein concentration was determined at OD 280 nmeters using an extinction coefficient of 1.2 Od/mg protein. About 14.2 mg of protein was obtained giving a yield of about 65 %. The endotoxin levels were determined to be ∼0.04 EU/mg.

A portion of the purified material was formulated at 4.9 mg/mL in etanercept formulation buffer, 40 mg mannitol, 10 mg sucrose, 1.2 mg tromethylamine, per ml, pH 7.4. After formulation the endotoxin levels were ∼ 0.15 EU/mg.

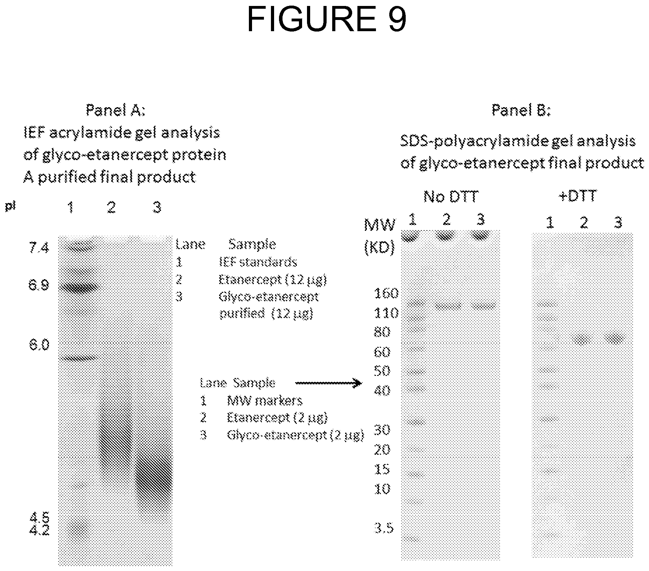

The final material was examined with IEF acrylamide gel analysis and SDS polyacrylamide gel analysis under reducing and non reducing conditions, Figure 9 Panels A and B. respectively. This analysis showed that the final product remained highly sialylated after endotoxin removal, purification and formulation. The final material was highly purified giving a single major band on gel analysis, similar to the unmodified etanercept. The molecular weight of the glyco-etanercept was about 2,000 Da higher than commercial etanercept, which was expected given the significant increase in sialic acid residues that have been added to the glycan chains.

### Example 4: Determination of TNF Binding activity

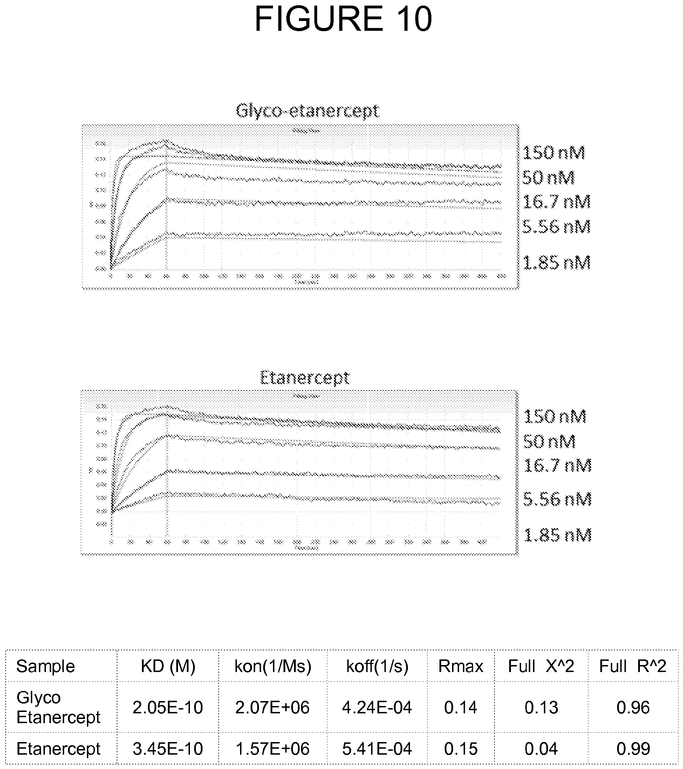

The purified glyco-etanercept was evaluated for its TNF binding affinity and compared with commercial etanercept using a Forte Bio (Pall, Inc) instrument. The sensor tip of the instrument contained immobilized strepavidin. Biotinylated tumor necrosis factor was then bound to the strepavidin- coated sensor tip. Binding affinities of etanercept and glyco-etanercept for the immobilized TNF on the sensor were carried out at the concentrations shown in Figure 10. The resulting Kd values for the two proteins were nearly identical with a slightly tighter binding affinity of glyco-etanercept compared with etanercept. These results show that the addition of sialic acid residues on to the protein and the manipulations to remove endotoxin had no deleterious effect on the affinity of the molecule for the TNF protein.

### Example 5: Glycoprofiling and glycan structural studies of N linked glycan on glycoengineered etanercept

The glycan structures on the glycoengineered etanercept were evaluated using glycoprofiling. The *N*-linked glycans were liberated from the protein using PNGase F treatment, followed by reductive amination, labeling with 2 amino benzamide. The glycan pool was resolved using normal phase chromatography on a BEH silica-based 1.7 µm column glycan HPLC column, 2.1 mm X 150 mm (Waters, inc.) using a Acquity UPLC-F1 instrument (Waters, inc) fitted with a variable wave length fluorescent detector λex= 330 nm and λem=420 nm, Figure 10. The identification of the signals was made based on their retention times using known standards and by molecular mass determinations made using MALDI-TOFF mass spectrometry.

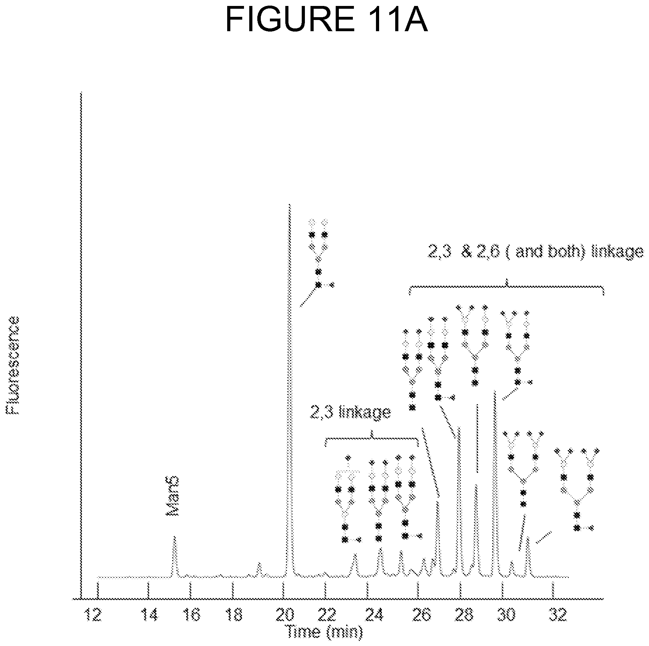

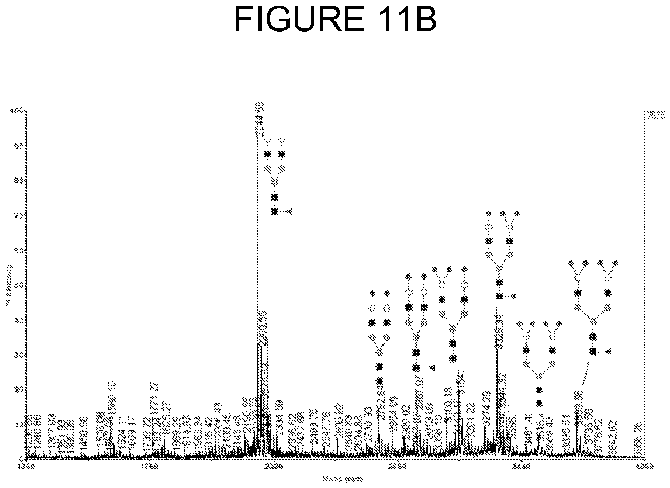

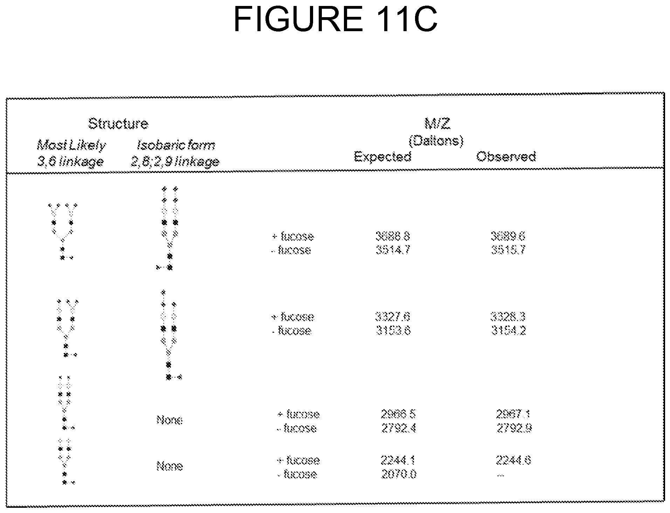

The PNGase F-released glycan pool was converted to their permethylated derivatives using DMSO-NaOH and methyliodide method (Anumula, K. and Taylor, PB., Anal Biochem, 203:101-8, 1992) and their respective masses determined using a 4800 Plus MALDI TOF/TOF™ Analyzer from AB-Sciex, operating in the positive mode. Glycan samples were suspended in a 5:1 molar mixture of dihydroxybenzoic acid and 5-*O*-methoxy salicylic acid containing methanol and 1 % trifluoroacetic acid in THF and spotted on the MALDI plate and allowed to dry. Structural assignments, based on the molecular mass, and annotations was made using Glycobench software (http://www.eurocarbdb.org/applications/ms-tools.) and are shown in Figures 11A, B,and C.

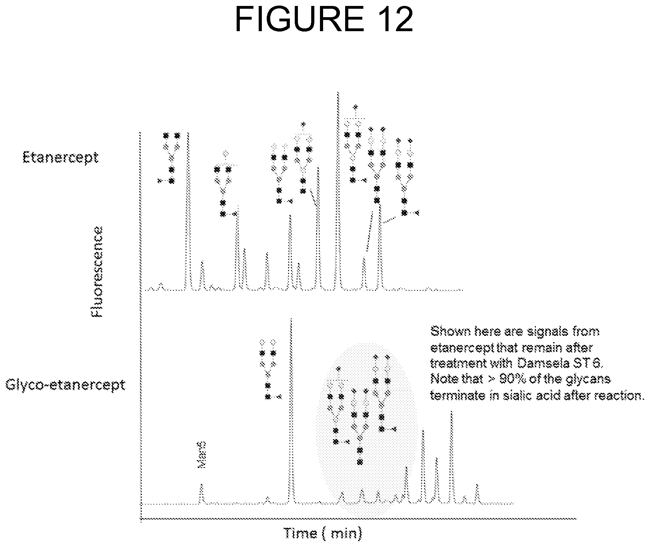

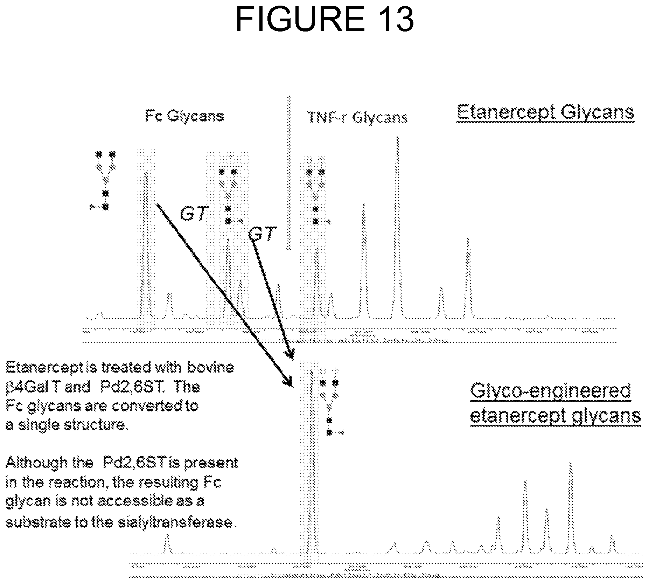

The HPLC glycoprofiles of glyco-etanercept and etanercept were compared Figure 12. These data show a significant change in the glyco profile of the molecule under these conditions. The Fc glycans are found to be completely galactosylated but they were not sialylated. This may reflect the steric inaccessabilty of the glycans in Fc region to the Pd2,6ST action under these conditions. In contrast, greater than 90% of the glycans in the TNF receptor region terminate with sialic acid residues with little, if any, exposed galactose residues remaining after sialyltransferase treatment (Figure 13).

Rather unexpectedly, based on the molecular masses of the components of the glycan mixture as well as the glycan structures identified in the starting etanercept substrate, some structures appeared to be biantennary, but contain at least one or two additional sialic acid residues per antennae, for a total of three and four sialic acids per molecule, respectively. The point of attachment of these additional sialic acids could not be determined from these data alone and, therefore, additional characterization was required. Structures of this type have not been reported as a component of naturally occurring or recombinant glycoproteins. Although these glycan structures were unexpected, this observation in keeping with the broad acceptor substrate specificity of the Pd2,6ST enzyme toward low molecular weight carbohydrate substrates.

Since mass spectral data is acquired on a glycan mixture, the correlation of a specific glycan structure (mass) with a specific peak in the glycoprofile is usually determined by inference. It is usually assumed that the higher molecular weight (more complex) molecules will exhibit longer retention times on the BEH chromatography system employed for glycoprofiling. However, isomeric forms of glycans with identical carbohydrate compositions may influence the chromatographic behavior of the oligosaccharides.

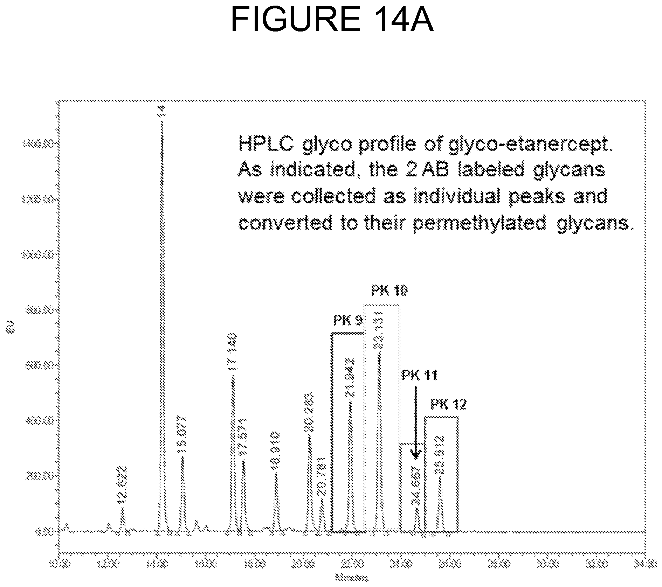

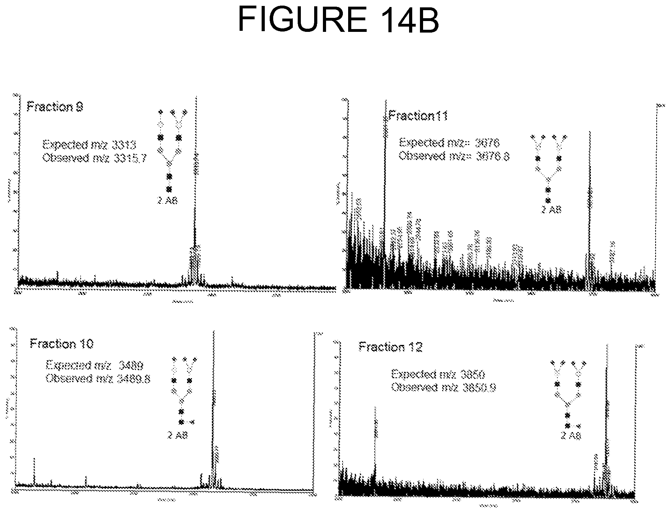

In order to unequivocally correlate the molecular mass of the glycan components with the signals of specific peaks in the 2-AB labeled glycan pool, HPLC fractionation of the glycans was carried out using the same glycoprofiling protocol. But, in this case, the individual peaks were collected as distinct fractions. Each fraction was then subjected to permethylation derivatization and the mass of the components in the isolated fractions was determined with MALDI mass spectral analysis. The results of these experiments are shown in Figures 14 A and 14 B. This analysis confirmed the structural assignments to specific peaks in the glycoprofile. The presence of tri and tetra sialylated species on the biantennary glycans was confirmed in those fractions with the longer retention times in the glycoprofile.

Given the substrate specificity of the Pd2,6ST sialyltransferase toward the 6 position of galactose on low molecular weight substrates, the it is most likely that the penultimate galactose residues on each branch of the biantennary structure was disubstituted with sialic acid, with one sialic acid at the 3 and a second sialic acid at the 6 position on the same galactose. Although the latter is the most likely structural form of these glycans, another isobaric variation of this molecule is possible. Alternatively, the additional sialic acid residues may be linked together in a 2,8 or 2,9 linkage bound to galactose. Disialo linkages are well known in both nerve tissue related glyco proteins and gangliosides.

Since the damsela enzyme has not been reported to give a sialic acid to sialic type linkage, the more plausible structure would be one in which galactose serves as the attachment point.

Further definition of the carbohydrate structure was made by carrying out linkage analysis of the component carbohydrate residues.

### Linkage analysis:

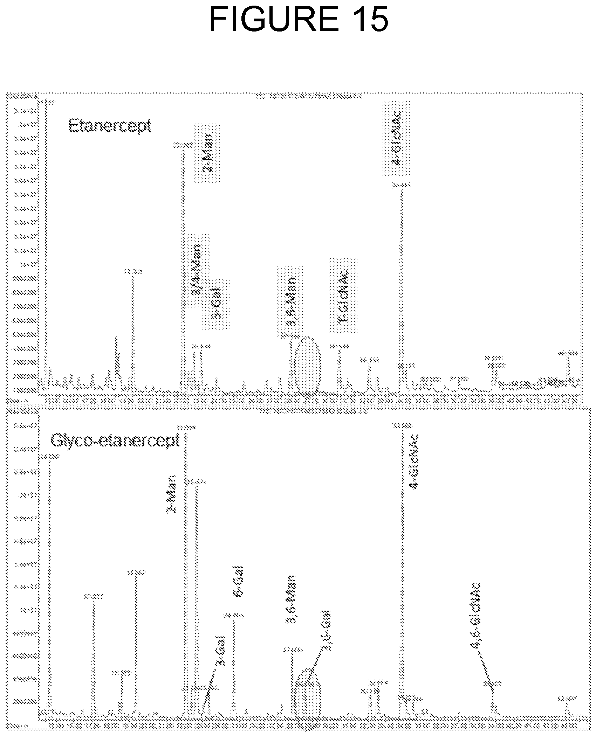

Linkage analysis of the carbohydrate residues was carried out in order to verify the predicted structural assignment of a 3,6 disubstituted galactose residue. The PNGase F-released glycan pool was converted to permethylated -alditol acetates following the procedure describe above. After acid hydrolysis, the resulting permethylated carbohydrates were reduced with sodium borohydride and acetylated to give permethylated alditol acetates. The resulting derivatives were resolved from one another using a Agilent Technologies 7802 A gas chromatograph fitted with an Agilent 5975 Series Mass Selective Detector on a Resetek 5s , 30m X 0.25 mmX 0.25 um column, using helium as carrier gas and eluting with a temperature gradient of 80 °C to 140 °C at 10 C /min followed by a second temperature gradient from 140 °C to 220 °C at 2 C /min with a third temperature gradient to 230 °C at 5 °C C/min column at 280 °C. The signals for the individual alditol acetate derivatives were identified based on their retention times using known alditol acetate standards, as shown in Figure 15. The resulting analysis verified the presence of 3,6 substituted galactose residues in the glycan pool from the glyco-etanercept molecule. This structure was not detected in unmodified etanercept.

### Glycan sequencing with LTQ Ms/Ms fragmentation analysis of permethylated glycan fractions-

The permethylated alditol acetate analysis verified the presence of the 3,6 disubstituted galactose residues in the glycan pool. Further delineation of the presence of this structural assignment was made using Ms/MS fragmentation analysis of the isolated glycan HPLC fractions, # 9 and #12 **Figure 14** as their permethylated derivatives. Samples were dissolved in 90% methanol and 1 mM NaOH and injected using s syringe pump attached to a LTQ-Orbitrap Discovery LTQ-Xl mass spectrometer (Thermo Scientific). Data was collected in the positive mode. At least 4 cycles of Ms/MS fragmentation were carried out on each sample and their resulting derivative ions. About 44 EV were used for each successive fragmentation cycle of the selected ions.

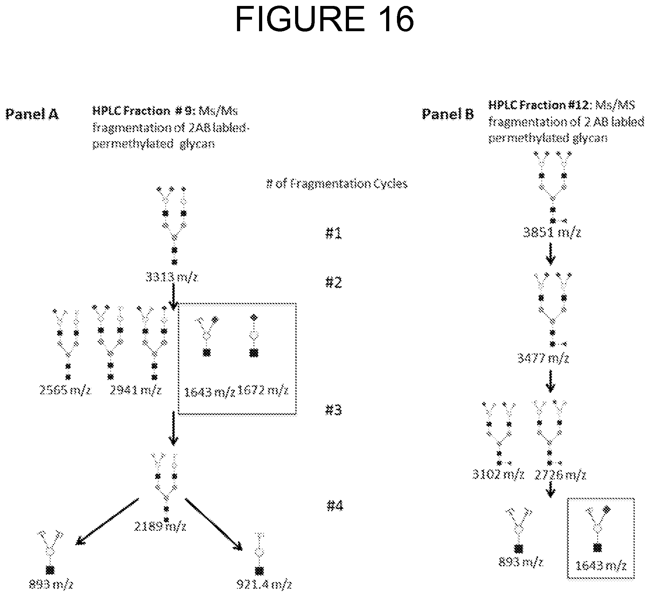

For HPLC Fraction # 9, the molecular mass of the resulting derivative ions was consistent with the proposed structure with one antenna containing two sialic acid residues substituted in branched-type arrangement on a single galactose and the second antenna containing a single sialic acid in linear -type arrangement on galactose, Figure 16, panel A. Fragmentation of the double charged, sodiated parent ion at 3315 m/z (as the permethylated 2 AB derivative) gave rise to a number of partially sialylated derivative ions, 2941m/z, and 2565 m/z corresponding to a loss of one and two sialic acid residues, respectively. Also observed were two low molecular weight ions at 1643 m/z and 1672 m/z. These fragments support the proposed structure of a combined linear and branched sialic acids on the terminal galactose residue. The signal with a m/z of 1672, indicated the presence of an additional OMe group on the galactose residue compared with the ion species at 1643 m/z. The presence of sialic acid limits the number of methyl groups from three, for the linear type structure, to two methyl groups on the disialylated structure, and hence, the molecular weight difference between these two ions.

HPLC fraction # 12 gave a molecular mass of 3851m/z as its triply charged permethylated 2 AB derivative, Figure 16 panel B. Additional fragmentation of this ion gave rise to a number of partially sialylated derivative ions containing two and three sialic acid residues. The derivative ion at 3477 m/z, was consistent with the fragment containing three sialic acids. Further irradiation gave a glycan ion with a single sialic acid at 2726 m/z. Further fragmentation gave rise to a glycan ion at 1643 m/z. Unlike the fragmentation pattern of HPLC fraction #9, which contained a single sialic linked to the terminal galactose on one antenna, no signal at 1671 m/z was observed here. These data support a structure for the HPLC fraction #12 glycan with both terminal galactose residues disubstituted with two sialic acids on each of the oligosaccharide branches.

In summary, the proposed structures with a disubstituted galactose residues were consistent with the data. No evidence for the presence of a 2,8 or 2,9 sialic acid disaccharide structure was apparent from this analysis.

### Example 6: Glycoengineering of etanercept with recombinant human sialyltransferase rST6Gal 1 and recombinant human β4GalT

Commercial etanercept was modified with mammalian sialyltransferase and galactosyltransferase using a nearly identical protocol to that used above for the Pd2,6ST enzyme. The glycans on the resulting product were characterized similarly.

Typically small scale analytical reactions were carried out initially to determine optimal reaction time and enzyme concentrations levels. Sialyltransferase levels were varied, along with reaction times maintaining the 4GalT as a constant level in all reactions. Reaction products were monitored using IEF acrylamide gel analysis. Etanercept was obtained from the local pharmacy. The incipient buffer was exchanged with 50 mM 2-(N-morpholino)ethanesulfonic acid, MES buffer, pH 6.4 using a centrifuge concentrator (100 KDa cut off membrane). The final concentration was adjusted to 8 mg protein/mL. Human ST6Gal1 (accession # P15907.1) with the stem region deleted (AA 1-75) as an *N*-terminal fusion with green fluorescent protein the chimeric protein was produced by transient transfection of HEK 293 cells (custom preparation Exon bio, inc). The protein was purified using metal ion chromatography and was judged to be homogeneous with SDS polyacrylamide gel electrophoresis. After buffer exchange into 150 mM NaCl, 50 mM MES buffer, pH 6.2 containing 50% glycerol, the material stored at - 20 °C.

Determination *enzyme activity-* The enzyme activity of the recombinant product was determined using the coupled enzyme method of Wu, Z.L. et al (Glycobiology, 21: 727-30, 2011). In this assay the ST6Gal1 activity was determined by quantifying the amount of CMP that was formed as one of the reaction products using asialofetuin as an acceptor substrate. The CMP was measured using 5' nucleotidase CD 73, a phosphatase which cleaves inorganic phosphate from the CMP moiety. The malachite green method was used for quantification of the released inorganic phosphate (R and D systems, product number EA 002). The specific activity of the purified recombinant ST6Gal1 was determined to be ∼1 µmol/min/mg protein.

The human β 4GalT (accession # P15291) enzyme was prepared as a recombinant product similarly, deleting the stem region (-AA1-78). Unlike the ST6Gal1, the hβ4 GalT was not produced as a GFP fusion protein, (custom product, Exon Bio,inc). The enzyme activity was measured using a modification of the coupled enzyme assay that was employed for the ST6Gal1 enzyme. In this assay, the human pyrophosphatase, CD39L3/ENTPD3 was used to generate inorganic phosphate by cleaving the pyrophosphate bond from the UDP product after enzyme transfer of the sugar moiety to the carbohydrate substrate, GlcNAc . Inorganic phosphate was quantified using the malachite green assay.

Using this assay, the specific activity of the recombinant h4Gal T, was ∼ 8 µmol/min/mg protein. The protein was formulated in 50 mM Tris HCl buffer, pH 7.5, with 50 % glycerol and stored at - 80 C.

### Optimization of glycosyltransferase conditions:

The procedure for determining optimal glycosyltransferase reaction conditions of ST6Gal 1 and hβ4GalT were similar to those used with the Pd2,6ST enzyme. Both recombinant ST6Gal1 and hβ4GalT were buffer exchanged into 50 mM MES, pH 6.4. The final concentration of both enzymes was adjusted to 6 U/mL. Serial dilutions of ST6Gal1 were made to give solutions 6 U/mL, 3 U/mL, and 1.5 U/mL. Human β4Gal T (Sigma, inc) was buffer exchanged and suspended in MES butter to be 6 U/mL. Reactions were carried out with 160 µg of etanercept, 125 µg UDP galactose (Carbosynth, inc) (∼ 5.6 mM) and 125 µg CMP-sialic acid (Carbosynth, Inc)(∼5.4 mM), 0.033, 0.015 and 0.008 U of ST6Gal1. Each reaction contained 0.033 U h β4Gal T 1, and 1.7 mM MnCl₂ in a total volume of ∼ 38 µL in 0.5 mL Eppendorf conical centrifuge tube. A reaction in which sugar nucleotides were substituted with buffer served as a control. The reaction was initiated by transfer of the tubes to a 37 °C heating bath and a 5 µL aliquot was taken from the reaction containing 0.033 U of ST6Gal1 immediately and stored on ice and served as a zero time control. The remaining reaction samples were heated for 4, 8, and 24 h. At the designated time, an aliquot (5 µL) of each reaction mixture was removed and stored on ice until the product was examined using IEF acrylamide gel analysis.

### IEF examination of the reaction products:

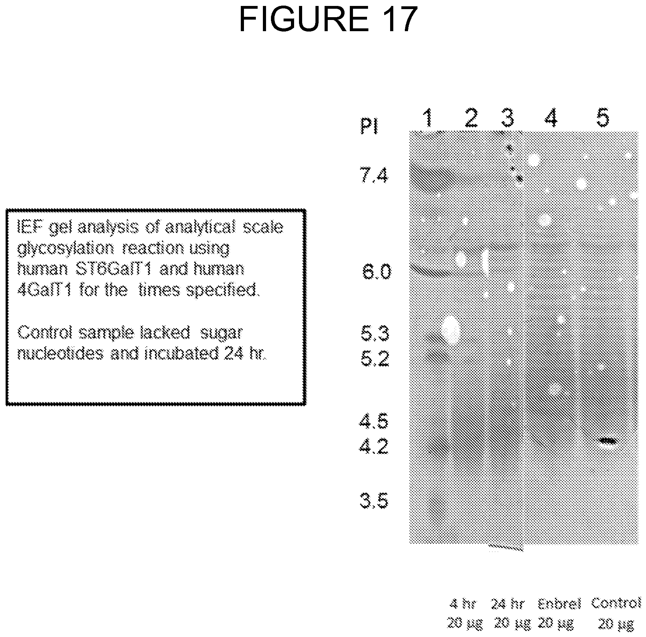

The reaction results (complete gel data not shown) were similar to those of the Pd2,6ST enzyme. The reaction occurred very rapidly as the zero time control showed near complete conversion of the substrate. The etanercept multibanded glyco forms were converted to a diffuse band with a significant shift toward a more acidic isoelectric point. As shown in Figure 17, in contrast to the results obtained with the damsela enzyme, there was no detectable break down of the product using 0.033 U of enzyme for 24 hours at 37 °C.

### Large scale preparation of etanercept reaction with hST6Gal1 and hβ4GalT

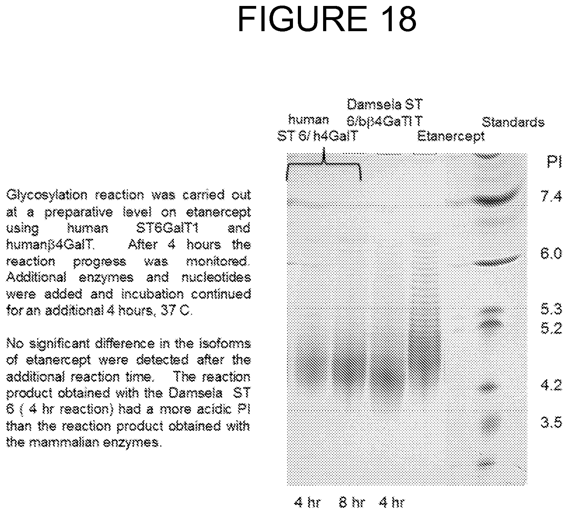

The reaction was scaled up (∼ 32 X) over the analytical scale reaction. To a 2 mL polypropylene screw capped tub, ∼5 mg of buffer-exchanged etanercept in 50 mM MES buffer was added, followed by 4 mg UDP-Gal and 4 mg of CMP Sialic acid, ∼0.25 U β4 GalT and ∼ 0.26 U of ST6Gal1 and 1.8 mM MnCl₂ in a total volume of ∼1.2 mL. The reaction was allowed to proceed at 37 °C for 4 hours. A sample was removed from the reaction mixture and stored on ice. At this time, an additional bolus of sugar nucleotides and glycosyltransferases were added and the reaction allowed to proceed an additional 4 hours at 37 °C (total volume ∼ 1.7 mL). The progress of the reaction was monitored with IEF acrylamide gel and the results are shown in Figure 17. By IEF gel analysis, the reaction was apparently complete after 4 hours and no additional shift in the electrophoretic mobility of the protein was detected with additional sugar nucleotides, transferase enzymes and reaction time. As shown in Figure 18, the product had a more acidic PI than the starting etanercept but it was notably less so than the reaction product obtained with Pd2,6ST enzyme.

### Protein A purification:

The crude reaction product was adjusted to be pH 7.4, 10 mM Tris-HCl using a 100X concentrated buffer solution. The reaction was applied at least 5 times at room temperature to a 1 mL protein A Sepharose column. After washing the column with 10 column volumes of 10 mM Tris, pH 7.4, the modified etanercept was eluted with 5 column volumes of Gentle AG/Ab elution buffer pH 6.6 (Pierce chemical co.). The eluting buffer was removed with buffer exchange (10 mM Tris pH 7.4) using a centrifuge membrane concentrator (100 kDa cut off membrane). The final material was formulated into 20 mM Tris pH 7.4, 150 mM NaCl. About 2.0 mg of purified material or a 40% overall yield was obtained.

### Glycoprofiling analysis of N-linked glycans:

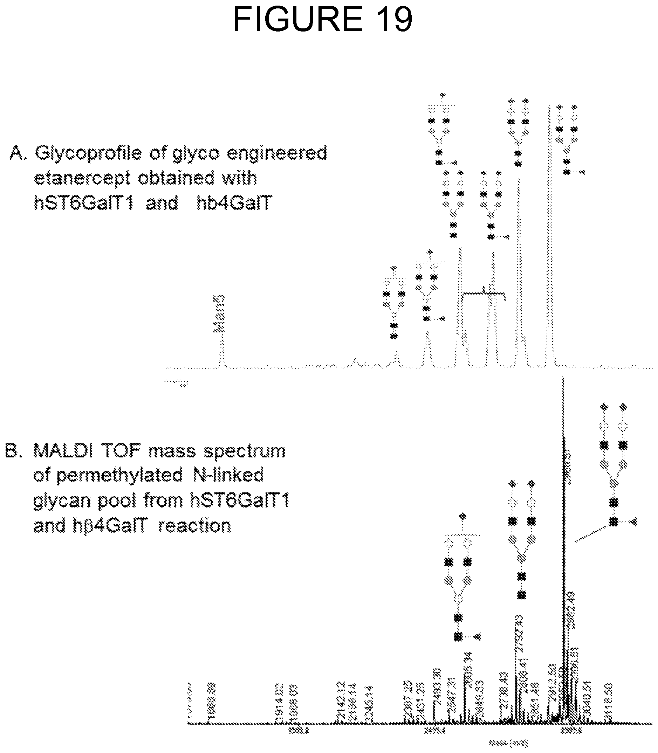

Identification of the modified *N*-linked glycans that resulted from glycosyltransferase treatment of etanercept was made using HPLC glycoprofiling of the PNGase F-released glycans as described previously. Structural analysis was further carried out using MALDI FOF mass spectrometry of the corresponding permethylated glycan pool, Figure 19 Panels A and B.

This analysis revealed the presence of only three major glycan structures (mass spectra, Panel B) within the glycan pool. Greater than 70% had complete sialylation of terminal galactose residues. However, HPLC analysis (Panel A) showed the presence of at least 7-8 major glycan components. The multiple signals in the HPLC analysis represent structural isomeric forms of the three major molecular species that are identified with mass spectrometry analysis. The native glycans on etanercept contain only sialic acids that are linked at the 3 position of galactose (the mammalian host cells, Chinese Hamster Ovary cells, do not express a 2,6 sialyltransferase). The hST6Gal1 enzyme transfers sialic acid to the 6 position of galactose specifically, thus a complex mixture of structures some of which have only 2,3 linked sialic acid, some which have only 2,6 sialic acid, and some which are a mixture of both 2,3 and 2,6 sialic acids results after glycotransferase reaction. These structures have identical molecular masses but migrate differently on HPLC chromatography. Interestingly, galactose residues disubstituted were not detected with the human sialyltransferase, even under extended reaction times.

Further, the Fc glycans apparently acquire both galactose as well as sialic acid residues under these reaction conditions. The human ST6Gal1 enzyme, unlike the microbial Pd2,6 ST glycotransferase displays greater accessibility to the sterically restricted Fc glycan structures, Figure 19.

### Example 7: Glycoengineering with 2,3 sialyltransferase CSTII (Campylobacter jejuni)

The action of the CST II enzyme toward etanercept was evaluated. The CST II enzyme differs from either the damselae enzyme or the hST6Gal1 enzyme because it transfers sialic acid to the 3 position of galactose, and under some conditions can also transfer sialic acid to the 8 position of the existing sialic acid residue linked to the 3 position of galactose.

A recombinant form of the CST II enzyme was prepared in e.coli and was purified as a fusion protein with maltose binding protein to facilitate purification (Pumtiwitt, C et al. Glycoconjugate J. 30: 857-70, 2013: Lee, H. J. et al. J Biol. Chem. 286: 35922-35932, 2011; Gilbert, M, et al J. Biol. Chem 275 3896-06, 2000; Chu, C. P. et al. Nat. Struct. Mol. Biol. 11: 163-170, 2004).

### Determination of optimal reaction conditions:

Small scale reactions were carried out with variations in reaction time and enzyme concentrations in a manner similar to that with the Pd2,6ST enzyme and the hST6Gal1 enzymes. Reactions were carried out with 160 µg of etanercept, 125 µg UDP galactose (Carbosynth, inc) (∼ 5.6 mM) and 125 µg CMP-sialic acid (Carbosynth, Inc)(∼5.4 mM), 0.008, 0.08 and 0.8 U of CSTII. Each reaction contained 0.033 U β4Gal T 1, and 1.7 mM MnCl₂ in a total volume of ∼ 38 µL in 0.5 mL Eppendorf conical centrifuge tube. A reaction in which sugar nucleotides were substituted with buffer served as a control. The reaction was initiated by transfer of the tubes to a 37 °C heating bath and a 5 µL aliquot was taken from the reaction containing 0.8 U of CSTII immediately and stored on ice and served as a zero time control. The remaining reaction samples were heated for 1 and 4 hours. At the designated time, an aliquot (5 µL) of each reaction mixture was removed and stored on ice until the product was examined using IEF acrylamide gel analysis.

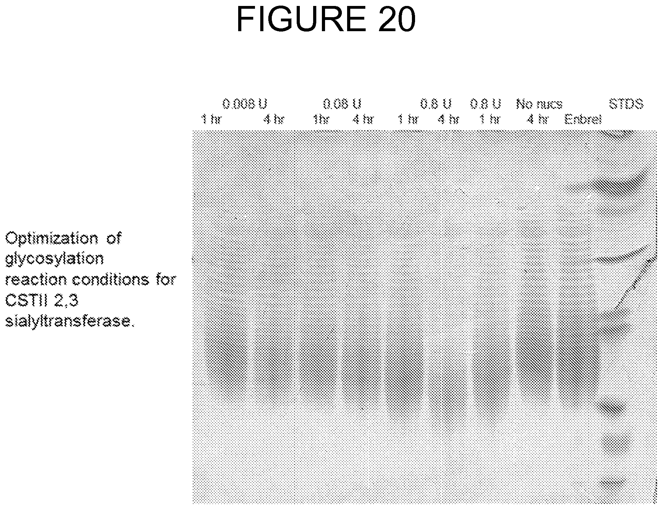

The results of this experiment indicated that a complete reaction was obtained using 0.8 U of enzyme with 4 hours of incubation, Figure 20.

### Large scale preparation:

Additional CSTII-modified glyco-etanercept was prepared on a preparative scale to provide sufficient material for glycol-profiling studies. The protocol was similar to that used above for the mammalian sialyltransferase , at a scale up over the analytical scale reactions of about 32 times. To a 2 mL polypropylene screw capped tub, ∼ 5 mg of buffer-exchanged etanercept in 50 mM MES buffer was added, followed by 4 mg UDP-Gal and 4 mg of CMP sialic acid, ∼0.25 U β4 GalT and -25 U of CST II enzyme and 1.8 mM MnCl₂ in a total volume of ∼1.2 mL. The reaction was allowed to proceed at 37 °C. After 2 h an analytical sample was removed from the reaction mixture and stored on ice, and an additional bolus of CMP sialic acid (only) was added and the reaction allowed to proceed an additional 2 hours at 37 °C (total volume ∼ 1.7 mL). The progress of the reaction was monitored with IEF acrylamide gel Figure 20.

### Protein A purification

The crude reaction product was adjusted to be pH 7.4, 10 mM Tris-HCl using a 100X concentrated buffer solution. The reaction was applied at least 5 times at room temperature to a 1 mL protein A Sepharose column. After washing the column with 10 column volumes of 10 mM Tris, pH 7.4, the modified etanercept was eluted with 5 column volumes of Gentle AG/Ab elution buffer pH 6.6 (Pierce Chemical Co.). The eluting buffer was removed by buffer exchange (10 mM Tris pH 7.4) using a centrifuge membrane concentrator (100 kDa cut off membrane). The final material was formulated into 20 mM Tris pH 7.4, 150 mM NaCl. About 2.3 mg of purified material or a 46% overall yield was obtained.

### Glycoprofiling analysis of N-linked Glycans on CSTII modified etanercept:

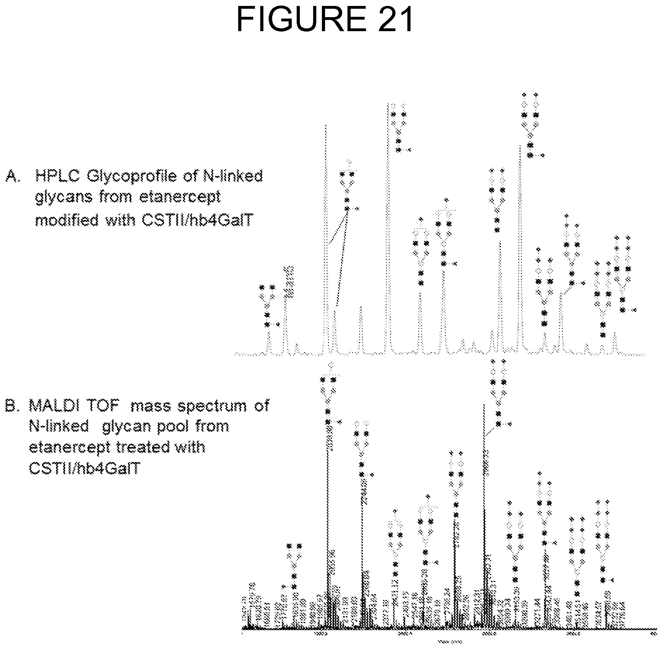

The *N*-linked glycans on the CSTII modified etanercept were analyzed with HPLC glycoprofiling and mass spectrometry of the permethylated glycan pool, Figure 21; Panel A and B, respectively. A complex pattern of glycans was observed. Under these conditions, galactosylation of the Fc glycans was incomplete when compared to the results obtained with the bovine β4GalT enzyme. This may reflect the accessibility differences of these two enzymes to the Fc glycans sites or this may be due to the limited reaction time and enzyme levels employed for the CSTII condition. Near complete galactosylation of the Fc glycans was observed using the hβ4GalT enzyme when used in combination with the hST6Gal1 transferase. However, in the latter case, two additions of both enzymes and sugar nucleotides were employed and the reaction time was carried out for 8 hours compared with a 4 hour reaction time and single addition of enzyme using the CSTII reaction system, Figure 21 Panel A

The glycan pattern of the TNF receptor region of the protein was also complex indicating that partial sialylation was achieved under these conditions; about 70% of the available galactose residues were covered with sialic acid residues. Biantennary glycans with three or four sialic acid residues were also identified with mass spectral analysis of the permethylated glycan pool, Figure 21 Panel B. Unlike the 3,6 disubstituted galactose type structures that are found with the PdST2,6 enzyme, in this case the sialic acid moiety is in 2,8 linkage to the terminal sialic acid residue. This type of reaction product is to be expected given the well defined substrate specificity of the Campylobacter 2,3 sialyltransferase; although this type of structure has not been reported on Fc Fusion proteins (Gilbert, M and Wakarchuk, W.W. US patent 7,138,258; Blixt, O, et al Carb Res. 340: 1963-1972 (2005), Lindhout, T. et al, PNAS 108: 7397-7402).

### IEF Acrylamide Gel Analysis of Glycoengineered Etanercept Variants-

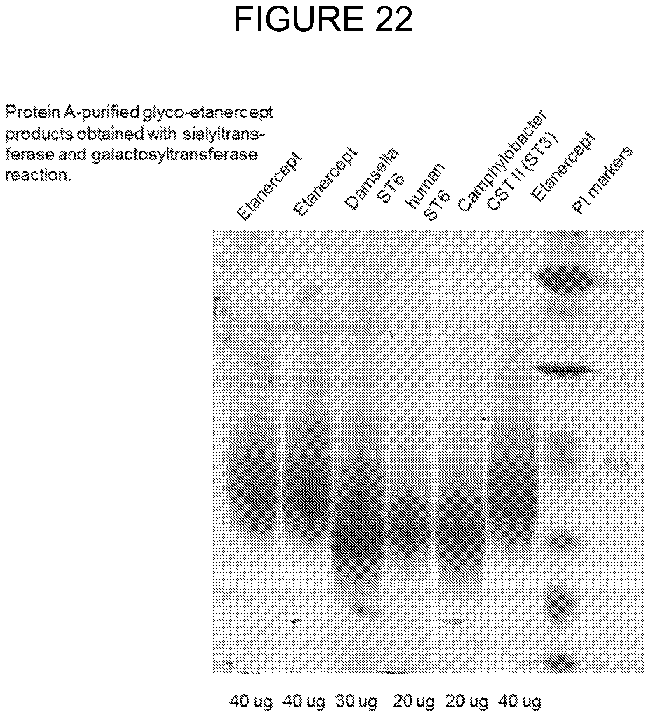

The three, protein A purified, reaction products obtained herein were compared to unmodified commercial etanercept with IEF acrylamide gel electrophoresis analysis. This data Figure 22 shows that the Pd2,6ST-modified product had the most acidic PI, and therefore contained higher levels of sialic acid than the CST II or hST6Gal1 modified molecule. The CST II-engineered protein was slightly more acidic than the human ST6GalT-altered material. Glyco profiling analysis and the relative levels of sialylation of the three etanercept variants as predicted by IEF analysis are consistent.

### Example 8: Pharmacokinetic Analysis in Rats

The effect of glycoengineering etanercept on its serum clearance time was evaluated in a pharmacokinetic study in rats and the results compared with unmodified etanercept which served as a reference. Two groups of 5 male Sprague Dawley rats weighting approximately 200-250 gms were cannulated at their jugular vein. At time = 0 etanercept and glyco-etanercept (prepared using the Pd2,6ST enzyme) were administered via intravenous injection at a dose of 5 mg/kg at a dosing volume of 5 mL/kg. At various time points (prebleed, 10, 30, 60 min.; 3, 6 12, 24, 48 72, 96 hrs.; 5, 7,9,11,13, days, five rats from each group were bled, taking about 0.5 mL blood. The collected samples were centrifuged and the serum was removed and stored frozen until analysis.

### Elisa evaluation of serum samples:

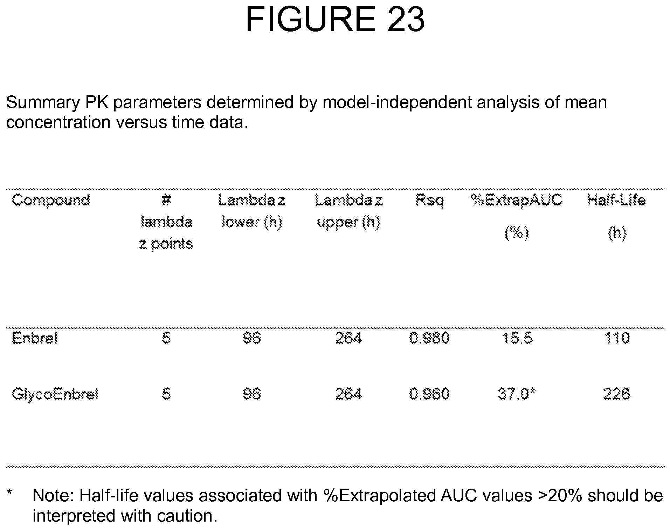

Human IgG ELISA quantitation set (Bethyl Labs, Cat. No E80-104) was used to measure the level of glyco-etanercept or etanercept in the serum samples collected as described in duplicate samples. The serum levels of the two proteins were plotted as a percentage of the starting dose on a semilog plot vs time of sampling. Figure 23 shows the pharmacokinetic analysis of glyco-etanercept in comparison to unmodified etanercept. The data shows that the initial drop in serum levels of the two proteins are very similar up to about 150 hours. At this point, the levels of etanercept continue to show a sharp decline to almost negligible levels while the glyco-etanercept shows only a modest decay rate, if any. With a t ½ set to 1 for etanercept, the t ½ for glycoetanercept in this region is greater than 1.6 times the value of etanercept. Thus the data demonstrates an extended residence time of the glyc- engineered version of etanercept in the serum.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

### Example 9: Glycosylation of abatacept with hβ4Gal T and Pd2,6 ST

### Reaction pH 6.4 in MES Buffer

Buffer-exchanged abatacept (∼ 5 mg) in 50 mM MES buffer pH 6.4 was added to a 2 mL polypropylene tube, followed by 7 µmoles (4 mg) UDP-Gal and 6 µmoles (4 mg) of CMP Sialic acid, ∼0.25 U human β4GalT4 and ∼ 0.26 U of Pd 2,6 ST and 2.0 µmoles MnCl₂ in a total volume of ∼1.28 mL of 50 mM MES buffer, pH 6.4. The reaction was allowed to proceed at 37 °C for 2 hours. A sample was removed from the reaction mixture and stored on ice. At this time, an additional 6 µmoles (4 mg) of CMP sialic acid was added and the reaction allowed to proceed for 2 hours at 37 °C.

### Protein A purification:

The crude reaction product was adjusted to be pH 7.4, 10 mM Tris-HCl using a 100X concentrated buffer solution. The reaction was applied at least 5 times at room temperature to a 1 ml protein A Sepharose column. After washing the column with 10 column volumes of 10 mM Tris, pH 7.4, the modified abatacept was eluted with 5 column volumes of Gentle AG/Ab elution buffer pH 6.6 (Pierce chemical co.). The eluting buffer was removed with buffer exchange (10 mM Tris pH 7.4) using a centrifuge membrane concentrator (100 kDa cut off membrane). The final material was formulated into 20 mM Tris pH 7.4, 150 mM NaCl. About 3.4 mg of purified material or ∼ 68% overall yield was obtained.

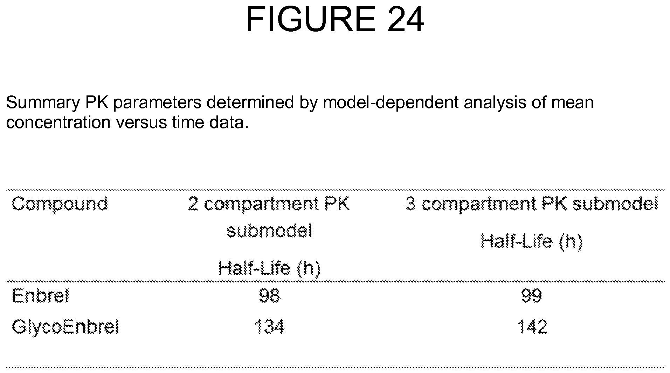

The reaction product was monitored with IEF acrylamide gel, and compared with the starting abatacept. Figure 24. About nine electrophoretic forms were detected in the starting abatacept material, These collapsed to about 3-4 isoforms after glycosyltransferase treatment. The resulting reaction product had a more acidic PI than the starting material indicating that additional sialic acid residues had been added to the glycans.

### Reaction at pH 7.5 in Tris-buffered Saline

Abatacept was treated with hβ4GalT and Pd2,6ST similar to that described above except that the reaction was carried out on about 6 mg protein in 50 mM Tris buffer pH 7.5 containing 150 mM NaCl and the reaction time was increased to 5 hr at 37 °C, Purification on Protein A chromatography was carried out as described above giving a yield of about 3.9 mg or ∼ 65%

### Glycoprofiling of Abatacept and Glyco-Abatacept

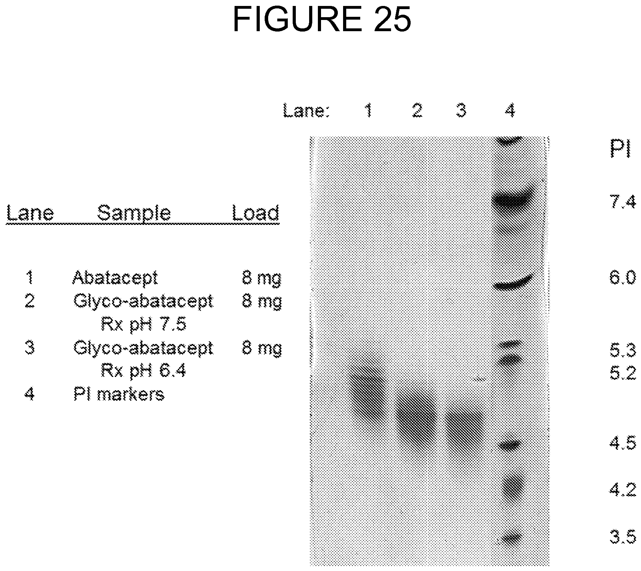

Glycoprofiling of abatacept and the product modified with Pd2,6ST and human β4GalT was carried out using MALDI TOF mass spectral analysis of the PNGase F released N-glycans as their permethylated derivatives , Figure 25 panel A and B respectively. Unmodified abatacept showed that a significant percentage of the N-glycans were incomplete structures and contained no sialic acid residues. After glycosyltransferase treatment only those glycans at the Fc region contained terminal galactose residues. The presence of disialylated galactose residues was verified in the modified molecule, similar to what was observed with etanercept treated with these enzymes.

### Example 10: Preparation of Polysialic Acid Glycans on Fc Fusion Proteins

The addition of polysialic acid residues ((Neu5Ac)ₙ) on to the glycans of Fc fusion proteins was achieved by a two step glycosylation procedure. The first step involved the addition of a 2,8 sialic acid residue to the terminus of the glycan chain . This linkage serveed a primer for the polysialyltransferase enzyme (PST). The two step process allowed for maximal addition of the primer 2,8 sialic acid residues on to an existing 2,3 sialic acid linked to galactose. Short reaction times were necessary at this step in order to minimize the reverse reaction (sialidase activity) of the enzyme . The addition of polymeric sialic acid, in contrast, required extended reaction times to maximize the polymer length.

In a typical preparation, the procedure for preparation of glyco etanercept reacted with the CST II enzyme is carried out as described earlier, except that the reaction time was limited to 30 minutes or less. After isolation of the modified Fc fusion protein with protein A chromatography, the resulting protein (∼3 mg) with disialic acid structures was reacted in 50 mM sodium phosphate pH 8.0, 10 mM MgCl₂, 10 mM CMP sialic acid, and 0.08 mg/mL of PST enzyme. PST used in these studies was constructed by a domain swap strategy interchanging coding domains from NmB PST and NMC PST (Peterson, D. C. et al J Bacteriology 1576-1582 (2011)). The resulting chimeric protein was purified using a maltose binding protein tag.

The reaction product was identified using SDS gel acrylamide electrophoresis, staining with coomassie blue dye and inspecting for a polymeric ladder of higher molecular weight protein bands.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims be covered thereby.

## Claims

1. An Fc fusion protein, comprising an immunoglobulin Fc region linked to a biologically active polypeptide comprising one or more oligosaccharide, wherein:
i. more than 10% of all oligosaccharides on the biologically active polypeptide terminate in sialic acid; and
ii. at least one of the oligosaccharides linked to the biologically active polypeptide comprises a biantennary glycan structure terminating in at least 3 sialic acid molecules, or a triantennary glycan structure terminating in at least 4 sialic acid molecules, or a tetraantennary glycan structure terminating in at least 5 sialic molecules; and
wherein the serum half-life of the Fc fusion protein is at least 30% longer relative to a corresponding Fc fusion protein in which none of the oligosaccharides comprise a biantennary glycan structure terminating in 3 or 4 sialic acid molecules.

2. The Fc fusion protein of claim 1, wherein at least 5%, 10%, 15%, 20% of the oligosaccharides linked to the biologically active polypeptide comprise a biantennary glycan structure terminating in at least 3 sialic acid molecules.

3. The Fc fusion protein of claim 1, wherein at least 5%, 10%, 15%, 20% of the oligosaccharides linked to the biologically active polypeptide comprise a triantennary glycan structure terminating in at least 4 sialic acid molecules.

4. The Fc fusion protein of claim 1, wherein at least 5%, 10%, 15%, 20% of the oligosaccharides linked to the biologically active polypeptide comprise a tetraantennary glycan structure terminating in at least 5 sialic molecules.

5. The Fc fusion protein of claim 1, wherein at least one of the oligosaccharides linked to the biologically active polypeptide comprises a biantennary glycan structure terminating in at least 3 or 4 sialic acid molecules.

6. The Fc fusion protein of claim 1, wherein more than 20%, 30%, 40%, 50%, 60%, 70%, 80%, or 90% of all oligosaccharides in the Fc fusion protein terminate in sialic acid.

7. The Fc fusion protein of claim 1, wherein:
(a) at least one N-linked oligosaccharide terminates in sialic acid molecules linked to
i. a 3 and a 6 position of a first galactose residue on a first antenna of the N-linked oligosaccharide; and
ii. a 6 position of a second galactose residue on a second antenna of the N-linked oligosaccharide; or
(b) at least one N-linked oligosaccharide terminates in sialic acid molecules linked to
i. a 3 and a 6 position of a first galactose residue on a first antenna of the N-linked oligosaccharide; and
ii. a 3 position of a second galactose residue on a second antenna of the N-linked oligosaccharide; or
(c) at least one N-linked oligosaccharide terminates in sialic acid molecules linked to
i. a 3 position of a first galactose residue on a first antenna of the N-linked oligosaccharide; and
ii. a 3 and a 6 position of a second galactose residue on a second antenna of the N-linked oligosaccharide; or
(d) at least one N-linked oligosaccharide terminates in sialic acid molecules linked to
i. a 6 position of a first galactose residue on a first antenna of the N-linked oligosaccharide;
ii. a 3 and a 6 position of a second galactose residue on a second antenna of the N-linked oligosaccharide; or
(e) at least one N-linked oligosaccharide terminates in
i. a 2,8 sialic acid molecule linked to a first sialic acid residue linked to a 3 or a 6 position of a first galactose residue on a first antenna of the N-linked oligosaccharide; and
ii. a 2,8 sialic acid molecule linked to a second sialic acid residue linked to a 3 or a 6 position of a second galactose residue on a second antenna of the N-linked oligosaccharide; or
(f) at least one N-linked oligosaccharide terminates in
i. a 2,8 sialic acid molecule linked to a first sialic acid residue linked to a 3 or a 6 position of a first galactose residue on a first antenna of the N linked oligosaccharide; and
ii. a 2,8 sialic acid molecule linked to a 3 or a 6 position of a second galactose residue on a second antenna of the N-linked oligosaccharide; or
(g) at least one of N-linked oligosaccharide terminates in
i. a 2,8 sialic acid molecule linked to a 3 or a 6 position of a first galactose residue on a first antenna of the N-linked oligosaccharide; and
ii. a 2,8 sialic acid molecule linked to a sialic acid residue linked to a 3 or a 6 position of a second galactose residue on a second antenna of the N-linked oligosaccharide; or
(h) at least one of N-linked oligosaccharide terminates in
i. a 2,8 sialic acid molecule linked to a 2,3 sialic acid molecule linked to a first galactose residue on a first antenna of the oligosaccharide; and
ii. a 2,8 sialic acid molecule linked to a 2,3 sialic acid molecule linked to a second galactose residue on a second antenna of the oligosaccharide; or
(i) at least one N-linked oligosaccharide terminates in
i. a 2,8 sialic acid molecule linked to a 2,3 sialic acid molecule linked to a first galactose residue on a first antenna of the oligosaccharide; and
ii. a 2,3 sialic acid molecule linked to a second galactose residue on a second antenna of the oligosaccharide; or
(j) at least one N-linked oligosaccharide terminates in sialic acid molecules linked to
i. a 3 and a 6 position of a first galactose residue on a first antenna of the N-linked oligosaccharide; and
ii. a 3 and a 6 position of a second galactose residue on a second antenna of the N-linked oligosaccharide.

8. The Fc fusion protein of claim 1, wherein at least one of N-linked oligosaccharides is selected from the group consisting of

Neu5Ac Neu5Ac Neu5Ac
Gal Gal
GlcNAc GlcNAc
Man Man
Man
GlcNAc
GlcNAc
3,6 disialic-3 arm; 3 or 6 mono sialic-6 arm ,

Neu5Ac Neu5Ac Neu5Ac
Gal Gal
GlcNAc GlcNAc
Man Man
Man
GlcNAc
GlcNAc Fuc
3,6 disialic-3 arm; 3 or 6 mono sialic-6 arm ,

Neu5Ac Neu5Ac Neu5Ac
Gal Gal
GlcNAc GlcNAc
Man Man
Man
GlcNAc
GlcNAc
3,6 disialic-6 arm; 3 or 6 mono sialic-3 arm ,

Neu5Ac Neu5Ac Neu5Ac Neu5Ac Neu5Ac Neu5Ac Neu5Ac Neu5Ac Neu5Ac Neu5Ac Neu5Ac
Gal Gal Gal Gal Gal Gal
GlcNAc GlcNAc GlcNAc GlcNAc GlcNAc GlcNAc
Man Man Man Man Man Man
Man Man Man
GlcNAc GlcNAc GlcNAc
GlcNAc Fuc GlcNAc GlcNAc Fuc
3,6 disialic-6 arm; 3 or 6 mono sialic-3 arm , 3,6 disialic acid; both 3 and 6 arm or tetra sialic -bianatennary , 3,6 disialic acid; both 3 and 6 arm or tetra sialic -bianatennary ,

Neu5Ac Neu5Ac Neu5Ac Neu5Ac
Neu5Ac Neu5Ac Neu5Ac Neu5Ac
Gal Gal Gal Gal
GlcNAc GlcNAc GlcNAc GlcNAc
Man Man Man Man
Man Man
GlcNAc GlcNAc
GlcNAc GlcNAc Fuc
2,8 sialic-sialic; 2,3 sialic-galactose; 3 and 6 arm , 2,8 sialic-sialic; 2,3 sialic-galactose; 3 and 6 arm ,

Neu5Ac
Neu5Ac
Gal
GlcNAc
Man
Neu5Ac
Gal
GlcNAc
Man
Man
GlcNAc
GlcNAc
2,8 sialic-sialic; 2,3 sialic-galactose; 3 or 6 arm, and
Neu5Ac
Neu5Ac
Gal
GlcNAc
Man
Neu5Ac
Gal
GlcNAc
Man
Man
GlcNAc
GlcNAc—Fuc
2,8 sialic-sialic; 2,3 sialic-galactose; 3 or 6 arm.

9. The Fc fusion protein of claim 1, wherein at least one of N-linked oligosaccharide terminates in (Neu5Ac)ₙ, wherein n is an integer between 3-20.

10. The Fc fusion protein of claim 1 wherein the biologically active polypeptide comprises a TNFR2 fragment; a TNFR1 fragment, a CTLA-4 fragment, or protective antigen (PA).

11. The Fc fusion protein of claim 1, wherein the serum half-life of the Fc fusion protein is at least 40%, 50%, 60%, 70%, 80%, 100% or 200% longer relative to a corresponding Fc fusion protein in which none of the oligosaccharides comprise a biantennary glycan structure terminating in 3 or 4 sialic acid molecules.

12. A method of preparing the Fc fusion protein of any preceding claim, said method comprising:
a) providing a Fc fusion protein, wherein the Fc fusion protein comprises an immunoglobulin Fc region linked to a biologically active polypeptide comprising at least one oligosaccharide; and
b) exposing the Fc fusion protein to the action of at least one glycosyltransferase to result in the Fc fusion protein of any preceding claim.

13. The method of claim 12, wherein at least two glycosyltransferases are used.

14. The Fc fusion protein of any one of claims 1-11 for use in treating a disorder in a subject in need thereof.

15. The Fc fusion protein for use according to claim 14, wherein the disorder is an inflammatory disorder, for example rheumatoid arthritis, juvenile rheumatoid arthritis, psoriatic arthritis, ankylosing spondylitis.

## Patentansprüche

1. Fc-Fusionsprotein, umfassend eine Immunglobulin-Fc-Region, die mit einem biologisch aktiven Polypeptid verknüpft ist, das ein oder mehrere Oligosaccharide umfasst, wobei:
i. mehr als 10 % aller Oligosaccharide an dem biologisch aktiven Polypeptid mit Sialinsäure enden; und
ii. wenigstens eines der mit dem biologisch aktiven Polypeptid verknüpften Oligosaccharide eine biantennäre Glycanstruktur umfasst, die mit wenigstens 3 Sialinsäuremolekülen endet, oder eine triantennäre Glycanstruktur, die mit wenigstens 4 Sialinsäuremolekülen endet, oder eine tetraantennäre Glycanstruktur, die mit wenigstens 5 Sialinsäuremolekülen endet; und
wobei die Serumhalbwertszeit des Fc-Fusionsproteins im Verhältnis zu einem entsprechenden Fc-Fusionsprotein, in dem keines der Oligosaccharide eine biantennäre Glycanstruktur umfasst, die mit 3 oder 4 Sialinsäuremolekülen endet, wenigstens 30 % länger ist.

2. Fc-Fusionsprotein nach Anspruch 1, wobei wenigstens 5 %, 10 %, 15 %, 20 % der mit dem biologisch aktiven Polypeptid verknüpften Oligosaccharide eine biantennäre Glycanstruktur umfassen, die mit wenigstens 3 Sialinsäuremolekülen endet.

3. Fc-Fusionsprotein nach Anspruch 1, wobei wenigstens 5 %, 10 %, 15 %, 20 % der mit dem biologisch aktiven Polypeptid verknüpften Oligosaccharide eine triantennäre Glycanstruktur umfassen, die mit wenigstens 4 Sialinsäuremolekülen endet.

4. Fc-Fusionsprotein nach Anspruch 1, wobei wenigstens 5 %, 10 %, 15 %, 20 % der mit dem biologisch aktiven Polypeptid verknüpften Oligosaccharide eine tetraantennäre Glycanstruktur umfassen, die mit wenigstens 5 Sialinsäuremolekülen endet.

5. Fc-Fusionsprotein nach Anspruch 1, wobei wenigstens eines der mit dem biologisch aktiven Polypeptid verknüpften Oligosaccharide eine biantennäre Glycanstruktur umfasst, die mit wenigstens 3 oder 4 Sialinsäuremolekülen endet.

6. Fc-Fusionsprotein nach Anspruch 1, wobei mehr als 20 %, 30 %, 40 %, 50 %, 60 %, 70 %, 80 % oder 90 % aller Oligosaccharide in dem Fc-Fusionsprotein mit Sialinsäure enden.

7. Fc-Fusionsprotein nach Anspruch 1, wobei:
(a) wenigstens ein N-verknüpftes Oligosaccharid mit Sialinsäuremolekülen endet, die mit
i. einer 3- und einer 6-Position eines ersten Galactoserestes an einer ersten Antenne des N-verknüpften Oligosaccharids; und
ii. einer 6-Position eines zweiten Galactoserestes an einer zweiten Antenne des N-verknüpften Oligosaccharids verknüpft sind; oder
(b) wenigstens ein N-verknüpftes Oligosaccharid mit Sialinsäuremolekülen endet, die mit
i. einer 3- und einer 6-Position eines ersten Galactoserestes an einer ersten Antenne des N-verknüpften Oligosaccharids; und
ii. einer 3-Position eines zweiten Galactoserestes an einer zweiten Antenne des N-verknüpften Oligosaccharids verknüpft sind; oder
(c) wenigstens ein N-verknüpftes Oligosaccharid mit Sialinsäuremolekülen endet, die mit
i. einer 3-Position eines ersten Galactoserestes an einer ersten Antenne des N-verknüpften Oligosaccharids;
ii. einer 3- und einer 6-Position eines zweiten Galactoserestes an einer zweiten Antenne des N-verknüpften Oligosaccharids verknüpft sind; oder
(d) wenigstens ein N-verknüpftes Oligosaccharid mit Sialinsäuremolekülen endet, die mit
i. einer 6-Position eines ersten Galactoserestes an einer ersten Antenne des N-verknüpften Oligosaccharids;
ii. einer 3- und einer 6-Position eines zweiten Galactoserestes an einer zweiten Antenne des N-verknüpften Oligosaccharids verknüpft sind; oder
(e) wenigstens ein N-verknüpftes Oligosaccharid endet mit
i. einem 2,8-Sialinsäuremolekül, das mit einem ersten Sialinsäurerest verknüpft ist, der mit einer 3- oder einer 6-Position eines ersten Galactoserestes an einer ersten Antenne des N-verknüpften Oligosaccharids verknüpft ist; und
ii. einem 2,8-Sialinsäuremolekül, das mit einem zweiten Sialinsäurerest verknüpft ist, der mit einer 3- oder einer 6-Position eines zweiten Galactoserestes an einer zweiten Antenne des N-verknüpften Oligosaccharids verknüpft ist; oder
(f) wenigstens ein N-verknüpftes Oligosaccharid endet mit
i. einem 2,8-Sialinsäuremolekül, das mit einem ersten Sialinsäurerest verknüpft ist, der mit einer 3- oder einer 6-Position eines ersten Galactoserestes an einer ersten Antenne des N-verknüpften Oligosaccharids verknüpft ist; und
ii. einem 2,8-Sialinsäuremolekül, das mit einer 3- oder einer 6-Position eines zweiten Galactoserestes an einer zweiten Antenne des N-verknüpften Oligosaccharids verknüpft ist; oder
(g) wenigstens ein N-verknüpftes Oligosaccharid endet mit
i. einem 2,8-Sialinsäuremolekül, das mit einer 3- oder einer 6-Position eines ersten Galactoserestes an einer ersten Antenne des N-verknüpften Oligosaccharids verknüpft ist; und
ii. einem 2,8-Sialinsäuremolekül, das mit einem Sialinsäurerest verknüpft ist, der mit einer 3- oder einer 6-Position eines zweiten Galactoserestes an einer zweiten Antenne des N-verknüpften Oligosaccharids verknüpft ist; oder
(h) wenigstens ein N-verknüpftes Oligosaccharid endet mit
i. einem 2,8-Sialinsäuremolekül, das mit einem 2,3-Sialinsäuremolekül verknüpft ist, das mit einem ersten Galactoserest an einer ersten Antenne des Oligosaccharids verknüpft ist; und
ii. einem 2,8-Sialinsäuremolekül, das mit einem 2,3-Sialinsäuremolekül verknüpft ist, das mit einem zweiten Galactoserest an einer zweiten Antenne des N-verknüpften Oligosaccharids verknüpft ist; oder
(i) wenigstens ein N-verknüpftes Oligosaccharid endet mit
i. einem 2,8-Sialinsäuremolekül, das mit einem 2,3-Sialinsäuremolekül verknüpft ist, das mit einem ersten Galactoserest an einer ersten Antenne des Oligosaccharids verknüpft ist; und
ii. einem 2,3-Sialinsäuremolekül, das mit einem zweiten Galactoserest an einer zweiten Antenne des Oligosaccharids verknüpft ist; oder
(j) wenigstens ein N-verknüpftes Oligosaccharid mit Sialinsäuremolekülen endet, die verknüpft sind mit
i. einer 3- und einer 6-Position eines ersten Galactoserestes an einer ersten Antenne des N-verknüpften Oligosaccharids; und
ii. einer 3- und einer 6-Position eines zweiten Galactoserestes an einer zweiten Antenne des N-verknüpften Oligosaccharids.

8. Fc-Fusionsprotein nach Anspruch 1, wobei wenigstens eines der N-verknüpften Oligosaccharide aus der Gruppe ausgewählt ist, die aus 
Neu5Ac Neu5Ac Neu5Ac Neu5Ac Neu5Ac Neu5Ac
Gal Gal Gal Gal
GlcNAc GlcNAc GlcNAc GlcNAc
Man Man Man Man
Man Man
GlcNAc GlcNAc
GlcNAc GlcNAc Fuc

3,6-Disialinsäure-3-Arm; 3,6-Disialinsäure-3-Arm;,
3- oder 6-Monosialinsäure-6-Arm, 3- oder 6-Monosialinsäure-6-Arm,
 
Neu5Ac Neu5Ac Neu5Ac
Gal Gal
GlcNAc GlcNAc
Man Man
Man
GlcNAc
GlcNAc

3,6-Disialinsäure-6-Arm;
3- oder 6-Monosialinsäure-3-Arm,
 
Neu5Ac Neu5Ac Neu5Ac
Gal Gal
GlcNAc GlcNAc
Man Man
Man
GlcNAc
GlcNAc Fuc
3,6-Disialinsäure-6-Arm;
3- oder 6-Monosialinsäure-3-Arm,
 
Neu5Ac Neu5Ac Neu5Ac Neu5Ac Neu5Ac Neu5Ac Neu5Ac Neu5Ac
Gal Gal Gal Gal
GlcNAc GlcNAc GlcNAc GlcNAc
Man Man Man Man
Man Man
GlcNAc GlcNAc
GlcNAc GlcNAc Fuc
3,6-Disialinsäure; sowohl 3- als auch 6-Arm 3,6-Disialinsäure; sowohl 3- als auch 6-Arm
oder Tetrasialinsäure - biantennär, oder Tetrasialinsäure - biantennär
 
Neu5Ac Neu5Ac Neu5Ac Neu5Ac
Neu5Ac Neu5Ac Neu5Ac Neu5Ac
Gal Gal Gal Gal
GlcNAc GlcNAc GlcNAc GlcNAc
Man Man Man Man
Man Man
GlcNAc GlcNAc
GlcNAc GlcNAc Fuc
2,8-Sialinsäure-Sialinsäure; 2,3-Sialinsäure-Galactose; 2,8-Sialinsäure-Sialinsäure; 2,3-Sialinsäure-Galactose
3- und 6-Arm, 3- und 6-Arm
 
Neu5Ac Neu5Ac
Neu5Ac Neu5Ac Neu5Ac Neu5Ac
Gal Gal Gal Gal
GlcNAc GlcNAc GlcNAc GlcNAc
Man Man Man Man
Man Man
GlcNAc GlcNAc
GlcNAc GlcNAc Fuc
2,8-Sialinsäure-Sialinsäure;
2,3-Sialinsäure-Galactose; 3- oder 6-Arm, und 2,8-Sialinsäure-Sialinsäure;
2,3-Sialinsäure-Galactose; 3- oder 6-Arm.
 besteht.

9. Fc-Fusionsprotein nach Anspruch 1, wobei wenigstens eines der N-verknüpften Oligosaccharide mit (Neu5Ac)ₙ endet, wobei n eine ganze Zahl zwischen 3-20 ist.

10. Fc-Fusionsprotein nach Anspruch 1, wobei das biologisch aktive Polypeptid ein TNFR2-Fragment, ein TNFR1-Fragment, ein CTLA-4-Fragment oder protektives Antigen (PA) umfasst.

11. Fc-Fusionsprotein nach Anspruch 1, wobei die Serumhalbwertszeit des Fc-Fusionsproteins im Verhältnis zu einem entsprechenden Fc-Fusionsprotein, in dem keines der Oligosaccharide eine biantennäre Glycanstruktur umfasst, die mit 3 oder 4 Sialinsäuremolekülen endet, wenigstens 40 %, 50 %, 60 %, 70 %, 80 %, 100 % oder 200 % länger ist.

12. Verfahren zur Herstellung des Fc-Fusionsproteins nach einem der vorhergehenden Ansprüche, das umfasst:
a) Bereitstellen eines Fc-Fusionsproteins, wobei das Fc-Fusionsprotein eine Immunglobulin-Fc-Region umfasst, die mit einem biologisch aktiven Polypeptid verknüpft ist, das wenigstens ein Oligosaccharid umfasst; und
b) Exposition des Fc-Fusionsproteins gegenüber der Wirkung von wenigstens einer Glykosyltransferase, was zu dem Fc-Fusionsprotein nach einem der vorhergehenden Ansprüche führt.

13. Verfahren nach Anspruch 12, wobei wenigstens zwei Glykosyltransferasen verwendet werden.

14. Fc-Fusionsprotein nach einem der Ansprüche 1-11 zur Verwendung bei der Behandlung einer Erkrankung in einem Individuum, das dessen bedarf.

15. Fc-Fusionsprotein zur Verwendung nach Anspruch 14, wobei es sich bei der Erkrankung um eine inflammatorische Erkrankung, zum Beispiel rheumatoide Arthritis, juvenile rheumatoide Arthritis, Psoriasis-Arthritis, ankylosierende Spondylitis handelt.

## Revendications

1. Protéine de fusion Fc, comprenant une région Fc d'immunoglobuline liée à un polypeptide biologiquement actif comprenant un ou plusieurs oligosaccharides, où :
i. plus de 10% de tous les oligosaccharides sur le polypeptide biologiquement actif se terminent par de l'acide sialique ; et
ii. au moins l'un des oligosaccharides liés au polypeptide biologiquement actif comprend une structure de glycane biantennaire se terminant par au moins 3 molécules d'acide sialique, ou une structure de glycane triantennaire se terminant par au moins 4 molécules d'acide sialique, ou une structure de glycane tétra-antennaire se terminant par au moins 5 molécules d'acide sialique ; et
où la demi-vie sérique de la protéine de fusion Fc est au moins 30% plus longue par rapport à une protéine de fusion Fc correspondante dans laquelle aucun des oligosaccharides ne comprend de structure de glycane biantennaire se terminant par 3 ou 4 molécules d'acide sialique.

2. Protéine de fusion Fc selon la revendication 1, dans laquelle au moins 5%, 10%, 15%, 20% des oligosaccharides liés au polypeptide biologiquement actif comprennent une structure de glycane biantennaire se terminant par au moins 3 molécules d'acide sialique.

3. Protéine de fusion Fc selon la revendication 1, dans laquelle au moins 5%, 10%, 15%, 20% des oligosaccharides liés au polypeptide biologiquement actif comprennent une structure de glycane triantennaire se terminant par au moins 4 molécules d'acide sialique.

4. Protéine de fusion Fc selon la revendication 1, dans laquelle au moins 5%, 10%, 15%, 20% des oligosaccharides liés au polypeptide biologiquement actif comprennent une structure de glycane tétra-antennaire se terminant par au moins 5 molécules d'acide sialique.

5. Protéine de fusion Fc selon la revendication 1, dans laquelle au moins l'un des oligosaccharides liés au polypeptide biologiquement actif comprend une structure de glycane biantennaire se terminant par au moins 3 ou 4 molécules d'acide sialique.

6. Protéine de fusion Fc selon la revendication 1, dans laquelle plus de 20%, 30%, 40%, 50%, 60%, 70%, 80% ou 90% de tous les oligosaccharides dans la protéine de fusion Fc se terminent par de l'acide sialique.

7. Protéine de fusion Fc selon la revendication 1, dans laquelle :
(a) au moins un oligosaccharide N-lié se termine par des molécules d'acide sialique liées
i. à une position 3 et 6 d'un premier résidu galactose sur une première antenne de l'oligosaccharide N-lié ; et
ii. à une position 6 d'un deuxième résidu galactose sur une deuxième antenne de l'oligosaccharide N-lié ; ou
(b) au moins un oligosaccharide N-lié se termine par des molécules d'acide sialique liées
i. à une position 3 et 6 d'un premier résidu galactose sur une première antenne de l'oligosaccharide N-lié ; et
ii. à une position 3 d'un deuxième résidu galactose sur une deuxième antenne de l'oligosaccharide N-lié ; ou
(c) au moins un oligosaccharide N-lié se termine par des molécules d'acide sialique liées
i. à une position 3 d'un premier résidu galactose sur une première antenne de l'oligosaccharide N-lié ; et
ii. à une position 3 et 6 d'un deuxième résidu galactose sur une deuxième antenne de l'oligosaccharide N-lié ; ou
(d) au moins un oligosaccharide N-lié se termine par des molécules d'acide sialique liées
i. à une position 6 d'un premier résidu galactose sur une première antenne de l'oligosaccharide N-lié ; et
ii. à une position 3 et 6 d'un deuxième résidu galactose sur une deuxième antenne de l'oligosaccharide N-lié ; ou
(e) au moins un oligosaccharide N-lié se termine par
i. une molécule d'acide 2,8-sialique liée à un premier résidu d'acide sialique lié à une position 3 ou 6 d'un premier résidu galactose sur une première antenne de l'oligosaccharide N-lié ; et
ii. une molécule d'acide 2,8-sialique liée à un deuxième résidu d'acide sialique lié à une position 3 ou 6 d'un deuxième résidu galactose sur une deuxième antenne de l'oligosaccharide N-lié ; ou
(f) au moins un oligosaccharide N-lié se termine par
i. une molécule d'acide 2,8-sialique liée à un premier résidu d'acide sialique lié à une position 3 ou 6 d'un premier résidu galactose sur une première antenne de l'oligosaccharide N-lié ; et
ii. une molécule d'acide 2,8-sialique liée à une position 3 ou 6 d'un deuxième résidu galactose sur une deuxième antenne de l'oligosaccharide N-lié ; ou
(g) au moins l'un des oligosaccharides N-liés se termine par
i. une molécule d'acide 2,8-sialique liée à une position 3 ou 6 d'un premier résidu galactose sur une première antenne de l'oligosaccharide N-lié ; et
ii. une molécule d'acide 2,8-sialique liée à un résidu d'acide sialique lié à une position 3 ou 6 d'un deuxième résidu galactose sur une deuxième antenne de l'oligosaccharide N-lié ; ou
(h) au moins l'un des oligosaccharides N-liés se termine par
i. une molécule d'acide 2,8-sialique liée à une molécule d'acide 2,3-sialique liée à un premier résidu galactose sur une première antenne de l'oligosaccharide ; et
ii. une molécule d'acide 2,8-sialique liée à une molécule d'acide 2,3-sialique liée à un deuxième résidu galactose sur une deuxième antenne de l'oligosaccharide ; ou
(i) au moins un oligosaccharide N-lié se termine par
i. une molécule d'acide 2,8-sialique liée à une molécule d'acide 2,3-sialique liée à un premier résidu galactose sur une première antenne de l'oligosaccharide ; et
ii. une molécule d'acide 2,3-sialique liée à un deuxième résidu galactose sur une deuxième antenne de l'oligosaccharide ; ou
(j) au moins un oligosaccharide N-lié se termine par des molécules d'acide sialique liées
i. à une position 3 et 6 d'un premier résidu galactose sur une première antenne de l'oligosaccharide N-lié ; et
ii. à une position 3 et 6 d'un deuxième résidu galactose sur une deuxième antenne de l'oligosaccharide N-lié.

8. Protéine de fusion Fc selon la revendication 1, dans laquelle au moins l'un des oligosaccharides N-liés est choisi dans le groupe constitué par

Neu5Ac Neu5Ac Neu5Ac
Gal Gal
GlcNAc GlcNAc
Man Man
Man
GlcNAc
GlcNAc

Neu5Ac Neu5Ac Neu5Ac
Gal Gal
GlcNAc GlcNAc
Man Man
Man
GlcNAc
GlcNAc Fuc

bras 3,6-disialique-3 ; bras 3,6-disialique-3 ; bras
bras 3 ou 6- 3 ou 6-monosialique-6
monosialique-6

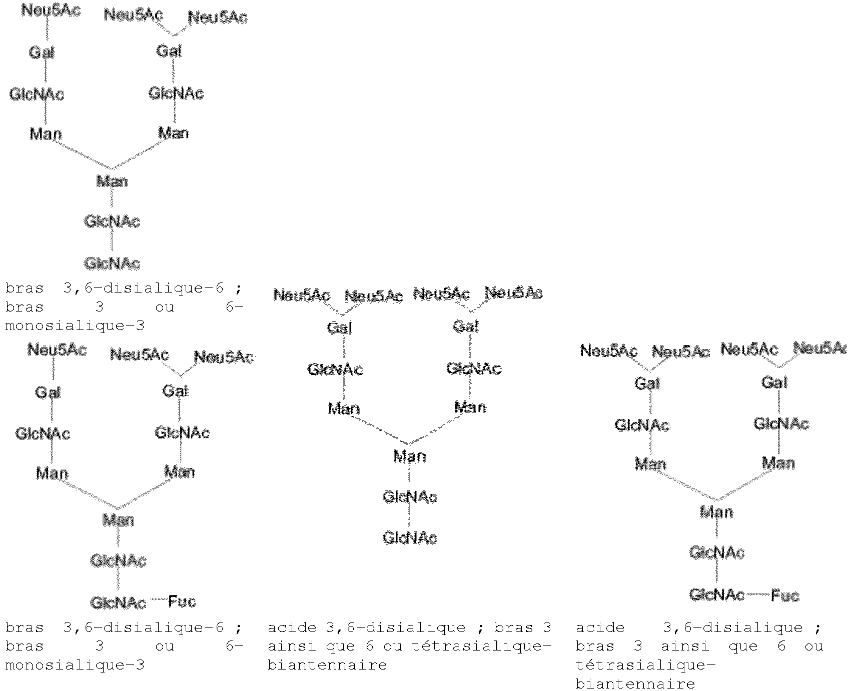
bras 3,6-disialique-6 ;
bras 3 ou 6-
monosialique-3
bras 3,6-disialique-6 ;
bras 3 ou 6-
monosialique-3
acide 3,6-disialique ; bras 3
ainsi que 6 ou tétrasialique-
biantennaire
acide 3,6-disialique ;
bras 3 ainsi que 6 ou
tétrasialique-
biantennaire

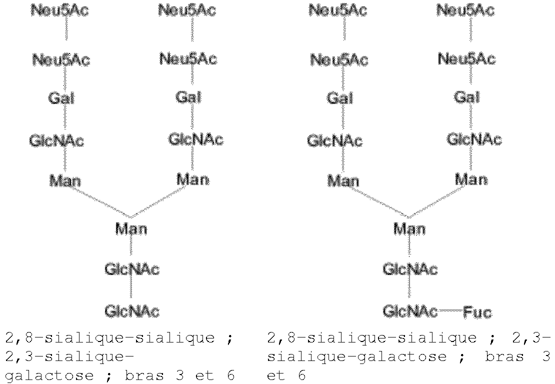

2,8-sialique-sialique ;
2,3-sialique-
galactose ; bras 3 et 6


2,8-sialique-sialique ; 2,3-
sialique-galactose ; bras 3
et 6

Neu5Ac
Neu5Ac
Gal
GlcNAc
Man
Neu5Ac
Gal
GlcNAc
Man
Man
GlcNAc
GlcNAc
Neu5Ac
Neu5Ac
Gal
GlcNAc
Man
Neu5Ac
Gal
GlcNAc
Man
Man
GlcNAc
GlcNAc—Fuc
2,8-sialique-sialique ;
2,3-sialique-
galactose ;  bras  3  et
6 ;
2,8-sialique-sialique ;
2,3-sialique-
galactose ; bras 3 et 6
et
.

9. Protéine de fusion Fc selon la revendication 1, dans laquelle au moins l'un des oligosaccharides N-liés se termine par (Neu5Ac)ₙ, où n est un nombre entier valant 3-20.

10. Protéine de fusion Fc selon la revendication 1, dans laquelle le polypeptide biologiquement actif comprend un fragment TNFR2, un fragment TNFR1, un fragment CTLA-4, ou un antigène protecteur (PA).

11. Protéine de fusion Fc selon la revendication 1, dans laquelle la demi-vie sérique de la protéine de fusion Fc est au moins 40%, 50%, 60%, 70%, 80%, 100% ou 200% plus longue par rapport à une protéine de fusion Fc correspondante dans laquelle aucun des oligosaccharides ne comprend de structure de glycane biantennaire se terminant par 3 ou 4 molécules d'acide sialique.

12. Méthode de préparation de la protéine de fusion Fc selon l'une quelconque des revendications précédentes, ladite méthode comprenant :
a) la fourniture d'une protéine de fusion Fc, où la protéine de fusion Fc comprend une région Fc d'immunoglobuline liée à un polypeptide biologiquement actif comprenant au moins un oligosaccharide ; et
b) l'exposition de la protéine de fusion Fc à l'action d'au moins une glycosyltransférase afin de conduire à la protéine de fusion Fc selon l'une quelconque des revendications précédentes.

13. Méthode selon la revendication 12, dans laquelle au moins deux glycosyltransférases sont utilisées.

14. Protéine de fusion Fc selon l'une quelconque des revendications 1-11, pour une utilisation dans le traitement d'un trouble chez un sujet en ayant besoin.

15. Protéine de fusion Fc pour une utilisation selon la revendication 14, où le trouble est un trouble inflammatoire, par exemple la polyarthrite rhumatoïde, la polyarthrite rhumatoïde juvénile, l'arthrite psoriasique, la spondylarthrite ankylosante.
